# EUROPEAN PATENT SPECIFICATION

(11) **EP 4 516 793 B1**
(45) Date of publication and mention of the grant of the patent: **16.09.2026**
(21) Application number: 23795556.2
(22) Date of filing: 27.04.2023
(51) Int. Cl.: C07D 471/10, C07D 493/00, C07D 495/00, C07D 498/00, C07D 513/00, A61K 31/437, A61K 31/4985, A61K 31/38, A61K 31/335, A61P 25/06

(54) **PIPERIDINE CARBOXAMIDE AZAINDANE DERIVATIVE, METHOD FOR PREPARING SAME, AND USE THEREOF**
PIPERIDINCARBOXAMIDAZAINDANDERIVAT, VERFAHREN ZUR HERSTELLUNG DAVON UND VERWENDUNG DAVON
DÉRIVÉ D'AZAINDANE DE PIPÉRIDINE CARBOXAMIDE, SON PROCÉDÉ DE PRÉPARATION ET SON UTILISATION

(30) Priority: 29.04.2022 CN 202210467997; 01.07.2022 CN 202210769471; 01.12.2022 CN 202211530536
(43) Date of publication of application: 05.03.2025
(73) Proprietor: Brise Pharmaceuticals Co., Ltd., Shanghai 201315 (CN)
(72) Inventor: YING, Yongcheng, Shanghai 201203 (CN); LI, Guochun, Shanghai 201203 (CN); YUAN, Xiaohui, Shanghai 201203 (CN); YU, Shanghai, Shanghai 201203 (CN); GU, Liming, Shanghai 201203 (CN); LOU, Wanqiao, Shanghai 201203 (CN)
(74) Representative: Lavoix
(86) International application number: PCT/CN2023/091223
(87) International publication number: WO 2023/208124

(56) References cited:
- WO-A1-2013/169567
- CN-A- 101 443 323
- CN-A- 103 328 478
- US-A1- 2013 231 358

## Description

### FIELD OF THE INVENTION

The present application relates to a piperidinecarboxamide azaindane derivative, a preparation method thereof, a pharmaceutical composition containing the derivative or a deuterated derivative thereof, and the use of the derivative as a therapeutic agent or a preventive agent, in particular as a calcitonin gene-related peptide (CGRP) receptor antagonist.

### BACKGROUND OF THE INVENTION

Migraine is a common trigeminal neuralgia headache. The pain may last for 4 to 72 hours and is characterized by throbbing moderate or severe pain on one or both sides of the head that recurs repeatedly, or is accompanied by nausea, vomiting, and sensitivity to light, sound, smell, or touch, which seriously affects the patient's life (Steiner TJ et al., J Neurol Neurosurg Psychiatry 2004, 75: 808-811). The World Health Organization (WHO) has listed migraine as one of the top ten most disabling diseases, compared with other people, patients with migraine are more likely to experience depression, anxiety, sleep disturbances, other pain, and fatigue. According to statistics, migraine affects 1.3 billion patients worldwide, about 11% of adults, among which female patients are three times more than male patients. There are about 40 million patients in the United States, about 8 million patients in Japan, and 13 million patients in China. In the United States, medical costs and lost productivity due to migraine are estimated to be $80 billion each year, a huge drain on resources. At present, the pathogenesis of migraine is still not very clear internationally. The more recognized one is the trigeminovascular reflex theory, which effectively combines nerves, blood vessels and neurotransmitters, better explains the pathogenesis of migraine, and has been widely accepted.

Currently, migraine treatment is clinically divided into symptomatic treatment and preventive treatment. The first-line treatment for symptomatic treatment is still the use of non-steroidal anti-inflammatory drugs, ergotamines or triptans. For severe patients, opioids and other drugs are even used in combination. Triptans are currently the first-line treatment for migraine, but some patients are insensitive to this class and the treatment effect is not obvious. In addition, triptans have side effects that cause cardiovascular risks. These problems also limit the use of triptans. Commonly used prescriptions for preventive treatment are anti-epileptic drugs, tricyclic antidepressants, and beta-blockers, only some of which have the effect of preventing migraines. Since these preventive drugs were originally used to treat other diseases, they are not specific to the prevention of migraines and have significant side effects, so they are not the preferred choice for preventive treatment of migraines. It is conceivable that in the field of migraine, there is still a need for continuous exploration to seek drugs with better therapeutic effects.

Calcitonin gene-related peptide (CGRP) is a neuropeptide containing 37 amino acid residues discovered by Amara et al. in 1982. It is widely present in the central and peripheral nervous systems, especially in the cell bodies and terminals of sensory neurons (Amara SG et al., Science 1982, 298: 240-244). Peripheral CGRP is in the dorsal root ganglion, and central CGRP is in the trigeminal ganglion. Both are synthesized in the cell bodies of sensory neurons and then rapidly transported to the central and peripheral ends. The central ends serve as afferent fibers of sensory neurons and are mainly responsible for the transmission of pain and temperature sensations. In the periphery, sensory nerve fibers containing CGRP are widely distributed in various tissues and organs, and are released through axon reflexes in response to a variety of stimuli.

CGRP is currently the most powerful endogenous vasodilator. In the field of pain, especially migraine, CGRP has become a research focus and hotspot. Several clinical studies have confirmed that during the onset of migraine, the level of CGRP in plasma increases, and the intensity and duration of migraine are positively correlated with the plasma CGRP level (Han TH et al., Arch Drug Inf 2010, 3: 55-62). In addition, Goadsby et al. found that during the onset of migraine, the CGRP level in the external jugular vein increased, but not in the cubital vein, indicating that CGRP is released intracranially during migraine (Goadsby PJ et al., Ann Neurol 1990, 28: 183-187). Animal studies have also found that CGRP released by trigeminal nerve activation can cause dilation of brain and meningeal blood vessels, release of inflammatory mediators by mast cells, and transmission of harmful biological information released by intracranial blood vessels to the central nervous system (Williamson D et al., Microsc Res Tech 2001, 53: 167-178). Various studies have shown that migraine is closely related to the abnormal release and increased level of CGRP.

The molecular weight of CGRP is about 3800 Da, and it is composed of 2800 base pairs. In its 37 amino acid sequences, the 2nd and 7th positions at the N-terminus are connected by a disulfide bond, and the C-terminus is a phenylalanine residue. These two structures are essential groups for the biological activity of CGRP. Currently, there are two known types of human CGRP: α-CGRP and β-CGRP, wherein α-CGRP is mainly expressed in the nervous system, such as in the hypothalamus, cerebellum, brainstem and trigeminal system, while β-CGRP is mainly expressed in the intestinal sensory system. α-CGRP is formed by splicing of the calcitonin (CT) gene, while β-CGRP is encoded by a separate gene. Although the two forms of CGRP differ by three amino acids, they have similar biological effects in the circulatory system circulation system (Edvinsson L, Expert Opinion on Therapeutic Targets 2007, 11: 1179-1188).

CGRP receptor belongs to G protein-coupled receptor, which is composed of 7 transmembrane protein complexes (calcitonin receptor like receptor, CLR), 1 transmembrane protein receptor activity modifying protein 1 (RAMP1) and 1 intracellular protein (receptor component protein, RCP) (Evans BN et al., J Biology Chem 2000, 275: 38-43). RAMP1 is a class of small transmembrane proteins that mediate the membrane translocation of CLR in the form of a molecular chaperone. RCP is a class of small polypeptides that mediate the transduction of downstream signals of CLR. At present, the mechanism of CGRP's involvement in migraine is not clear. Most scholars believe that CGRP, as a multifunctional neuropeptide, participates in the process of neurogenic inflammation, peripheral and central sensitization, and cortical spreading depression, thereby inducing migraine.

As more and more research is done on CGRP and its receptors, our understanding of it is also increasing day by day. It took 35 years from the first isolation of CGRP in 1983 to the approval of three CGRP monoclonal antibody drugs in the United States in 2018. Currently, there are four CGRP monoclonal antibody drugs on the market. In addition to CGRP monoclonal antibodies, the research and development of CGRP receptor antagonists has also attracted much attention. After all, small molecule compounds have obvious advantages in terms of the friendliness of drug delivery. So far, three small molecule CGRP receptor antagonists have been launched on the market.

There were surprises and setbacks in the development of CGRP receptor antagonists. Taking Olcegepant, Telcagepant, and MK-3207 as examples, although multiple clinical trials have confirmed the effectiveness of these drugs, these compounds had to be suspended due to severe adverse reactions such as liver toxicity in multiple patients during clinical applications. By continuously optimizing the screening to find more suitable compounds, fortunately, in December 2019, the FDA approved the marketing of Ubrogepant, a drug developed by Abbvie for the treatment of acute migraine. In February of the following year, Rimegepant, developed by Biohaven, was also successfully approved for the treatment of acute migraine. In May 2021, Rimegepant was approved to expand its indications for the preventive treatment of episodic migraine. In September 2021, Atogepant developed by Abbvie was approved by the FDA for the preventive treatment of episodic migraine. The successful launch of these three small molecule drugs has brought hope to migraine patients around the world, but they also have side effects such as constipation, nausea, and drowsiness, so it is necessary to seek safer and more effective CGRP small molecule drugs. Further CGRP modulators are disclosed in CN103328478A, US2013/231358 A1, WO2013/169567 A1 and CN101443323A.

### SUMMARY OF THE INVENTION

In view of the above technical problems, the present application provides a substituted piperidinecarboxamide azaindane derivative represented by general formula (I) or a stereoisomer, tautomer, deuterated derivative or pharmaceutically acceptable salt thereof: 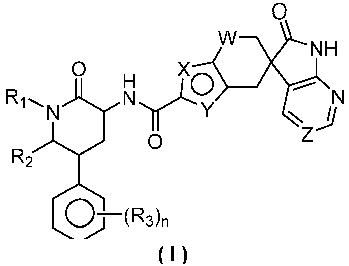 wherein:
R₁ is selected from a hydrogen atom, formyl, alkyl, cycloalkyl, heterocyclyl; wherein the alkyl, cycloalkyl, heterocyclyl is optionally substituted by one or more Rₐ substituents;
each Rₐ is the same or different and is independently selected from deuterium, tritium, halogen, amino, hydroxyl, cyano, alkoxy, alkyl, cycloalkyl, or heterocycloalkyl, wherein the amino, hydroxyl, cyano, alkoxy, alkyl, cycloalkyl, or heterocycloalkyl is optionally further substituted with one or more substituents selected from alkyl, haloalkyl, halogen, amino, hydroxyl, cyano or alkoxy;
R₂ is selected from alkyl, deuterated alkyl, aminoalkyl, haloalkyl, or hydroxyalkyl;
each R₃ is the same or different and is independently selected from halogen, amino, hydroxyl, cyano, alkoxy, alkyl, cycloalkyl, heterocyclyl, aryl, or heteroaryl;
X and Y are the same or different and are each independently selected from =CR₄-, =N-, -NR₅-, -O-, -S-, -S(O)- or -S(O)₂-;
R₄ and Rs are the same or different and are each independently selected from a hydrogen atom or alkyl;
W is selected from -CH₂- or a single bond;
Z is selected from =CH- or =N-; and
n is 0, 1, 2, 3, 4 or 5.

In a specific embodiment, R₂ is alkyl, and the remaining R₁, R₃, X, Y, W and Z are defined as above for the general formula (I).

In a specific embodiment, R₂ is C₁₋₆ alkyl, and the remaining R₁, R₃, X, Y, W and Z are defined as above for the general formula (I).

In a specific embodiment, R₂ is methyl, and the remaining R₁, R₃, X, Y, W and Z are defined as above for the general formula (I).

In a specific embodiment, R₁ is selected from alkyl or alkyl substituted by a Rₐ substituent, and the remaining R₂, R₃, X, Y, W and Z are defined as above for the general formula (I).

In a specific embodiment, R₁ is selected from C₁₋₆ alkyl or C₁₋₆ alkyl substituted by Rₐ substituent, and the remaining R₂, R₃, X, Y, W and Z are defined as above for the general formula (I).

In a specific embodiment, R₁ is selected from isopropyl, 2,2,2-trifluoroethyl, 2,2-difluoroethyl, 2-methylpropyl, 3,3,3-trifluoropropyl and 3,3,3-trifluoro-2-hydroxypropyl, and the remaining R₂, R₃, X, Y, W and Z are defined as above for the general formula (I).

In a specific embodiment, R₁ is 2,2,2-trifluoroethyl, and the remaining R₂, R₃, X, Y, W and Z are defined as above for the general formula (I).

In a specific embodiment, X is selected from =N-, -NR₅-, -O-, or -S-, and the remaining R₁, R₂, R₃, Y, W and Z are defined as above for the general formula (I).

In a specific embodiment, Y is selected from =CR₄-, -O-, -NR₅-, =N-, or -S-, and the remaining R₁, R₂, R₃, X, W and Z are defined as above for the general formula (I).

In a specific embodiment, R₄ or Rs is a hydrogen atom.

In a specific embodiment, R₃ is the same and is halogen, and the remaining R₁, R₂, X, Y, W and Z are defined as above for the general formula (I).

In a specific embodiment, R₃ is fluorine, n is 3, and the remaining R₁, R₂, X, Y, W and Z are defined as above for the general formula (I).

In a specific embodiment, W is a single bond, and the remaining R₁, R₂, R₃, X, Y and Z are defined as above for the general formula (I).

In a specific embodiment, Z is =CH, and the remaining R₁, R₂, R₃, X, Y and W are defined as above for the general formula (I).

In a specific embodiment, n is 0, and the remaining R₁, R₂, R₃, X, Y, W and Z are defined as above for the general formula (I).

A preferred embodiment of the present application provides the compound represented by the general formula (I) or a stereoisomer, tautomer, deuterated derivative or pharmaceutically acceptable salt thereof, which is a compound represented by general formula (II) or a stereoisomer, tautomer, deuterated derivative or pharmaceutically acceptable salt thereof: 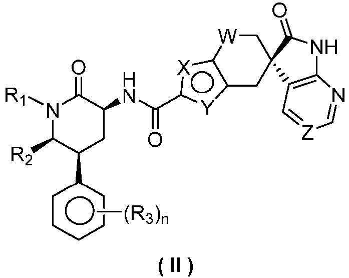 wherein: R₁, R₂, R₃, n, X, Y, W and Z are as defined in the general formula (I).

A preferred embodiment of the present application provides the compound represented by the general formula (II) or a stereoisomer, tautomer, deuterated derivative or pharmaceutically acceptable salt thereof, which is a compound represented by general formula (III) or a stereoisomer, tautomer, deuterated derivative or pharmaceutically acceptable salt thereof: 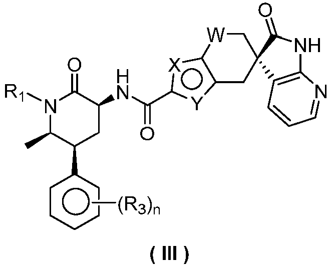 wherein: R₁, R₃, n, X, Y and W are as defined in the general formula (I).

A preferred embodiment of the present application provides the compound represented by the general formula (III) or a stereoisomer, tautomer, deuterated derivative or pharmaceutically acceptable salt thereof, which is a compound represented by general formula (IV) or a stereoisomer, tautomer, deuterated derivative or pharmaceutically acceptable salt thereof: 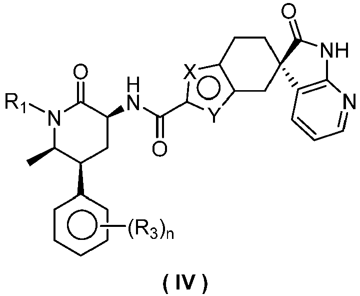 wherein: R₁, R₃, n, X and Y are as defined in the general formula (I).

A preferred embodiment of the present application provides the compound represented by the general formula (III) or a stereoisomer, tautomer, deuterated derivative or pharmaceutically acceptable salt thereof, which is a compound represented by general formula (V) or a stereoisomer, tautomer, deuterated derivative or pharmaceutically acceptable salt thereof: 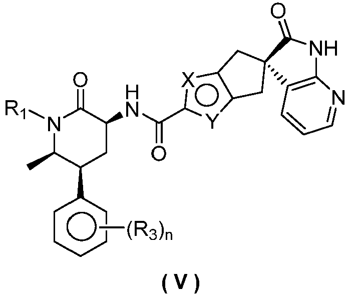 wherein: R₁, R₃, n, X and Y are as defined in the general formula (I).

A preferred embodiment of the present application provides the compound represented by the general formula (I), (II), (III), (IV) or (V) or a stereoisomer, tautomer, deuterated derivative or pharmaceutically acceptable salt thereof, wherein R₁ is selected from isopropyl, 2,2,2-trifluoroethyl, 2,2-difluoroethyl, 2-methylpropyl, 3,3,3-trifluoropropyl or 3,3,3-trifluoro-2-hydroxypropyl.

A preferred embodiment of the present application provides the compound represented by the general formula (I), (II), (III), (IV) or (V) or a stereoisomer, tautomer, deuterated derivative or pharmaceutically acceptable salt thereof, wherein R₁ is selected from 2,2,2-trifluoroethyl.

A preferred embodiment of the present application provides the compound represented by the general formula (I), (II), (III), (IV) or (V) or a stereoisomer, tautomer, deuterated derivative or pharmaceutically acceptable salt thereof, wherein R₃ is selected from fluorine, and n is selected from 3.

In a specific embodiment, X is selected from =N-, -NR₅-, -O-, or -S-; Y is selected from =CR₄-, -O-, -NR₅-, =N-, or -S-; W is selected from -CH₂- or a single bond, and the remaining R₁, R₂, R₃ and Z are defined as above for the general formula (I).

In a specific embodiment, X is selected from -NR₅-, -O-, or -S-; Y is selected from =CR₄-, -O-, -NR₅-, =N-, or -S-; W is selected from -CH₂- or a single bond, and the remaining R₁, R₂, R₃ and Z are defined as above for the general formula (I).

In a specific embodiment, X is selected from -O-, or -S-; Y is selected from =CR₄-, -O-, -NR₅-, =N-, or -S-; W is selected from -CH₂- or a single bond, and the remaining R₁, R₂, R₃ and Z are defined as above for the general formula (I).

In a specific embodiment, X is -S-; Y is selected from =CR₄-, -O-, -NR₅-, =N-, or -S-; W is selected from -CH₂- or a single bond, and the remaining R₁, R₂, R₃ and Z are defined as above for the general formula (I).

In a specific embodiment, X is selected from =N-, -NR₅-, -O-, or -S-; Y is selected from =CR₄-, -NR₅-, =N-, or -S-; W is selected from -CH₂- or a single bond, and the remaining R₁, R₂, R₃ and Z are defined as above for the general formula (I).

In a specific embodiment, X is selected from -NR₅-, -O-, or -S-; Y is selected from =CR₄-, -NR₅-, =N-, or -S-; W is selected from -CH₂- or a single bond, and the remaining R₁, R₂, R₃ and Z are defined as above for the general formula (I).

In a specific embodiment, X is selected from -O-, or -S-; Y is selected from =CR₄-, -NR₅-, =N-, or -S-; W is selected from -CH₂- or a single bond, and the remaining R₁, R₂, R₃ and Z are defined as above for the general formula (I).

In a specific embodiment, X is -S-; Y is selected from =CR₄-, -NR₅-, =N-, or -S-; W is selected from -CH₂- or a single bond, and the remaining R₁, R₂, R₃ and Z are defined as above for the general formula (I).

In a specific embodiment, X is selected from =N-, -NR₅-, -O-, or -S-; Y is selected from =CR₄-, =N-, or -S-; W is selected from -CH₂- or a single bond, and the remaining R₁, R₂, R₃ and Z are defined as above for the general formula (I).

In a specific embodiment, X is selected from -NR₅-, -O-, or -S-; Y is selected from =CR₄-, =N-, or -S-; W is selected from -CH₂- or a single bond, and the remaining R₁, R₂, R₃ and Z are defined as above for the general formula (I).

In a specific embodiment, X is selected from -O-, or -S-; Y is selected from =CR₄-, =N-, or -S-; W is selected from -CH₂- or a single bond, and the remaining R₁, R₂, R₃ and Z are defined as above for the general formula (I).

In a specific embodiment, X is -S-; Y is selected from =CR₄-, =N-, or -S-; W is selected from -CH₂- or a single bond, and the remaining R₁, R₂, R₃ and Z are defined as above for the general formula (I).

In a specific embodiment, X is selected from =N-, -NR₅-, -O-, or -S-; Y is selected from =CR₄-, or -S-; W is selected from -CH₂- or a single bond, and the remaining R₁, R₂, R₃ and Z are defined as above for the general formula (I).

In a specific embodiment, X is selected from -NR₅-, -O-, or -S-; Y is selected from =CR₄-, or -S-; W is selected from -CH₂- or a single bond, and the remaining R₁, R₂, R₃ and Z are defined as above for the general formula (I).

In a specific embodiment, X is selected from -O-, or -S-; Y is selected from =CR₄-, or -S-; W is selected from -CH₂- or a single bond, and the remaining R₁, R₂, R₃ and Z are defined as above for the general formula (I).

In a specific embodiment, X is -S-; Y is selected from =CR₄-, or -S-; W is selected from -CH₂- or a single bond, and the remaining R₁, R₂, R₃ and Z are defined as above for the general formula (I).

In a specific embodiment, X is selected from =N-, -NR₅-, -O-, or -S-; Y is =CR₄-; W is selected from -CH₂- or a single bond, and the remaining R₁, R₂, R₃ and Z are defined as above for the general formula (I).

In a specific embodiment, X is selected from -NR₅-, -O-, or -S-; Y is =CR₄-; W is selected from -CH₂- or a single bond, and the remaining R₁, R₂, R₃ and Z are defined as above for the general formula (I).

In a specific embodiment, X is selected from -O-, or -S-; Y is =CR₄-; W is selected from -CH₂- or a single bond, and the remaining R₁, R₂, R₃ and Z are defined as above for the general formula (I).

In a specific embodiment, X is -S-; Y is =CR₄-; W is selected from -CH₂- or a single bond, and the remaining R₁, R₂, R₃ and Z are defined as above for the general formula (I).

In a specific embodiment, X is selected from =N-, -NR₅-, -O-, or -S-; Y is selected from =CR₄-, -NR₅-, =N-, or -S-; W is -CH₂-, and the remaining R₁, R₂, R₃ and Z are defined as above for the general formula (I).

In a specific embodiment, X is selected from -NR₅-, -O-, or -S-; Y is selected from =CR₄-, -NR₅-, =N-, or -S-; W is -CH₂-, and the remaining R₁, R₂, R₃ and Z are defined as above for the general formula (I).

In a specific embodiment, X is selected from -O-, or -S-; Y is selected from =CR₄-, -NR₅-, =N-, or -S-; W is -CH₂-, and the remaining R₁, R₂, R₃ and Z are defined as above for the general formula (I).

In a specific embodiment, X is -S-; Y is selected from =CR₄-, -NR₅-, =N-, or -S-; W is -CH₂-, and the remaining R₁, R₂, R₃ and Z are defined as above for the general formula (I).

In a specific embodiment, X is selected from =N-, -NR₅-, -O-, or -S-; Y is selected from =CR₄-, =N-, or -S-; W is -CH₂-, and the remaining R₁, R₂, R₃ and Z are defined as above for the general formula (I).

In a specific embodiment, X is selected from -NR₅-, -O-, or -S-; Y is selected from =CR₄-, =N-, or -S-; W is -CH₂-, and the remaining R₁, R₂, R₃ and Z are defined as above for the general formula (I).

In a specific embodiment, X is selected from -O-, or -S-; Y is selected from =CR₄-, =N-, or -S-; W is -CH₂-, and the remaining R₁, R₂, R₃ and Z are defined as above for the general formula (I).

In a specific embodiment, X is -S-; Y is selected from =CR₄-, =N-, or -S-; W is -CH₂-, and the remaining R₁, R₂, R₃ and Z are defined as above for the general formula (I).

In a specific embodiment, X is selected from =N-, -NR₅-, -O-, or -S-; Y is selected from =CR₄-, or -S-; W is -CH₂-, and the remaining R₁, R₂, R₃ and Z are defined as above for the general formula (I).

In a specific embodiment, X is selected from -NR₅-, -O-, or -S-; Y is selected from =CR₄-, or -S-; W is -CH₂-, and the remaining R₁, R₂, R₃ and Z are defined as above for the general formula (I).

In a specific embodiment, X is selected from -O-, or -S-; Y is selected from =CR₄-, or -S-; W is -CH₂-, and the remaining R₁, R₂, R₃ and Z are defined as above for the general formula (I).

In a specific embodiment, X is -S-; Y is selected from =CR₄-, or -S-; W is -CH₂-, and the remaining R₁, R₂, R₃ and Z are defined as above for the general formula (I).

In a specific embodiment, X is selected from =N-, -NR₅-, -O-, or -S-; Y is =CR₄-; W is -CH₂-, and the remaining R₁, R₂, R₃ and Z are defined as above for the general formula (I).

In a specific embodiment, X is selected from -NR₅-, -O-, or -S-; Y is =CR₄-; W is -CH₂-, and the remaining R₁, R₂, R₃ and Z are defined as above for the general formula (I).

In a specific embodiment, X is selected from -O-, or -S-; Y is =CR₄-; W is -CH₂-, and the remaining R₁, R₂, R₃ and Z are defined as above for the general formula (I).

In a specific embodiment, X is -S-; Y is =CR₄-; W is -CH₂-, and the remaining R₁, R₂, R₃ and Z are defined as above for the general formula (I).

In a specific embodiment, X is selected from =N-, -NR₅-, -O-, or -S-; Y is selected from =CR₄-, -NR₅-, =N-, or -S-; W is a single bond, and the remaining R₁, R₂, R₃ and Z are defined as above for the general formula (I).

In a specific embodiment, X is selected from -NR₅-, -O-, or -S-; Y is selected from =CR₄-, -NR₅-, =N-, or -S-; W is a single bond, and the remaining R₁, R₂, R₃ and Z are defined as above for the general formula (I).

In a specific embodiment, X is selected from -O-, or -S-; Y is selected from =CR₄-, -NR₅-, =N-, or -S-; W is a single bond, and the remaining R₁, R₂, R₃ and Z are defined as above for the general formula (I) above.

In a specific embodiment, X is -S-; Y is selected from =CR₄-, -NR₅-, =N-, or -S-; W is a single bond, and the remaining R₁, R₂, R₃ and Z are defined as above for the general formula (I).

In a specific embodiment, X is selected from =N-, -NR₅-, -O-, or -S-; Y is selected from =CR₄-, =N-, or -S-; W is a single bond, and the remaining R₁, R₂, R₃ and Z are defined as above for the general formula (I).

In a specific embodiment, X is selected from -NR₅-, -O-, or -S-; Y is selected from =CR₄-, =N-, or -S-; W is a single bond, and the remaining R₁, R₂, R₃ and Z are defined as above for the general formula (I).

In a specific embodiment, X is selected from -O-, or -S-; Y is selected from =CR₄-, =N-, or -S-; W is a single bond, and the remaining R₁, R₂, R₃ and Z are defined as above for the general formula (I).

In a specific embodiment, X is -S-; Y is selected from =CR₄-, =N-, or -S-; W is a single bond, and the remaining R₁, R₂, R₃ and Z are defined as above for the general formula (I).

In a specific embodiment, X is selected from =N-, -NR₅-, -O-, or -S-; Y is selected from =CR₄-, or -S-; W is a single bond, and the remaining R₁, R₂, R₃ and Z are defined as above for the general formula (I).

In a specific embodiment, X is selected from -NR₅-, -O-, or -S-; Y is selected from =CR₄-, or -S-; W is a single bond, and the remaining R₁, R₂, R₃ and Z are defined as above for the general formula (I).

In a specific embodiment, X is selected from -O-, or -S-; Y is selected from =CR₄-, or -S-; W is a single bond, and the remaining R₁, R₂, R₃ and Z are defined as above for the general formula (I).

In a specific embodiment, X is -S-; Y is selected from =CR₄-, or -S-; W is a single bond, and the remaining R₁, R₂, R₃ and Z are defined as above for the general formula (I).

In a specific embodiment, X is selected from =N-, -NR₅-, -O-, or -S-; Y is =CR₄-; W is a single bond, and the remaining R₁, R₂, R₃ and Z are defined as above for the general formula (I).

In a specific embodiment, X is selected from -NR₅-, -O-, or -S-; Y is =CR₄-; W is a single bond, and the remaining R₁, R₂, R₃ and Z are defined as above for the general formula (I).

In a specific embodiment, X is selected from -O-, or -S-; Y is =CR₄-; W is a single bond, and the remaining R₁, R₂, R₃ and Z are defined as above for the general formula (I).

In a specific embodiment, X is -S-; Y is =CR₄-; W is a single bond, and the remaining R₁, R₂, R₃ and Z are defined as above for the general formula (I).

In a specific embodiment, X is selected from =N-, -NR₅-, -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 6.

In a specific embodiment, X is -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 6.

In a specific embodiment, X is -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 6.

In a specific embodiment, X is -S-; Y is selected from =CR₄-, or -S-; W is -CH₂-, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is =CR₄-; W is -CH₂-, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is =CR₄-; W is -CH₂-, and the remaining R₁, R₂, R₃ and Z are defined as above for the general formula (I), wherein the substitution position of R₃ is 2, 3, or 6.

In a specific embodiment, X is selected from -O-, or -S-; Y is =CR₄-; W is -CH₂-, and the remaining R₁, R₂, R₃ and Z are defined as above for the general formula (I), wherein the substitution position of R₃ is 2, 3, or 6.

In a specific embodiment, X is -S-; Y is =CR₄-; W is -CH₂-, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 6.

In a specific embodiment, X is -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 6.

In a specific embodiment, X is -S-; Y is selected from =CR₄-, =N-, or -S-; W is a single bond, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is selected from =CR₄-, or -S-; W is a single bond, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is selected from =CR₄-, or -S-; W is a single bond, and the remaining R₁, R₂, R₃ and Z are defined as above for the general formula (I), wherein the substitution position of R₃ is 2, 3, or 6.

In a specific embodiment, X is selected from -O-, or -S-; Y is selected from =CR₄-, or -S-; W is a single bond, and the remaining R₁, R₂, R₃ and Z are defined as above for the general formula (I), wherein the substitution position of R₃ is 2, 3, or 6.

In a specific embodiment, X is -S-; Y is selected from =CR₄-, or -S-; W is a single bond, and the remaining R₁, R₂, R₃ and Z are defined as above for the general formula (I), wherein the substitution position of R₃ is 2, 3, or 6.

In a specific embodiment, X is selected from =N-, -NR₅-, -O-, or -S-; Y is =CR₄-; W is a single bond, and the remaining R₁, R₂, R₃ and Z are defined as above for the general formula (I), wherein the substitution position of R₃ is 2, 3, or 6.

In a specific embodiment, X is selected from -NR₅-, -O-, or -S-; Y is =CR₄-; W is a single bond, and the remaining R₁, R₂, R₃ and Z are defined as above for the general formula (I), wherein the substitution position of R₃ is 2, 3, or 6.

In a specific embodiment, X is selected from -O-, or -S-; Y is =CR₄-; W is a single bond, and the remaining R₁, R₂, R₃ and Z are defined as above for the general formula (I), wherein the substitution position of R₃ is 2, 3, or 6.

In a specific embodiment, X is -S-; Y is =CR₄-; W is a single bond, and the remaining R₁, R₂, R₃ and Z are defined as above for the general formula (I), wherein the substitution positions of R₃ are 2, 3, or 6.

In a specific embodiment, X is selected from =N-, -NR₅-, -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 5.

In a specific embodiment, X is -S-; Y is selected from =CR₄-, -NR₅-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 5.

In a specific embodiment, X is -S-; Y is selected from =CR₄-, =N-, or -S-; W is -CH₂-, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, or -S-; W is -CH₂-, and the remaining R₁, R₂, R₃ and Z are defined as above for the general formula (I), wherein the substitution position of R₃ is 2, 3, or 5.

In a specific embodiment, X is -S-; Y is selected from =CR₄-, or -S-; W is -CH₂-, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is =CR₄-; W is -CH₂-, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is =CR₄-; W is -CH₂-, and the remaining R₁, R₂, R₃ and Z are defined as above for the general formula (I), wherein the substitution position of R₃ is 2, 3, or 5.

In a specific embodiment, X is selected from -O-, or -S-; Y is =CR₄-; W is -CH₂-, and the remaining R₁, R₂, R₃ and Z are defined as above for the general formula (I), wherein the substitution position of R₃ is 2, 3, or 5.

In a specific embodiment, X is -S-; Y is =CR₄-; W is -CH₂-, and the remaining R₁, R₂, R₃ and Z are defined as above for the general formula (I), wherein the substitution positions of R₃ are 2, 3, and 5.

In a specific embodiment, X is selected from =N-, -NR₅-, -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 5.

In a specific embodiment, X is -S-; Y is selected from =CR₄-, -NR₅-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, =N-, or -S-; W is a single bond, and the remaining R₁, R₂, R₃ and Z are defined as above for the general formula (I), wherein the substitution position of R₃ is 2, 3, or 5.

In a specific embodiment, X is -S-; Y is selected from =CR₄-, =N-, or -S-; W is a single bond, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is selected from =CR₄-, or -S-; W is a single bond, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is selected from =CR₄-, or -S-; W is a single bond, and the remaining R₁, R₂, R₃ and Z are defined as above for the general formula (I), wherein the substitution position of R₃ is 2, 3, or 5.

In a specific embodiment, X is selected from -O-, or -S-; Y is selected from =CR₄-, or -S-; W is a single bond, and the remaining R₁, R₂, R₃ and Z are defined as above for the general formula (I), wherein the substitution position of R₃ is 2, 3, or 5.

In a specific embodiment, X is -S-; Y is selected from =CR₄-, or -S-; W is a single bond, and the remaining R₁, R₂, R₃ and Z are defined as above for the general formula (I), wherein the substitution position of R₃ is 2, 3, or 5.

In a specific embodiment, X is selected from =N-, -NR₅-, -O-, or -S-; Y is =CR₄-; W is a single bond, and the remaining R₁, R₂, R₃ and Z are defined as above for the general formula (I), wherein the substitution position of R₃ is 2, 3, or 5.

In a specific embodiment, X is selected from -NR₅-, -O-, or -S-; Y is =CR₄-; W is a single bond, and the remaining R₁, R₂, R₃ and Z are defined as above for the general formula (I), wherein the substitution position of R₃ is 2, 3, or 5.

In a specific embodiment, X is selected from -O-, or -S-; Y is =CR₄-; W is a single bond, and the remaining R₁, R₂, R₃ and Z are defined as above for the general formula (I), wherein the substitution position of R₃ is 2, 3, or 5.

In a specific embodiment, X is -S-; Y is =CR₄-; W is a single bond, and the remaining R₁, R₂, R₃ and Z are defined as above for the general formula (I), wherein the substitution position of R₃ is 2, 3, or 5.

In a specific embodiment, R₂ is alkyl, and R₁ is selected from an alkyl or alkyl substituted by a Rₐ substituent.

In a specific embodiment, R₂ is methyl, and R₁ is selected from alkyl or alkyl substituted by a Rₐ substituent.

In a specific embodiment, R₂ is methyl, and R₁ is selected from C₁₋₆ alkyl or C₁₋₆ alkyl substituted by a Rₐ substituent.

In a specific embodiment, R₂ is methyl, and R₁ is C₁₋₆ alkyl substituted by one or more Rₐ substituents.

In a specific embodiment, R₂ is methyl, R₁ is C₁₋₆ alkyl substituted by one or more Rₐ substituents, and each Rₐ is independently selected from halogen.

In a specific embodiment, R₂ is methyl, R₁ is C₁₋₆ alkyl substituted by one or more Rₐ substituents, and Rₐ are each independently selected from fluorine, chlorine, or bromine.

In a specific embodiment, R₂ is methyl, R₁ is C₁₋₆ alkyl substituted by one or more Rₐ substituents, and Rₐ is fluorine.

In a specific embodiment, R₂ is methyl, and R₁ is selected from isopropyl, 2,2,2-trifluoroethyl, 2,2-difluoroethyl, 2-methylpropyl, 3,3,3-trifluoropropyl or 3,3,3-trifluoro-2-hydroxypropyl.

In a specific embodiment, R₂ is methyl and R₁ is 2,2,2-trifluoroethyl.

In a specific embodiment, R₂ is methyl, R₁ is 2,2,2-trifluoroethyl, and R₃ are the same or different, and are each independently selected from halogen, amino, hydroxyl, cyano, alkoxy, alkyl, cycloalkyl, heterocyclyl, aryl, or heteroaryl.

In a specific embodiment, R₂ is methyl, R₁ is 2,2,2-trifluoroethyl, and R₃ are the same and are all halogen.

In a specific embodiment, R₂ is methyl, R₁ is 2,2,2-trifluoroethyl, and R₃ is fluorine.

In a specific embodiment, R₂ is methyl, R₁ is 2,2,2-trifluoroethyl, R₃ is fluorine, and n is 3.

In a specific embodiment, R₂ is methyl, R₁ is 2,2,2-trifluoroethyl, and n is 0.

In a specific embodiment, R₂ is methyl, R₁ is 2,2,2-trifluoroethyl, R₃ is fluorine, n is 3, and Z is selected from =CH- or =N-.

In a specific embodiment, R₂ is methyl, R₁ is 2,2,2-trifluoroethyl, n is 0, and Z is selected from =CH- or =N-.

In a specific embodiment, R₂ is methyl, R₁ is 2,2,2-trifluoroethyl, R₃ is fluorine, n is 3, and Z is =CH.

In a specific embodiment, R₂ is methyl, R₁ is 2,2,2-trifluoroethyl, n is 0, and Z is =CH.

In a specific embodiment, R₂ is methyl, R₁ is 2,2,2-trifluoroethyl, R₃ is fluorine, n is 3, Z is =CH, and W is -CH₂-.

In a specific embodiment, R₂ is methyl, R₁ is 2,2,2-trifluoroethyl, n is 0, Z is =CH, and W is -CH₂-.

In a specific embodiment, R₂ is methyl, R₁ is 2,2,2-trifluoroethyl, R₃ is fluorine, n is 3, Z is =CH, and W is a single bond.

In a specific embodiment, R₂ is methyl, R₁ is 2,2,2-trifluoroethyl, n is 0, Z is =CH, and W is a single bond.

In a specific embodiment, R₂ is methyl, R₁ is 2,2,2-trifluoroethyl, R₃ is fluorine, n is 3, Z is =CH, W is -CH₂-, and X is selected from =N-, -NR₅-, -O-, or -S-.

In a specific embodiment, R₂ is methyl, R₁ is 2,2,2-trifluoroethyl, n is 0, Z is =CH, W is -CH₂-, and X is selected from =N-, -NR₅-, -O-, or -S-.

In a specific embodiment, R₂ is methyl, R₁ is 2,2,2-trifluoroethyl, R₃ is fluorine, n is 3, Z is =CH, W is a single bond, and X is selected from =N-, -NR₅-, -O-, or -S-.

In a specific embodiment, R₂ is methyl, R₁ is 2,2,2-trifluoroethyl, n is 0, Z is =CH, W is a single bond, and X is selected from =N-, -NR₅-, -O-, or -S-.

In a specific embodiment, R₂ is methyl, R₁ is 2,2,2-trifluoroethyl, R₃ is fluorine, n is 3, Z is =CH, W is -CH₂-, and X is selected from -NR₅-, -O-, or -S-.

In a specific embodiment, R₂ is methyl, R₁ is 2,2,2-trifluoroethyl, n is 0, Z is =CH, W is -CH₂-, and X is selected from -NR₅-, -O-, or -S-.

In a specific embodiment, R₂ is methyl, R₁ is 2,2,2-trifluoroethyl, R₃ is fluorine, n is 3, Z is =CH, W is a single bond, and X is selected from -NR₅-, -O-, or -S-.

In a specific embodiment, R₂ is methyl, R₁ is 2,2,2-trifluoroethyl, n is 0, Z is =CH, W is a single bond, and X is selected from -NR₅-, -O-, or -S-.

In a specific embodiment, R₂ is methyl, R₁ is 2,2,2-trifluoroethyl, R₃ is fluorine, n is 3, Z is =CH, W is -CH₂-, and X is selected from -O- or -S-.

In a specific embodiment, R₂ is methyl, R₁ is 2,2,2-trifluoroethyl, n is 0, Z is =CH, W is -CH₂-, and X is selected from -O- or -S-.

In a specific embodiment, R₂ is methyl, R₁ is 2,2,2-trifluoroethyl, R₃ is fluorine, n is 3, Z is =CH, W is a single bond, and X is selected from -O- or -S-.

In a specific embodiment, R₂ is methyl, R₁ is 2,2,2-trifluoroethyl, n is 0, Z is =CH, W is a single bond, and X is selected from -O-, or -S-.

In a specific embodiment, R₂ is methyl, R₁ is 2,2,2-trifluoroethyl, R₃ is fluorine, n is 3, Z is =CH, W is -CH₂-, and X is -S-.

In a specific embodiment, R₂ is methyl, R₁ is 2,2,2-trifluoroethyl, n is 0, Z is =CH, W is -CH₂-, and X is -S-.

In a specific embodiment, R₂ is methyl, R₁ is 2,2,2-trifluoroethyl, R₃ is fluorine, n is 3, Z is =CH, W is a single bond, and X is -S-.

In a specific embodiment, R₂ is methyl, R₁ is 2,2,2-trifluoroethyl, n is 0, Z is =CH, W is a single bond, and X is -S-.

In a specific embodiment, R₂ is methyl, R₁ is 2,2,2-trifluoroethyl, R₃ is fluorine, n is 3, Z is =CH, W is -CH₂-, and X is -O-.

In a specific embodiment, R₂ is methyl, R₁ is 2,2,2-trifluoroethyl, n is 0, Z is =CH, W is -CH₂-, and X is -O-.

In a specific embodiment, R₂ is methyl, R₁ is 2,2,2-trifluoroethyl, R₃ is fluorine, n is 3, Z is =CH, W is a single bond, and X is -O-.

In a specific embodiment, R₂ is methyl, R₁ is 2,2,2-trifluoroethyl, n is 0, Z is =CH, W is a single bond, and X is -O-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is -CH₂-, X is selected from =N-, -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is selected from =N-, -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is -CH₂-, X is selected from -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is selected from -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is -CH₂-, X is selected from -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is -CH₂-, X is selected from -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is a single bond, X is selected from -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is selected from -O-, or -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is -CH₂-, X is -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is -CH₂-, X is -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is a single bond, X is -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is -S-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is -CH₂-, X is -O-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is -CH₂-, X is -O-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is a single bond, X is -O-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is -O-, and Y is selected from =CR₄-, -O-, -NR₅-, =N-, or -S-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, and Y is selected from =CR₄-, =N-, or -S-.

In a specific embodiment, R₂ is methyl, R₁ is 2,2,2-trifluoroethyl, n is 0, Z is =CH, W is -CH₂-, X is selected from =N-, -NR₅-, -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, and Y is selected from =CR₄-, =N-, or -S-.

In a specific embodiment, R₂ is methyl, R₁ is 2,2,2-trifluoroethyl, n is 0, Z is =CH, W is a single bond, X is selected from =N-, -NR₅-, -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, and Y is selected from =CR₄-, =N-, or -S-.

In a specific embodiment, R₂ is methyl, R₁ is 2,2,2-trifluoroethyl, n is 0, Z is =CH, W is -CH₂-, X is selected from -NR₅-, -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, and Y is selected from =CR₄-, -O-, -NR₅-, =N-, or -S-.

In a specific embodiment, R₂ is methyl, R₁ is 2,2,2-trifluoroethyl, n is 0, Z is =CH, W is a single bond, X is selected from -NR₅-, -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is -CH₂-, X is selected from -O-, or -S-, and Y is selected from =CR₄-, =N-, or -S-.

In a specific embodiment, R₂ is methyl, R₁ is 2,2,2-trifluoroethyl, n is 0, Z is =CH, W is -CH₂-, X is selected from -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is a single bond, X is selected from -O-, or -S-, and Y is selected from =CR₄-, =N-, or -S-.

In a specific embodiment, R₂ is methyl, R₁ is 2,2,2-trifluoroethyl, n is 0, Z is =CH, W is a single bond, X is selected from -O-, or -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is -CH₂-, X is -S-, and Y is selected from =CR₄-, =N-, or -S-.

In a specific embodiment, R₂ is methyl, R₁ is 2,2,2-trifluoroethyl, n is 0, Z is =CH, W is -CH₂-, X is -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is a single bond, X is -S-, and Y is selected from =CR₄-, =N-, and -S-.

In a specific embodiment, R₂ is methyl, R₁ is 2,2,2-trifluoroethyl, n is 0, Z is =CH, W is a single bond, X is -S-, and Y is selected from =CR₄-, =N-, or -S-.

In a specific embodiment, R₂ is methyl, R₁ is 2,2,2-trifluoroethyl, R₃ is fluorine, n is 3, Z is =CH, W is -CH₂-, X is -O-, and Y is selected from =CR₄-, =N-, or -S-.

In a specific embodiment, R₂ is methyl, R₁ is 2,2,2-trifluoroethyl, n is 0, Z is =CH, W is -CH₂-, X is -O-, and Y is selected from =CR₄-, =N-, or -S-.

In a specific embodiment, R₂ is methyl, R₁ is 2,2,2-trifluoroethyl, R₃ is fluorine, n is 3, Z is =CH, W is a single bond, X is -O-, and Y is selected from =CR₄-, =N-, or -S-.

In a specific embodiment, R₂ is methyl, R₁ is 2,2,2-trifluoroethyl, n is 0, Z is =CH, W is a single bond, X is -O-, and Y is selected from =CR₄-, =N-, or -S-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, and Y is selected from =CR₄-, or =N-.

In a specific embodiment, R₂ is methyl, R₁ is 2,2,2-trifluoroethyl, n is 0, Z is =CH, W is -CH₂-, X is selected from =N-, -NR₅-, -O-, or -S-, and Y is selected from =CR₄-, or =N-.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, and Y is selected from =CR₄-, or =N-.

In a specific embodiment, R₂ is methyl, R₁ is 2,2,2-trifluoroethyl, n is 0, Z is =CH, W is a single bond, X is selected from =N-, -NR₅-, -O-, or -S-, and Y is selected from =CR₄-, or =N-.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, and Y is selected from =CR₄-, or =N-.

In a specific embodiment, R₂ is methyl, R₁ is 2,2,2-trifluoroethyl, n is 0, Z is =CH, W is -CH₂-, X is selected from -NR₅-, -O-, or -S-, and Y is selected from =CR₄-, or =N-.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, and Y is selected from =CR₄-, -O-, -NR5-, or =N-.

In a specific embodiment, R₂ is methyl, R₁ is 2,2,2-trifluoroethyl, n is 0, Z is =CH, W is a single bond, X is selected from -NR₅-, -O-, or -S-, and Y is selected from =CR4-, or =N-.

In a specific embodiment, R₂ is methyl, R₁ is 2,2,2-trifluoroethyl, R₃ is fluorine, n is 3, Z is =CH, W is -CH₂-, X is selected from -O-, or -S-, and Y is selected from =CR4-, or =N-.

In a specific embodiment, R₂ is methyl, R₁ is 2,2,2-trifluoroethyl, n is 0, Z is =CH, W is -CH₂-, X is selected from -O-, or -S-, and Y is selected from =CR4-, or =N-.

In a specific embodiment, R₂ is methyl, R₁ is 2,2,2-trifluoroethyl, R₃ is fluorine, n is 3, Z is =CH, W is a single bond, X is selected from -O-, or -S-, and Y is selected from =CR4-, or =N-.

In a specific embodiment, R₂ is methyl, R₁ is 2,2,2-trifluoroethyl, n is 0, Z is =CH, W is a single bond, X is selected from -O-, or -S-, and Y is selected from =CR4-, or =N-.

In a specific embodiment, R₂ is methyl, R₁ is 2,2,2-trifluoroethyl, R₃ is fluorine, n is 3, Z is =CH, W is -CH₂-, X is -S-, and Y is selected from =CR4-, or =N-.

In a specific embodiment, R₂ is methyl, R₁ is 2,2,2-trifluoroethyl, n is 0, Z is =CH, W is -CH₂-, X is -S-, and Y is selected from =CR4-, or =N-.

In a specific embodiment, R₂ is methyl, R₁ is 2,2,2-trifluoroethyl, R₃ is fluorine, n is 3, Z is =CH, W is a single bond, X is -S-, and Y is selected from =CR4-, or =N-.

In a specific embodiment, R₂ is methyl, R₁ is 2,2,2-trifluoroethyl, n is 0, Z is =CH, W is a single bond, X is -S-, and Y is selected from =CR4-, or =N-.

In a specific embodiment, R₂ is methyl, R₁ is 2,2,2-trifluoroethyl, R₃ is fluorine, n is 3, Z is =CH, W is -CH₂-, X is -O-, and Y is selected from =CR4-, or =N-.

In a specific embodiment, R₂ is methyl, R₁ is 2,2,2-trifluoroethyl, n is 0, Z is =CH, W is -CH₂-, X is -O-, and Y is selected from =CR4-, or =N-.

In a specific embodiment, R₂ is methyl, R₁ is 2,2,2-trifluoroethyl, R₃ is fluorine, n is 3, Z is =CH, W is a single bond, X is -O-, and Y is selected from =CR4-, or =N-.

In a specific embodiment, R₂ is methyl, R₁ is 2,2,2-trifluoroethyl, n is 0, Z is =CH, W is a single bond, X is -O-, and Y is selected from =CR4-, or =N-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, and Y is =N-.

In a specific embodiment, R₂ is methyl, R₁ is 2,2,2-trifluoroethyl, n is 0, Z is =CH, W is -CH₂-, X is selected from =N-, -NR₅-, -O-, or -S-, and Y is =N-.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, and Y is =N-.

In a specific embodiment, R₂ is methyl, R₁ is 2,2,2-trifluoroethyl, n is 0, Z is =CH, W is a single bond, X is selected from =N-, -NR₅-, -O-, or -S-, and Y is =N-.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, and Y is =N-.

In a specific embodiment, R₂ is methyl, R₁ is 2,2,2-trifluoroethyl, n is 0, Z is =CH, W is -CH₂-, X is selected from -NR₅-, -O-, or -S-, and Y is =N-.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, and Y is =N-.

In a specific embodiment, R₂ is methyl, R₁ is 2,2,2-trifluoroethyl, n is 0, Z is =CH, W is a single bond, X is selected from -NR₅-, -O-, or -S-, and Y is =N-.

In a specific embodiment, R₂ is methyl, R₁ is 2,2,2-trifluoroethyl, R₃ is fluorine, n is 3, Z is =CH, W is -CH₂-, X is selected from -O-, or -S-, and Y is =N-.

In a specific embodiment, R₂ is methyl, R₁ is 2,2,2-trifluoroethyl, n is 0, Z is =CH, W is -CH₂-, X is selected from -O-, or -S-, and Y is =N-.

In a specific embodiment, R₂ is methyl, R₁ is 2,2,2-trifluoroethyl, R₃ is fluorine, n is 3, Z is =CH, W is a single bond, X is selected from -O-, or -S-, and Y is =N-.

In a specific embodiment, R₂ is methyl, R₁ is 2,2,2-trifluoroethyl, n is 0, Z is =CH, W is a single bond, X is selected from -O-, or -S-, and Y is =N-.

In a specific embodiment, R₂ is methyl, R₁ is 2,2,2-trifluoroethyl, R₃ is fluorine, n is 3, Z is =CH, W is -CH₂-, X is -S-, and Y is =N-.

In a specific embodiment, R₂ is methyl, R₁ is 2,2,2-trifluoroethyl, n is 0, Z is =CH, W is -CH₂-, X is selected from -S-, and Y is =N-.

In a specific embodiment, R₂ is methyl, R₁ is 2,2,2-trifluoroethyl, R₃ is fluorine, n is 3, Z is =CH, W is a single bond, X is -S-, and Y is =N-.

In a specific embodiment, R₂ is methyl, R₁ is 2,2,2-trifluoroethyl, n is 0, Z is =CH, W is a single bond, X is -S-, and Y is =N-.

In a specific embodiment, R₂ is methyl, R₁ is 2,2,2-trifluoroethyl, R₃ is fluorine, n is 3, Z is =CH, W is -CH₂-, X is -O-, and Y is =N-.

In a specific embodiment, R₂ is methyl, R₁ is 2,2,2-trifluoroethyl, n is 0, Z is =CH, W is -CH₂-, X is selected from -O-, and Y is =N-.

In a specific embodiment, R₂ is methyl, R₁ is 2,2,2-trifluoroethyl, R₃ is fluorine, n is 3, Z is =CH, W is a single bond, X is -O-, and Y is =N-.

In a specific embodiment, R₂ is methyl, R₁ is 2,2,2-trifluoroethyl, n is 0, Z is =CH, W is a single bond, X is -O-, and Y is =N-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, and Y is =CR₄-.

In a specific embodiment, R₂ is methyl, R₁ is 2,2,2-trifluoroethyl, n is 0, Z is =CH, W is -CH₂-, X is selected from =N-, -NR₅-, -O-, or -S-, and Y is =CR₄-.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, and Y is =CR₄-.

In a specific embodiment, R₂ is methyl, R₁ is 2,2,2-trifluoroethyl, n is 0, Z is =CH, W is a single bond, X is selected from =N-, -NR₅-, -O-, or -S-, and Y is =CR₄-.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, and Y is =CR₄-.

In a specific embodiment, R₂ is methyl, R₁ is 2,2,2-trifluoroethyl, n is 0, Z is =CH, W is -CH₂-, X is selected from -NR₅-, -O-, or -S-, and Y is =CR₄-.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, and Y=CR₄-.

In a specific embodiment, R₂ is methyl, R₁ is 2,2,2-trifluoroethyl, n is 0, Z is =CH, W is a single bond, X is selected from -NR₅-, -O-, or -S-, and Y is =CR₄-.

In a specific embodiment, R₂ is methyl, R₁ is 2,2,2-trifluoroethyl, R₃ is fluorine, n is 3, Z is =CH, W is -CH₂-, X is selected from -O-, or -S-, and Y is =CR₄-.

In a specific embodiment, R₂ is methyl, R₁ is 2,2,2-trifluoroethyl, n is 0, Z is =CH, W is -CH₂-, X is selected from -O-, or -S-, and Y is =CR₄-.

In a specific embodiment, R₂ is methyl, R₁ is 2,2,2-trifluoroethyl, R₃ is fluorine, n is 3, Z is =CH, W is a single bond, X is selected from -O-, or -S-, and Y is =CR₄-.

In a specific embodiment, R₂ is methyl, R₁ is 2,2,2-trifluoroethyl, n is 0, Z is =CH, W is a single bond, X is selected from -O-, or -S-, and Y is =CR₄-.

In a specific embodiment, R₂ is methyl, R₁ is 2,2,2-trifluoroethyl, R₃ is fluorine, n is 3, Z is =CH, W is -CH₂-, X is -S-, and Y is =CR₄-.

In a specific embodiment, R₂ is methyl, R₁ is 2,2,2-trifluoroethyl, n is 0, Z is =CH, W is -CH₂-, X is -S-, and Y is =CR₄-.

In a specific embodiment, R₂ is methyl, R₁ is 2,2,2-trifluoroethyl, R₃ is fluorine, n is 3, Z is =CH, W is a single bond, X is -S-, and Y is =CR₄-.

In a specific embodiment, R₂ is methyl, R₁ is 2,2,2-trifluoroethyl, n is 0, Z is =CH, W is a single bond, X is -S-, and Y is =CR₄-.

In a specific embodiment, R₂ is methyl, R₁ is 2,2,2-trifluoroethyl, R₃ is fluorine, n is 3, Z is =CH, W is -CH₂-, X is -O-, and Y is =CR₄-.

In a specific embodiment, R₂ is methyl, R₁ is 2,2,2-trifluoroethyl, n is 0, Z is =CH, W is -CH₂-, X is selected from -O-, and Y is =CR₄-.

In a specific embodiment, R₂ is methyl, R₁ is 2,2,2-trifluoroethyl, R₃ is fluorine, n is 3, Z is =CH, W is a single bond, X is -O-, and Y is =CR₄-.

In a specific embodiment, R₂ is methyl, R₁ is 2,2,2-trifluoroethyl, n is 0, Z is =CH, W is a single bond, X is -O-, and Y is =CR₄-.

In a specific embodiment, R₂ is methyl, R₁ is 2,2,2-trifluoroethyl, R₃ is fluorine, n is 3, Z is =CH, W is -CH₂-, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, n is 0, Z is =CH, W is -CH₂-, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is a single bond, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, n is 0, Z is =CH, W is a single bond, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is -CH₂-, X is selected from -NR₅-, -O-, or -S-, Y is =CR₄-, and R₄ is a hydrogen atom.

In a specific embodiment, R₂ is methyl, R₁ is 2,2,2-trifluoroethyl, n is 0, Z is =CH, W is -CH₂-, X is selected from -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is a single bond, X is selected from -NR₅-, -O-, or -S-, Y=CR₄-, and R₄ is a hydrogen atom.

In a specific embodiment, R₂ is methyl, R₁ is 2,2,2-trifluoroethyl, n is 0, Z is =CH, W is a single bond, X is selected from -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is -CH₂-, X is selected from -O-, or -S-, Y is =CR₄-, and R₄ is a hydrogen atom.

In a specific embodiment, R₂ is methyl, R₁ is 2,2,2-trifluoroethyl, n is 0, Z is =CH, W is -CH₂-, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is a single bond, X is selected from -O-, or -S-, Y is =CR₄-, and R₄ is a hydrogen atom.

In a specific embodiment, R₂ is methyl, R₁ is 2,2,2-trifluoroethyl, n is 0, Z is =CH, W is a single bond, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is -CH₂-, X is -S-, Y is =CR₄-, and R₄ is a hydrogen atom.

In a specific embodiment, R₂ is methyl, R₁ is 2,2,2-trifluoroethyl, n is 0, Z is =CH, W is -CH₂-, X is -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is a single bond, X is -S-, Y is =CR₄-, and R₄ is a hydrogen atom.

In a specific embodiment, R₂ is methyl, R₁ is 2,2,2-trifluoroethyl, n is 0, Z is =CH, W is a single bond, X is -S-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is -CH₂-, X is -O-, Y is =CR₄-, and R₄ is a hydrogen atom.

In a specific embodiment, R₂ is methyl, R₁ is 2,2,2-trifluoroethyl, n is 0, Z is =CH, W is -CH₂-, X is -O-, Y is =CR₄-, and R₄ is a hydrogen atom.

In a specific embodiment, R₂ is methyl, R₁ is 2,2,2-trifluoroethyl, R₃ is fluorine, n is 3, Z is =CH, W is a single bond, X is -O-, Y is =CR₄-, and R₄ is a hydrogen atom.

In a specific embodiment, R₂ is methyl, R₁ is 2,2,2-trifluoroethyl, n is 0, Z is =CH, W is a single bond, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is a single bond, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is selected from -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is a single bond, X is selected from -NR₅-, -O-, or -S-, Y=CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is a single bond, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is a single bond, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is a single bond, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is -CH₂-, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is a single bond, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is -CH₂-, X is selected from -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is a single bond, X is selected from -NR₅-, -O-, or -S-, Y=CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is -CH₂-, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6, Z is =CH, W is a single bond, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6 or 2, 3, and 5; Z is =CH, W is -CH₂-, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is a single bond, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is -CH₂-, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 6; Z is =CH, W is a single bond, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is -CH₂-, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is a single bond, X is selected from =N-, -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is -CH₂-, X is selected from -NR₅-, -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is a single bond, X is selected from -NR₅-, -O-, or -S-, Y=CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is -CH₂-, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is a single bond, X is selected from -O-, or -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is -CH₂-, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is a single bond, X is -S-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is -CH₂-, X is -O-, Y is =CR₄-, and R₄ is a hydrogen atom.

In a specific embodiment, R₂ is methyl, R₁ is 2,2,2-trifluoroethyl, R₃ is fluorine, n is 3, wherein the three fluorine substitution positions are 2, 3, and 5; Z is =CH, W is a single bond, X is -O-, Y is =CR₄-, and R₄ is a hydrogen atom.

In the above specific embodiments, wherein: the definitions of R₁, R₂, R₃, X, Y, W and Z not mentioned are as described in the general formula (I).

In a preferred embodiment of the present application, the compound represented by the general formula (I) is selected from:

| **Compound number** | **Structure** | **Name** |
|---|---|---|
| Example 1 | 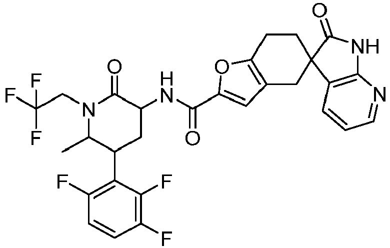 | N-(6-methyl-2-oxo-1-(2,2,2-trifluoroethyl)-5-(2,3,6-tri fluorophenyl)piperidin-3-yl)-2'-oxo-1',2',6,7-tetrahydr o-4H-spiro[benzofuran-5,3'-pyrrolo[2,3-b]pyridine]-2-carboxamide |
| Example 1A | 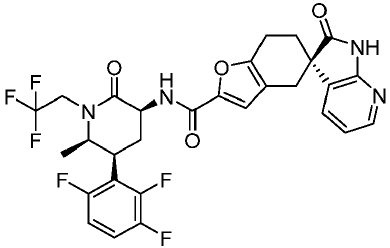 | (R)-N-((3S,5S,6R)-6-methyl-2-oxo-1-(2,2,2-trifluoroe thyl)-5-(2,3,6-trifluorophenyl)piperidin-3-yl)-2'-oxo-l' ,2',6,7-tetrahydro-4H-spiro[benzofuran-5,3'-pyrrolo[2, 3-b]pyridine]-2-formamide |
| Example 1B | 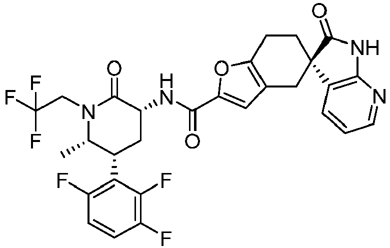 | (R)-N-((3R,5R,6S)-6-methyl-2-oxo-1-(2,2,2-trifluoroe thyl)-5-(2,3,6-trifluorophenyl)piperidin-3-yl)-2'-oxo-l' ,2',6,7-tetrahydro-4H-spiro[benzofuran-5,3'-pyrrolo[2, 3-b]pyridine]-2-formamide |
| Example 1C | 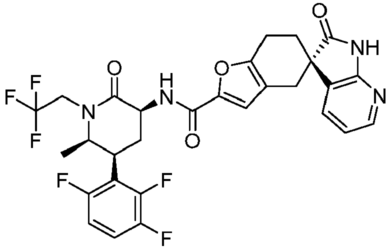 | (S)-N-((3S,5S,6R)-6-methyl-2-oxo-1-(2,2,2-trifluoroct hyl)-5-(2,3,6-trifluorophenyl)piperidin-3-yl)-2'-oxo-1', 2',6,7-tetrahydro-4H-spiro [benzofuran-5,3'-pyrrolo [2,3 -b]pyridine]-2-formamide |
| Example 1D | 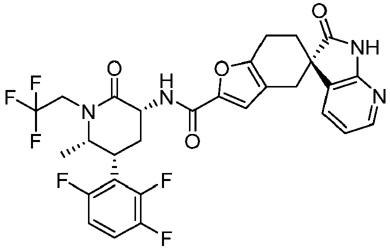 | (S)-N-((3R,5R,68)-6-methyl-2-oxo-1-(2,2,2-trifluoroe thyl)-5-(2,3,6-trifluorophenyl)piperidin-3-yl)-2'-oxo-l' ,2',6,7-tetrahydro-4H-spiro[benzofuran-5,3'-pyrrolo[2, 3-b]pyridine]-2-formamide |
| Example 2 | 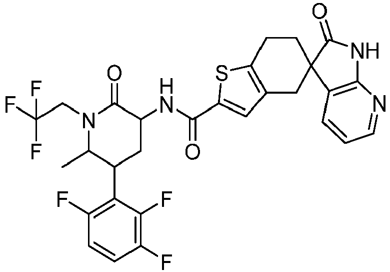 | N-(6-methyl-2-oxo-1-(2,2,2-trifluoroethyl)-5-(2,3,6-tri fluorophenyl)piperidin-3-yl)-2'-oxo-1',2',6,7-tetrahydr o-4H-spiro[benzo[b]thiophene-5,3'-pyrrolo[2,3-b]pyri dine]-2-formamide |
| Example 3 | 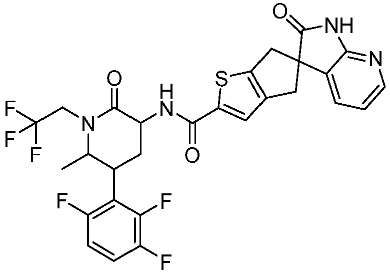 | N-(6-methyl-2-oxo-1-(2,2,2-trifluoroethyl)-5-(2,3,6-tri fluorophenyl)piperidin-3-yl)-2'-oxo-1',2',4,6-tetrahydr ospiro[cyclopenta[b]thiophene-5,3'-pyrrolo[2,3-b]pyri dine]-2-formamide |
| Example 3A | 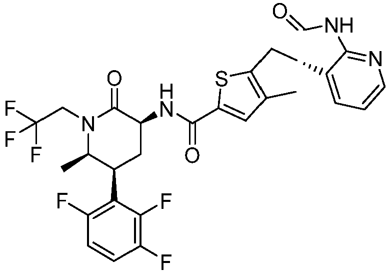 | (S)-N-((3S,5S,6R)-6-methyl-2-oxo-1-(2,2,2-trifluoroct hyl)-5-(2,3,6-trifluorophenyl)piperidin-3-yl)-2'-oxo-1', 2',4,6-tetrahydrospiro[cyclopenta[b]thiophene-5,3'-pyr rolo[2,3-b]pyridine]-2-formamide |
| Example 3B | 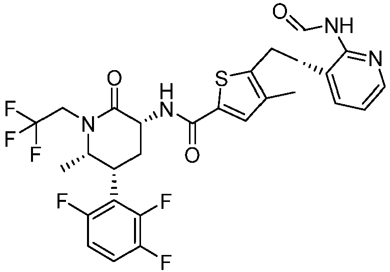 | (S)-N-((3R,5R,6S)-6-methyl-2-oxo-1-(2,2,2-trifluoroe thyl)-5-(2,3,6-trifluorophenyl)piperidin-3-yl)-2'-oxo-l' ,2',4,6-tetrahydrospiro [cyclopenta[b]thiophene-5,3'-py rrolo[2,3-b]pyridine]-2-formamide |
| Example 3C | 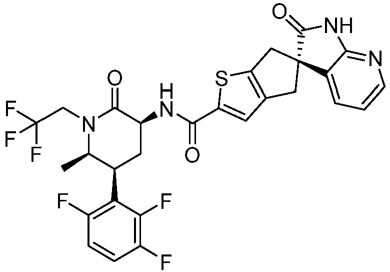 | (R)-N-((3 S,5 S,6R)-6-methyl-2-oxo-1-(2,2,2-trifluoroe thyl)-5-(2,3,6-trifluorophenyl)piperidin-3-yl)-2'-oxo-l' ,2',4,6-tetrahydrospiro [cyclopenta[b]thiophene-5,3'-py rrolo[2,3-b]pyridine]-2-formamide |
| Example 3D | 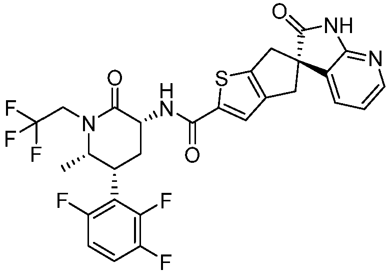 | (R)-N-((3R,5R,6S)-6-methyl-2-oxo-1-(2,2,2-trifluoroe thyl)-5-(2,3,6-trifluorophenyl)piperidin-3-yl)-2'-oxo-l' ,2',4,6-tetrahydrospiro [cyclopenta[b]thiophene-5,3'-py rrolo[2,3-b]pyridine]-2-formamide |
| Example 4 | 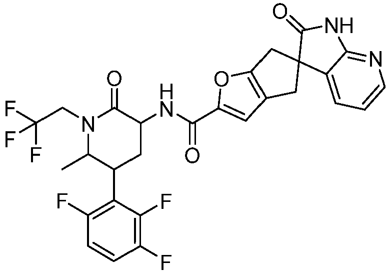 | N-(6-methyl-2-oxo-1-(2,2,2-trifluoroethyl)-5-(2,3,6-tri fluorophenyl)piperidin-3-yl)-2'-oxo-1',2',4,6-tetrahydr ospiro[cyclopenta[b]furan-5,3'-pyrrolo[2,3-b]pyridine] -2-formamide |
| Example 5A | 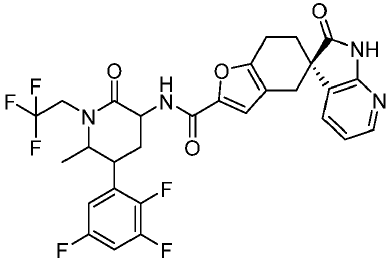 | (5R)-N-(6-methyl-2-oxo-1-(2,2,2-trifluoroethyl)-5-(2, 3,5-trifluorophenyl)piperidin-3-yl)-2'-oxo-1',2',6,7-tetr ahydro-4H-spiro[benzofuran-5,3'-pyrrolo[2,3-b]pyridi ne]-2-formamide |
| Example 5B | 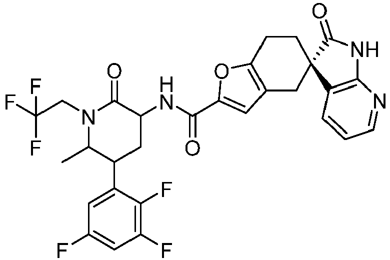 | (5 S)-N-(6-methyl-2-oxo-1-(2,2,2-trifluoroethyl)-5-(2,3 ,5-trifluorophenyl)piperidin-3-yl)-2'-oxo-1',2',6,7-tetra hydro-4H-spiro[benzofuran-5,3'-pyrrolo[2,3-b]pyridin e]-2-formamide |
| Example 6A | 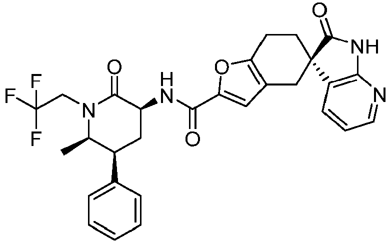 | (R)-N-((3S,5S,6R)-6-methyl-2-oxo-5-phenyl-1-(2,2,2-trifluoroethyl)piperidin-3-yl)-2'-oxo-1',2',6,7-tetrahydr o-4H-spiro[benzofuran-5,3'-pyrrolo[2,3-b]pyridine]-2-formamide |
| Example 6B | 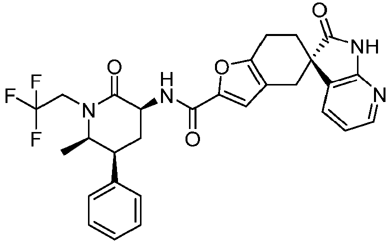 | (S)-N-((3 S,5 S,6R)-6-methyl-2-oxo-5-phenyl-1-(2,2,2-trifluoroethyl)piperidin-3-yl)-2'-oxo-1',2',6,7-tetrahydr o-4H-spiro[benzofuran-5,3'-pyrrolo[2,3-b]pyridine]-2-formamide |
| Example 6C | 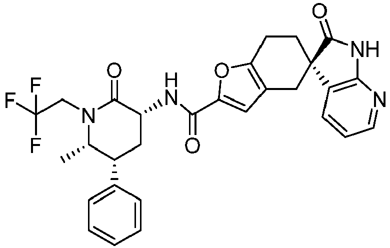 | (R)-N-((3R,5R,6S)-6-methyl-2-oxo-5-phenyl-1-(2,2,2-trifluoroethyl)piperidin-3-yl)-2'-oxo-1',2',6,7-tetrahydr o-4H-spiro[benzofuran-5,3'-pyrrolo[2,3-b]pyridine]-2-formamide |
| Example 6D | 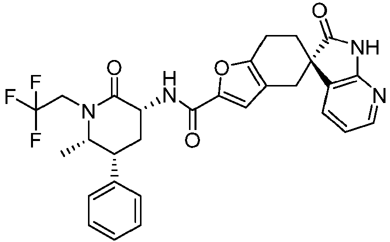 | (S)-N-((3R,5R,6S)-6-methyl-2-oxo-5-phenyl-1-(2,2,2-trifluoroethyl)piperidin-3-yl)-2'-oxo-1',2',6,7-tetrahydr o-4H-spiro[benzofuran-5,3'-pyrrolo[2,3-b]pyridine]-2-formamide |
| Example 7A | 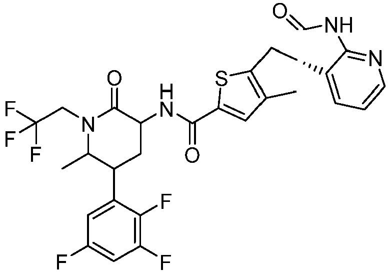 | (5S)-N-(6-methyl-2-oxo-1-(2,2,2-trifluoroethyl)-5-(2,3 ,5-trifluorophenyl)piperidin-3-yl)-2'-oxo-1',2',4,6-tetra hydrospiro[cyclopenta[b]thiophen-5,3'-pyrrolo[2,3-b]p yridine]-2-formamide |
| Example 7B | 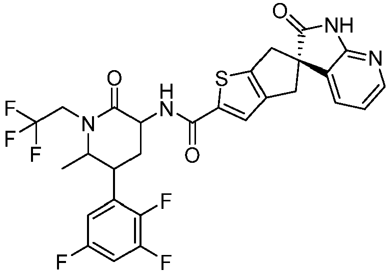 | (5R)-N-(6-methyl-2-oxo-1-(2,2,2-trifluoroethyl)-5-(2, 3,5-trifluorophenyl)piperidin-3-yl)-2'-oxo-1',2',4,6-tetr ahydrospiro[cyclopenta[b]thiophene-5,3'-pyrrolo[2,3-b]pyridine]-2-formamide |
| Example 8A | 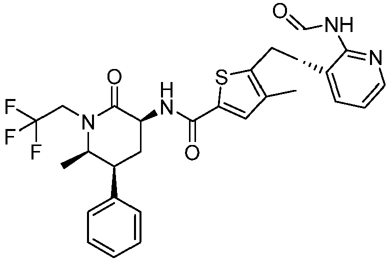 | (S)-N-((3 S,5 S,6R)-6-methyl-2-oxo-5-phenyl-1-(2,2,2-trifluoroethyl)piperidin-3-yl)-2'-oxo-1',2',4,6-tetrahydr ospiro[cyclopenta[b]thiophene-5,3'-pyrrolo[2,3-b]pyri dine]-2-formamide |
| Example 8B | 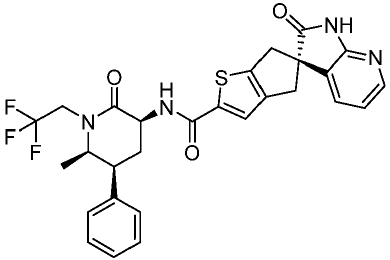 | (R)-N-((3S,5S,6R)-6-methyl-2-oxo-5-phenyl-1-(2,2,2-trifluoroethyl)piperidin-3-yl)-2'-oxo-1',2',4,6-tetrahydr ospiro[cyclopenta[b]thiophene-5,3'-pyrrolo[2,3-b]pyri dine]-2-formamide |
| Example 8C | 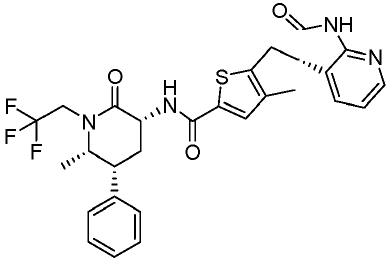 | (S)-N-((3R,5R,6S)-6-methyl-2-oxo-5-phenyl-1-(2,2,2-trifluoroethyl)piperidin-3-yl)-2'-oxo-1',2',4,6-tetrahydr ospiro[cyclopenta[b]thiophene-5,3'-pyrrolo[2,3-b]pyri dine]-2-formamide |
| Example 8D | 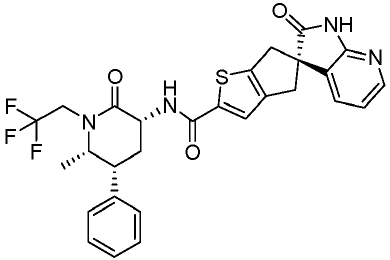 | (R)-N-((3R,5R,6S)-6-methyl-2-oxo-5-phenyl-1-(2,2,2-trifluoroethyl)piperidin-3-yl)-2'-oxo-1',2',4,6-tetrahydr ospiro[cyclopenta[b]thiophene-5,3'-pyrrolo[2,3-b]pyri dine]-2-formamide |
| Example 9 | 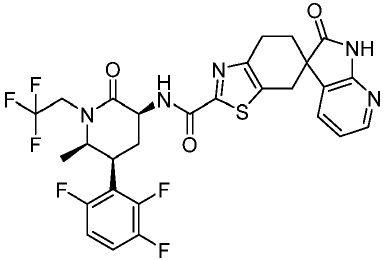 | N-((3S,5 S,6R)-6-methyl-2-oxo-1-(2,2,2-trifluoroethyl) -5-(2,3,6-trifluorophenyl)piperidin-3-yl)-2'-oxo-1',2',4, 7-tetrahydro-5H-spiro[benzo[d]thiazole-6,3'-pyrrolo[2 ,3-b]pyridine]-2-formamide |
| Example 10 | 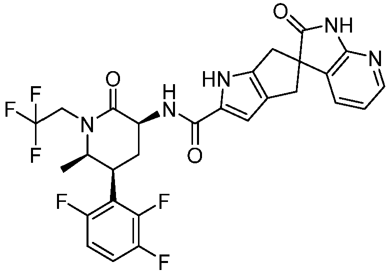 | N-((3S,5 S,6R)-6-methyl-2-oxo-1-(2,2,2-trifluoroethyl) -5-(2,3,6-trifluorophenyl)piperidin-3-yl)-2'-oxo-1',2',4, 6-tetrahydro-1H-spiro[cyclopenta[b]pyrrole-5,3'-pyrro lo[2,3-b]pyridine]-2-formamide |
| Example 11 | 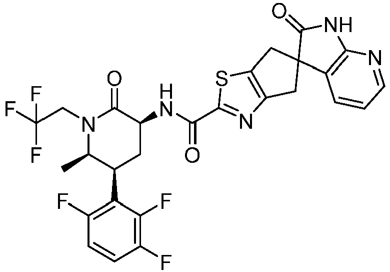 | N-((3S,5 S,6R)-6-methyl-2-oxo-1-(2,2,2-trifluoroethyl) -5-(2,3,6-trifluorophenyl)piperidin-3-yl)-2'-oxo-1',2',4, 6-tetrahydrospiro[cyclopenta[d]thiazole-5,3'-pyrrolo[2 ,3-b]pyridine]-2-formamide |
| Example 12 | 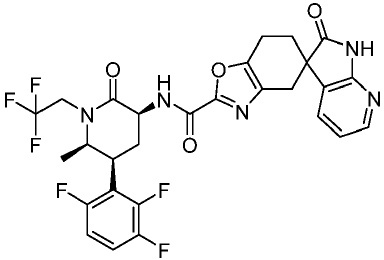 | N-((3S,5 S,6R)-6-methyl-2-oxo-1-(2,2,2-trifluoroethyl) -5-(2,3,6-trifluorophenyl)piperidin-3-yl)-2'-oxo-1',2',6, 7-tetrahydro-4H-spiro [benzo [d]oxazole-5,3'-pyrrolo[2, 3-b]pyridine]-2-formamide |
| Example 13 | 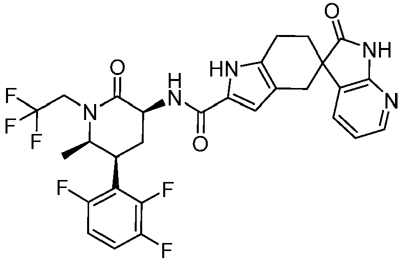 | N-((3S,5 S,6R)-6-methyl-2-oxo-1-(2,2,2-trifluoroethyl) -5-(2,3,6-trifluorophenyl)piperidin-3-yl)-2'-oxo-1,1',2', 4,6,7-hexahydrospiro[indole-5,3'-pyrrolo[2,3-b]pyridi ne]-2-formamide |
| Example 13A | 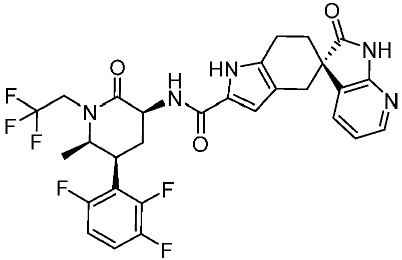 | (S)-N-((3S,SS,6R)-6-methyl-2-oxo-1-(2,2,2-trifluoroet hyl)-5-(2,3,6-trifluorophenyl)piperidin-3-yl)-2'-oxo-1, 1',2',4,6,7-hexahydrospiro [indole-5,3'-pyrrolo [2,3-b]p yridine]-2-formamide |
| Example 13B | 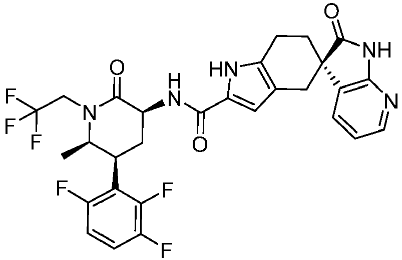 | (R)-N-((3 S,5 S,6R)-6-methyl-2-oxo-1-(2,2,2-trifluoroe thyl)-5-(2,3,6-trifluorophenyl)piperidin-3-yl)-2'-oxo-1, 1',2',4,6,7-hexahydrospiro[indole-5,3'-pyrrolo[2,3-b]p yridine]-2-formamide |

or a stereoisomer, tautomer, deuterated derivative or pharmaceutically acceptable salt thereof.

Note: If there is a discrepancy between a drawn structure and the name given for that structure, the drawn structure will be given greater weight.

Furthermore, the present application provides a pharmaceutical composition, which comprises an effective anount of the compound represented by the general formula (I), (II), (III), (IV) or (V) or a stereoisomer, tautomer, deuterated derivative or pharmaceutically acceptable salt thereof, and a pharmaceutically acceptable carrier, excipient, or a combination thereof.

The present application provides use of the compound represented by the general formula (I), (II), (III), (IV) or (V) or a stereoisomer, tautomer, deuterated derivative or pharmaceutically acceptable salt thereof, or a pharmaceutical composition thereof for preparing a CGRP receptor antagonist.

The present application also provides use of the compound represented by the general formula (I), (II), (III), (IV) or (V) or a stereoisomer, tautomer, deuterated derivative or a pharmaceutically acceptable salt thereof, or a pharmaceutical composition thereof in the preparation of a medicament for preventing and/or treating a disease mediated by CGRP, wherein preferably, the disease mediated by CGRP is cerebrovascular or vascular disorder diseases; wherein the cerebrovascular or vascular disorder diseases mediated by CGRP is selected from the group consisting of: episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

The present application further provides use of the compound represented by the general formula (I), (II), (III), (IV) or (V) or a stereoisomer, tautomer, deuterated derivative or a pharmaceutically acceptable salt thereof, or a pharmaceutical composition thereof in the preparation of a mendicament for preventing and/or treating cerebrovascular or vascular disorder diseases.

The present application provides use of the compound represented by the general formula (I), (II), (III), (IV) or (V) or a stereoisomer, tautomer, deuterated derivative or a pharmaceutically acceptable salt thereof, or a pharmaceutical composition thereof in the preparation of a mendicament for preventing and/or treating episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

The present application further provides a compound represented by the general formula (I), (II), (III), (IV) or (V), or a stereoisomer, tautomer, deuterated derivative or pharmaceutically acceptable salt thereof, or a pharmaceutical composition thereof for use in the prevention or treatment of diseases mediated by CGRP, wherein the disease mediated by CGRP is cerebrovascular or vascular disorder diseases.

Furthermore, the cerebrovascular or vascular disorder diseases mediated by CGRP are selected from the group consisting of: episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

This application provides the compound represented by the general formula (I), (II), (III), (IV) or (V) or a stereoisomer, tautomer, deuterated derivative or a pharmaceutically acceptable salt thereof, or a pharmaceutical composition thereof for use in preventing and/or treating episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and Chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

The compounds of the present application are optionally in the form of a mixture of a single optical isomer, a single enantiomer, or a racemic isomer, in the form of tautomers, and in the form of free bases or corresponding acid addition salts formed with pharmacologically acceptable acids.

The compounds of the present application may exist as tautomers. All tautomeric forms of the compounds of the present application are contemplated as being within the scope of the present application.

### BRIEF DESCRIPTION OF THE DRAWINGS

Figure 1 is a blood flow rate change-time curve (%);
Figure 2 is the area under the blood flow rate-time curve (%·min).

### DETAIL DESCRIPTION OF THE INVENTION

Unless otherwise stated, some of the terms used in the specification and claims of this application are defined as follows:
"Alkyl" when used as a group or a part of a group refers to a straight chain or branched aliphatic hydrocarbon group including C₁-C₂₀. Preferred is C₁-C₁₀ alkyl, more preferred is C₁-C₆ alkyl. Examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl can be substituted or unsubstituted.
"Cycloalkyl" refers to saturated or partially saturated monocyclic, fused, bridged, and spiro carbon rings. Preferred is C₃-C₁₂ cycloalkyl, more preferred is C₃-C₈ cycloalkyl, and most preferred is C₃-C₆ cycloalkyl. Examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like, preferred is cyclopropyl and cyclohexenyl. The cycloalkyl can be optionally substituted or unsubstituted.
"Heterocyclyl", "heterocycloalkyl", "heterocycle" and "heterocyclic" are used interchangeably in this application and refer to non-aromatic heterocyclyl, wherein one or more of the atoms forming the ring are heteroatoms, such as oxygen, nitrogen, sulfur atoms, etc., including monocyclic, polycyclic, fused, bridged and spirocyclic rings. It preferably has a 5- to 7-membered monocyclic ring or a 7- to 10-membered bicyclic or tricyclic ring, which may comprise 1, 2 or 3 atoms selected from nitrogen, oxygen and/or sulfur. Examples of "heterocyclyl" include, but are not limited to, morpholinyl, oxetanyl, thiomorpholinyl, tetrahydrofuranyl, tetrahydropyranyl, 1,1-dioxo-thiomorpholinyl, piperidinyl, 2-oxo-piperidinyl, pyrrolidinyl, 2-oxo-pyrrolidinyl, piperazin-2-one, 8-oxa-3-aza-bicyclo[3.2.1]octyl, piperazinyl, and hexahydropyrimidine. The heterocyclyl can be substituted or unsubstituted.
"Aryl" refers to a carbocyclic aromatic system comprising one or two rings, wherein the rings may be fused together. The term "aryl" includes monocyclic or bicyclic aromatic groups, such as phenyl, naphthyl, and tetrahydronaphthyl. Preferred aryl is C₆-C₁₀ aryl, more preferred aryl is phenyl and naphthyl, and the most preferred aryl is naphthyl. Aryl can be substituted or unsubstituted.
"Heteroaryl" refers to an aromatic 5- to 6-membered monocyclic or 8- to 10-membered bicyclic ring which may comprise 1 to 4 atoms selected from nitrogen, oxygen and/or sulfur. Examples of "heteroaryl" include, but are not limited to, furanyl, pyridinyl, 2-oxo-1,2-dihydropyridinyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolinyl, indazolyl, benzisothiazolyl, benzoxazolyl, and benzisoxazolyl. A heteroaryl group can be substituted or unsubstituted.
"Alkoxy" refers to the group (alkyl-O-). Wherein, alkyl is as defined herein. The alkoxy group of C₁-C₆ is preferred. Examples of alkoxy include, but are not limited to, methoxy, ethoxy, n-propoxy, isopropoxy, n-butoxy, isobutoxy, tert-butoxy, and the like.
"Hydroxy" refers to an -OH group.
"Halogen" refers to fluorine, chlorine, bromine and iodine.
"Amino" refers to -NH₂.
"Cyano" refers to -CN.
"Nitro" refers to -NO₂.
"Carboxy" refers to -C(O)OH.
"DMSO" refers to dimethyl sulfoxide.
"BOC" refers to tert-butoxycarbonyl.
"TFA" refers to trifluoroacetic acid.
"PMB" refers to p-methoxybenzyl.
"SEM" refers to (trimethylsilyl)ethoxymethyl.
"Hydroxyalkyl" refers to alkyl substituted with a hydroxy group.
"Aminoalkyl" refers to alkyl substituted with an amino group.
"Leaving group", or leaving radical, is an atom or functional group that leaves a larger molecule in a chemical reaction. It is a term used in nucleophilic substitution reactions and elimination reactions. In a nucleophilic substitution reaction, the reactant attacked by the nucleophile is called a substrate, and the atom or atomic group that breaks away from the substrate molecule with a pair of electrons is called a leaving group. Groups that easily accept electrons and have a strong ability to bear negative charges are good leaving groups. The smaller the pKa of the leaving group's conjugate acid, the easier it is for the leaving group to dissociate from other molecules. The reason is that when the pKa of its conjugate acid is smaller, the corresponding leaving group does not need to combine with other atoms, and the tendency to exist in the form of anion (or electrically neutral leaving group) is strengthened. Common leaving groups include, but are not limited to, halogen, mesyl, -OTs, -OTf, or -OH.
"Substituted" means that one or more hydrogen atoms, preferably up to 5, more preferably 1 to 3 hydrogen atoms in the group are each independently replaced by a corresponding number of substituents. It goes without saying that the substituents are only in their possible chemical positions and the person skilled in the art will be able to determine (either experimentally or theoretically) which substitutions are possible or impossible without undue effort. For example, an amino or hydroxyl group having a free hydrogen may be unstable when combined with a carbon atom having an unsaturated (e.g., olefinic) bond.
"Substitution" or "substituted" as used in this specification, unless otherwise specified, means that the group may be substituted by one or more groups selected from the following: alkyl, alkoxy, alkylthio, alkylamino, halogen, mercapto, hydroxyl, nitro, cyano, cycloalkyl, heterocyclyl, aryl, heteroaryl, cycloalkyloxy, heterocycloalkyloxy, cycloalkylthio, heterocycloalkylthio, amino, haloalkyl, and hydroxyalkyl;
"Pharmaceutically acceptable salts" refer to certain salts of the above compounds that can retain the original biological activity and are suitable for medical use. The pharmaceutically acceptable salt of the compound represented by the general formula (I) may be a metal salt or an amine salt with a suitable acid.

Those skilled in the art will appreciate that salts of the compounds represented by the general formula (I), (II), (III), (IV) or (V), including pharmaceutically acceptable salts, may be prepared. These salts can be prepared in situ during the final isolation and purification of the compounds, or by separately reacting the purified compound in its free acid or free base form with a suitable base or acid, respectively. Bases generally used to form pharmaceutically acceptable salts include organic bases or inorganic bases, and acids generally used to form pharmaceutically acceptable salts include organic acids or inorganic acids.

The pharmaceutically acceptable salts of the present application can be synthesized from basic or acidic moieties by conventional chemical methods. Generally, the salts can be prepared by reacting free acid forms of these compounds with a chemical amount of the appropriate base, or by reacting free base forms of these compounds with a chemical amount of the appropriate acid. These reactions are generally carried out in water or in an organic solvent, or in a mixture of the two. Generally, it will be desirable to employ non-aqueous media such as diethyl ether, ethyl acetate, ethanol, isopropanol or acetonitrile. Lists of other suitable salts can be found in "Remington's Pharmaceutical Sciences", 20th edition, Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

"Deuterated derivatives" are compounds among the above compounds that comprise deuterium bound to carbon at at least one position, and the amount of deuterium bound to carbon exceeding its natural content.

A "pharmaceutical composition" means a mixture comprising one or more of the compounds described herein or pharmaceutically acceptable salts and other other components such as pharmaceutically acceptable carriers and excipients. The purpose of a pharmaceutical composition is to facilitate administration to an organism, facilitate the absorption of the active ingredients and thereby exert biological activity.

The pharmaceutical composition involved in the present application can be formulated for a specific administration route, such as oral administration, parenteral administration, and rectal administration. In addition, the pharmaceutical composition of the present application can be prepared in solid form (including but not limited to capsules, tablets, pills, granules, powders or suppositories) or in liquid form (including but not limited to solutions, suspensions or emulsions). The pharmaceutical composition can be subjected to conventional pharmaceutical operations (e.g., sterilization) and/or can comprise conventional inert diluents, lubricants or buffers and adjuvants, such as preservatives, stabilizers, wetting agents, emulsifiers and buffers.

Typically, the pharmaceutical composition is a tablet or capsule, which comprises the active ingredient and one or more of the following:
a) diluents, such as lactose, dextrose, sucrose, mannitol, sorbitol, cellulose, glycine, etc.;
b) lubricants, such as silicon dioxide, talc, stearic acid, its magnesium or calcium salts and/or polyethylene glycol; for tablets further comprising
c) binding agents, such as magnesium aluminum silicate, starch paste, gelatin, tragacanth, methylcellulose, sodium carboxymethylcellulose and/or polyvinylpyrrolidone; if desired further comprising
d) disintegrants, such as starch, agar, alginic acid or its sodium salt, or effervescent mixtures; and/or
e) absorbents, colorants, flavorings and sweeteners.

Tablets may be film coated or enteric coated according to methods known in the art.

Suitable compositions for oral administration include an effective amount of a compound of formula (I) or a pharmaceutically acceptable salt thereof in the form of tablets, lozenges, aqueous or oily suspensions, dispersible powders or granules, emulsions, hard or soft capsules, or syrups or elixirs. Compositions for oral use are prepared according to any method known in the art for the preparation of pharmaceutical compositions and can comprise one or more agents selected from sweeteners, flavoring agents, coloring agents and preservatives in order to provide a neat and palatable preparation. Tablets may comprise the active ingredient in admixture with non-toxic pharmaceutically acceptable excipients which are suitable for the manufacture of tablets. These excipients are, for example, inert diluents (e.g., calcium carbonate, sodium carbonate, lactose, calcium phosphate or sodium phosphate); granulating and disintegrating agents (e.g., corn starch, or alginic acid); binding agents (e.g., starch, gelatin or acacia); and lubricants (e.g., magnesium stearate, stearic acid or talc). The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Preparations for oral administration can be presented in hard gelatin capsules, wherein the active ingredient is mixed with an inert solid diluent (such as calcium carbonate, calcium phosphate or kaolin), or in soft gelatin capsules, wherein the active ingredient is mixed with water or an oil medium (such as peanut oil, liquid paraffin or olive oil).

Certain injectable compositions are aqueous isotonic solutions or suspensions, and suppositories are advantageously prepared from fatty emulsions or suspensions. Said compositions may be sterilized and/or comprise adjuvants, such as preservatives, stabilizers, wetting agents, emulsifiers, solution promoters, salts for regulating the osmotic pressure and/or buffers. Furthermore, they may also comprise other therapeutically valuable substances. The composition is prepared according to conventional mixing, granulating or coating methods, respectively, and comprises about 0.1-75% or about 1-50% of the active ingredient.

Since water may facilitate the degradation of certain compounds, the present application also provides anhydrous pharmaceutical compositions and dosage forms comprising the compounds of the present application as active ingredients.

Anhydrous pharmaceutical compositions and dosage forms of the present application can be prepared using anhydrous or low moisture comprising ingredients and low moisture or low humidity conditions. An anhydrous pharmaceutical composition can be prepared and stored so as to maintain its anhydrous nature. Accordingly, anhydrous compositions are packaged using materials known to prevent contact with water such that they can be included in suitable formulary kits. Examples of suitable packaging include, but are not limited to, hermetically sealed foils, plastics, unit dose comprisers (e.g., vials), blister packs, and strip packs.

The present application further provides pharmaceutical compositions and dosage forms comprising one or more agents that reduce the decomposition rate of the compounds of the present application as active ingredients. Such agents (which are herein referred to as "stabilizers") include, but are not limited to, antioxidants (e.g., ascorbic acid), pH buffers, or salt buffers, and the like.

For an individual of about 50-70 kg, the pharmaceutical composition or combination product of the present application can be a unit dose of about 1-1000 mg of active ingredient, or about 1-500 mg or about 1-250 mg or about 1-150 mg or about 0.5-100 mg, or about 1-50 mg of active ingredient. The therapeutically effective dose of the compound, pharmaceutical composition or combination thereof will depend on the species, weight, age and individual condition of the individual, the disorder or disease being treated or its severity. A physician, clinician or veterinarian of ordinary skill can readily determine the effective amount of each of the active ingredients required to prevent, treat or inhibit the progress of the disorder or disease.

"Stereoisomer" of a compound of a given stereochemical configuration refers to the opposite enantiomer of the compound and includes any diastereomers of the geometric isomers (Z/E) of the compound. For example, if a compound has an S,R,Z stereochemical configuration, its stereoisomers will include its opposite enantiomer having the R,S,Z configuration, and its diastereomers having the S,S,Z configuration, the R,R,Z configuration, the S,R,E configuration, the R,S,E configuration, the S,S,E configuration, and the R,R,E configuration. If the stereochemical configuration of a compound is not specified, then "stereoisomer" refers to any of the possible stereochemical configurations of that compound.

The compounds represented by general formula (I), (II), (III), (IV) or (V), stereoisomers, or complexes of tautomers or stereoisomers thereof may be administered alone or in combination with one or more pharmaceutically active compounds. Generally, one or more of these compounds are administered in the form of a pharmaceutical composition (formulation) in combination with one or more pharmaceutically acceptable excipients. The choice of excipient depends on the specific mode of administration, the effect of the excipient on solubility and stability, the nature of the dosage form, and the like. Useful pharmaceutical compositions and methods for their preparation may be found, for example, in A. R. Gennaro (ed.), Remington: The Science and Practice of Pharmaceuticals (20th ed., 2000).

The compounds of the present application may comprise asymmetric centers or chiral centers and therefore exist in different stereoisomeric forms. It is contemplated that all stereoisomeric forms of the compounds of the present application, including but not limited to diastereomers, enantiomers and atropisomers and geometric (conformational) isomers and mixtures thereof, such as racemic mixtures, are within the scope of the present application.

Unless otherwise indicated, structures depicted herein also encompass all isomers (e.g., diastereomers, enantiomers, and atropisomers and geometric (conformational) isomeric forms of such structures; for example, R and S configurations at various asymmetric centers, (Z) and (E) double bond isomers, and (Z) and (E) conformational isomers. Therefore, individual stereoisomers as well as mixtures of enantiomers, diastereomers, and geometric (conformational) isomers of the compounds of the present application are within the scope of the present application.

The term "stereoisomer" refers to isomers produced by different spatial arrangements of atoms in molecules. They can be divided into cis-trans isomers and enantiomers, or into two major categories: enantiomers and diastereomers. Stereoisomers are a type of isomers. Isomers that have the same order of connection between atoms or atomic groups in a molecule but different spatial arrangements are called stereoisomers.

The term "substantially enantiomerically pure" refers to greater than 90% enantiomeric purity of a given stereocenter. Thus, the term "substantially enantiomerically pure" means greater than 80% ee (enantiomeric excess). For compounds that exist as stereoisomers, such stereoisomers may be substantially enantiomerically pure at the stereocenter, or preferably may have greater than 97% enantiomeric purity, or more preferably greater than 99% enantiomeric purity.

### Synthesis method of the compound of the present application

In order to achieve the purpose of present application, the present application adopts the following technical solutions:
The present application provides a method for preparing a compound represented by general formula (I) or a stereoisomer, tautomer, deuterated derivative or pharmaceutically acceptable salt thereof, the method including:

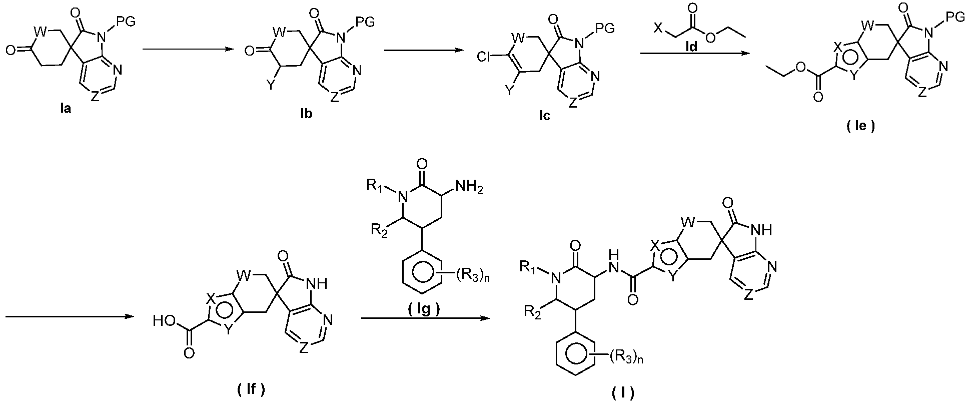

The compound represented by the general formula (Ia) undergoes electrophilic addition and rearrangement to obtain the compound represented by the general formula (Ib), the compound represented by the general formula (Ib) undergoes chlorination reaction to obtain the compound represented by the general formula (Ic), the compound represented by the general formula (Ic) and the compound represented by the general formula (Id) undergo cyclization reaction to obtain the compound represented by the general formula (Ie), the compound represented by the general formula (Ie) is deprotected to obtain the compound represented by the general formula (If), the compound represented by the general formula (If) and the compound represented by the general formula (Ig) undergo condensation reaction to obtain the compound represented by the general formula (I);
wherein:
PG is an amino protecting group;
X or Y are the same or different and are each independently selected from =CR₄-, =N-, -NR₅-, -O-, -S-, -S(O)-, -S(O)₂-, 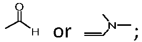
R₁, R₂, R₃, W, Z and n are defined as described in the general formula (I).

### DETAILED DESCRIPTION

The following Examples are used to further describe the present application, but these Examples are not intended to limit the scope of the present application.

### Example

The Examples provide the preparation of representative compounds represented by formula (I) and related structural identification data. It must be noted that the following Examples are used to illustrate the present application rather than to limit the present application. The ¹H NMR spectrum was measured using a Bruker instrument (400 MHz), and the chemical shift is expressed in ppm. Tetramethylsilane internal standard (0.00 ppm) was used. ¹H NMR notation: s = singlet, d = doublet, t = triplet, m = multiplet, br = broadened, dd = doublet of doublet, dt = doublet of triplet. When coupling constants are given, they are given in Hz.

The mass spectrum is obtained by LC/MS, and the ionization method can be ESI or APCI.

For the thin layer chromatography silica gel plate, Yantai Huanghai HSGF254 or Qingdao GF254 silica gel plate is used. The silica gel plate used in thin layer chromatography (TLC) adopts a specification of 0.15mm-0.2mm, and the specification used for thin layer chromatography separation and purification products is 0.4mm-0.5mm.

Column chromatography generally uses Yantai Huanghai silica gel, 200-300 mesh silica gel, as the carrier.

In the following examples, unless otherwise indicated, all temperatures are degrees Celsius. Unless otherwise indicated, various starting materials and reagents are commercially available or synthesized according to known methods. Commercially available raw materials and reagents are used directly without further purification. Unless otherwise indicated, commercial manufacturers include, but are not limited to, Aldrich Chemical Company, ABCR GmbH & Co. KG, Acros Organics, Guangzan Chemical Technology Co., Ltd. and Jingyan Chemical Technology Co., Ltd.
CD₃OD: deuterated methanol.
CDCl₃: deuterated chloroform.
DMSO-d₆: deuterated dimethyl sulfoxide.
D₂O: heavy water.

Unless otherwise specified in the Examples, the solution in the reaction refers to an aqueous solution.

The compound is purified by using C18 reverse phase column preparative or semi-preparative purification, silica gel column chromatography eluent system and thin layer chromatography, wherein the eluent system is selected from: A: petroleum ether and tetrahydrofuran system; B: acetonitrile and water system; C: petroleum ether and ethyl acetate system; wherein the volume ratio of the solvent varies according to the polarity of the compound, and a small amount of acidic or alkaline reagent, such as trifluoroacetic acid, acetic acid or triethylamine, can also be added for adjustment.

### Example 1

### N-(6-methyl-2-oxo-1-(2,2,2-trifluoroethyl)-5-(2,3,6-trifluorophenyl)piperidin-3-yl)-2'-oxo-1' ,2',6,7-tetrahydro-4H-spiro[benzofuran-5,3'-pyrrolo[2,3-b]pyridine]-2-carboxamide

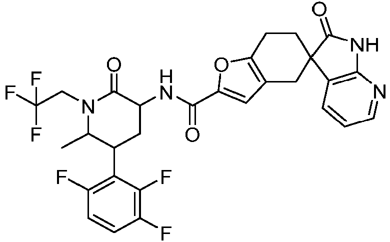

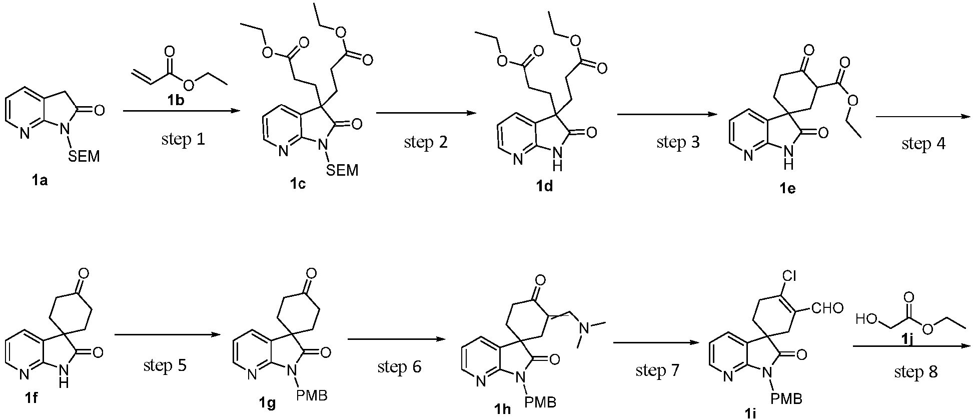

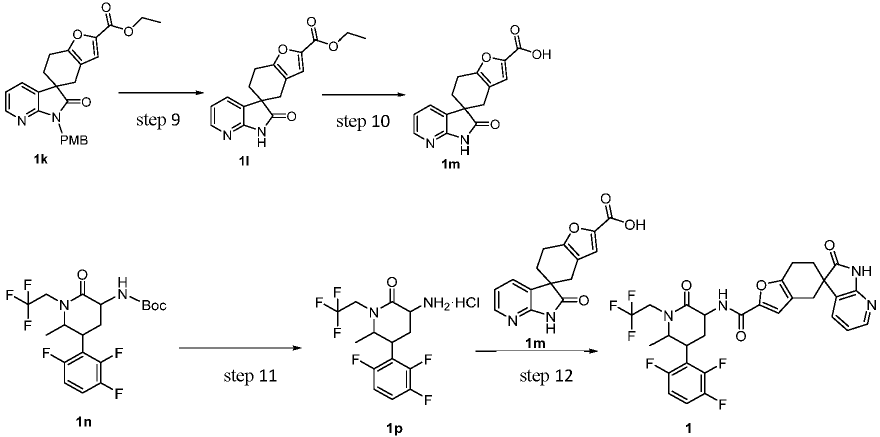

### Step 1

### Diethyl

### 3,3'-(2-oxo-1-((2-(trimethylsilyl)ethoxy)methyl)-2,3-dihydro-1H-pyrrolo[2,3-b]pyridine-3,3-diyl) dipropionate

1-((2-(trimethylsilyl)ethoxy)methyl)-1,3-dihydro-2H-pyrrolo[2,3-b]pyridin-2-one **1a** (2 g, 7.56 mmol) was dissolved in dimethyl sulfoxide (13 mL), added with potassium tert-butoxide solution (1 M, 378.22 µL), stirred at 25°C for 10 minutes, slowly added with ethyl acrylate **1b** (1.59 g, 15.89 mmol) dropwise at 45°C and stirred for 1 hour. After the reaction was completed, the reaction solution was added with water (20 mL), and extracted with ethyl acetate (40 mL×2). The organic phases were combined, dried over anhydrous sodium sulfate, and concentrated under reduced pressure. The resulting residue was separated and purified by column chromatography (eluent: System A) to obtain diethyl 3,3'-(2-oxo-1-((2-(trimethylsilyl)ethoxy)methyl)-2,3-dihydro-1H-pyrrolo[2,3-b]pyridine-3,3-diyl) dipropionate **1c** (2.4 g) with a yield of 61.46%.

MS m/z(ESI):465.3[M+1]

### Step 2

### Diethyl 3,3'-(2-oxo-2,3-dihydro-1H-pyrrolo[2,3-b]pyridine-3,3-diyl)dipropionate

### Diethyl

3,3'-(2-oxo-1-((2-(trimethylsilyl)ethoxy)methyl)-2,3-dihydro-1H-pyrrolo[2,3-b]pyridine-3,3-diyl) dipropionate **1c** (2.4 g, 5.17 mmol) was dissolved in dichloromethane (5 mL), added with trifluoroacetic acid (20 mL), and stirred at 25°C for 18 hours. After the reaction was completed, the reaction solution was concentrated under reduced pressure, added with tetrahydrofuran (20 mL), triethylamine (10 mL) and aqueous ammonia (20 mL), stirred at 25°C for 1 hour, concentrated under reduced pressure, adjusted to pH6 with 1M hydrochloric acid, and extracted with ethyl acetate (100 mL×3). The organic phases were combined, dried over anhydrous sodium sulfate, and concentrated under reduced pressure to obtain diethyl 3,3'-(2-oxo-2,3-dihydro-1H-pyrrolo[2,3-b]pyridine-3,3-diyl)dipropionate **1d** (1.6 g), which was directly used for the next step without purification.

MS m/z(ESI):335.2[M+1]

### Step 3

### Ethyl 2',4-dioxo-1',2'-dihydrospiro[cyclohexane-1,3'-pyrrolo[2,3-b]pyridine]-3-carboxylate

Diethyl 3,3'-(2-oxo-2,3-dihydro-1H-pyrrolo[2,3-b]pyridine-3,3-diyl)dipropionate **1d** (1.5 g, 4.49 mmol) was dissolved in tetrahydrofuran (30 mL), slowly added with potassium tert-butoxide solution (1 M, 13.46 mL) dropwise at low temperature and stirred for 20 minutes. After the reaction was completed, the reaction solution was quenched with saturated ammonium chloride solution (50 mL), and extracted with ethyl acetate (50 mL×3). The organic phases were combined, dried over anhydrous sodium sulfate, and concentrated under reduced pressure to obtain ethyl 2',4-dioxo-1',2'-dihydrospiro[cyclohexane-1,3'-pyrrolo[2,3-b]pyridine]-3-carboxylate **1e** (1.2 g), which was directly used for the next step without purification.

MS m/z(ESI):289.3[M+1]

### Step 4

### Spiro[cyclohexane-1,3'-pyrrolo[2,3-b]pyridine]-2',4(1'H)-dione

Ethyl 2',4-dioxo-1',2'-dihydrospiro[cyclohexane-1,3'-pyrrolo[2,3-b]pyridine]-3-carboxylate **1e** (1.1 g, 3.82 mmol) was dissolved in 3M hydrochloric acid (50 mL), added with methanol (5 mL) and dioxane (10 mL), stirred at 100°C for 2 hours, concentrated under reduced pressure, adjusted to pH8 with saturated sodium bicarbonate solution, and extracted three times with a mixed solution of ethyl acetate and tetrahydrofuran. The organic phases were combined, dried over anhydrous sodium sulfate, and concentrated under reduced pressure to obtain spiro[cyclohexane-1,3'-pyrrolo[2,3-b]pyridine]-2',4(1'H)-dione **1f** (700 mg), yield: 84.8%.

MS m/z(ESI):217.1[M+1]

¹H NMR (400 MHz, CDCl₃) δ 9.03 (s, 1H), 8.20 (dd, *J =* 5.6, 1.6 Hz,1H),7.51 (dd, *J =* 7.2, 1.6 Hz,1H), 7.01 (dd, *J =* 7.2, 5.2 Hz,1H), 3.18-3.10 (m, 2H), 2.48-2.310 (m, 2H), 2.26-2.13 (m, 4H).

### Step 5

### 1'-(4-methoxybenzyl)spiro[cyclohexane-1,3'-pyrrolo[2,3-b]pyridine]-2',4(1'H)-dione

Spiro[cyclohexane-1,3'-pyrrolo[2,3-b]pyridine]-2',4(1'H)-dione **1f** (0.54 g, 2.5 mmol) and cesium carbonate (1.22 g, 3.75 mmol) were added to N,N-dimethylformamide (3 mL), then slowly added with p-methoxybenzyl chloride (470 mg, 3.0 mmol) dropwise, reacted at 10°C for 16 hours, and monitored by LC-MS. After the reaction was completed, the reaction solution was poured into water (20 mL), extracted with ethyl acetate (30 mL×3), dried over anhydrous sodium sulfate, and concentrated under reduced pressure. The resulting residue was separated and purified by column chromatography (eluent: System A) to obtain 1'-(4-methoxybenzyl)spiro[cyclohexane-1,3'-pyrrolo[2,3-b]pyridine]-2',4(1'H)-dione **1g** (670 mg), yield: 79.7%.

MS m/z(ESI):337.2[M+1]

### Step 6

### 3-((Dimethylamino)methylene)-1'-(4-methoxybenzyl)spiro[cyclohexane-1,3'-pyrrolo[2,3-b] pyridine]-2',4(1'H)-dione

1'-(4-methoxybenzyl)spiro[cyclohexane-1,3'-pyrrolo[2,3-b]pyridine]-2',4(1'H)-dione **1g** (0.75 g, 2.23 mmol) was dissolved in dichloromethane (10 mL), added with 1-tert-butoxy-N,N,N',N'-tetramethylmethanediamine (700 mg, 4.01 mmol), reacted at 60°C for 16 hours, and monitored by LC-MS. After the reaction was completed, the reaction solution was poured into water (30 mL), extracted with ethyl acetate (40 mL×3), dried over anhydrous sodium sulfate, and concentrated under reduced pressure to obtain 3-((dimethylamino)methylene)-1'-(4-methoxybenzyl)spiro[cyclohexane-1,3'-pyrrolo[2,3-b]pyrid ine]-2',4(1'H)-dione **1h** (1.2 g), which was directly used for the next step without purification.

MS m/z(ESI):391.9[M+1]

### Step 7

### 4-Chloro-1'-(4-methoxybenzyl)-2'-oxo-1',2'-dihydrospiro[cyclohexane-1,3'-pyrrolo[2,3-b]py ridine]-3-ene-3-carbaldehyde

3-((Dimethylamino)methylene)-1'-(4-methoxybenzyl)spiro[cyclohexane-1,3'-pyrrolo[2,3-b] pyridine]-2',4(1'H)-dione **1h** (1.2 g) was dissolved in dichloromethane (10 mL), slowly added with phosphorus oxychloride (1.14 g, 9.88 mmol), reacted at 10°C for 3 hours, and monitored by LC-MS. After the reaction was completed, the reaction solution was concentrated under reduced pressure. The resulting residue was separated and purified by column chromatography (eluent: System A) to obtain 4-chloro-1'-(4-methoxybenzyl)-2'-oxo-1',2'-dihydrospiro[cyclohexane-1,3'-pyrrolo[2,3-b]pyridin e]-3-ene-3-carbaldehyde **1i** (350 mg), yield: 27.76%.

MS m/z(ESI):383.2[M+1]

### Step 8

### Ethyl

### 1'-(4-methoxybenzyl)-2'-oxo-1',2',6,7-tetrahydro-4H-spiro[benzofuran-5,3'-pyrrolo[2,3-b]pyridin e]-2-carboxylate

4-Chloro-1'-(4-methoxybenzyl)-2'-oxo-1',2'-dihydrospiro[cyclohexane-1,3'-pyrrolo[2,3-b]py ridine]-3-ene-3-carbaldehyde **1i** (0.1 g, 261.3 µmol) and ethyl 2-hydroxyacetate **1j** (119.65 mg, 1.15 mmol) were dissolved in tetrahydrofuran (2 mL), cooled to 0°C, added with sodium hydride (39.7 mg, 992.58 µmol, 60% purity), heated to 90°C under nitrogen protection, stirred for 4 hours, and monitored by LC-MS. After the reaction was completed, the reaction solution was poured into a saturated ammonium chloride solution (5 mL), extracted with ethyl acetate (20 mL×3), dried over anhydrous sodium sulfate, and concentrated under reduced pressure. The resulting residue was separated and purified by column chromatography (eluent: System A) to obtain ethyl 1'-(4-methoxybenzyl)-2'-oxo-1',2',6,7-tetrahydro-4H-spiro[benzofuran-5,3'-pyrrolo[2,3-b]pyridin e]-2-carboxylate **1k** (42 mg), yield: 37%.

MS m/z(ESI):433.3[M+1]

### Step 9

### Ethyl

### 2'-oxo-1',2',6,7-tetrahydro-4H-spiro[benzofuran-5,3'-pyrrolo[2,3-b]pyridine]-2-carboxylate

### ethyl

1'-(4-methoxybenzyl)-2'-oxo-1',2',6,7-tetrahydro-4H-spiro[benzofuran-5,3'-pyrrolo[2,3-b]pyridin e]-2-carboxylate **1k** (62 mg, 138.23 µmol) and aluminum chloride (41.74 mg, 276.46 µmol) were added to anisole (1 mL), heated to 130°C, reacted for 4 hours, and monitored by LC-MS. After the reaction was completed, the reaction solution was poured into water (5 mL), extracted with ethyl acetate (20 mL×3), dried over anhydrous sodium sulfate, concentrated under reduced pressure, and purified by thin layer plate to obtain ethyl 2'-oxo-1',2',6,7-tetrahydro-4H-spiro[benzofuran-5,3'-pyrrolo[2,3-b]pyridine]-2-carboxylate 11 (35 mg), yield: 77%.

MS m/z(ESI):312.9[M+1]

### Step 10

### 2'-Oxo-1',2',6,7-tetrahydro-4H-spiro[benzofuran-5,3'-pyrrolo[2,3-b]pyridine]-2-carboxylic acid

### Ethyl

2'-oxo-1',2',6,7-tetrahydro-4H-spiro[benzofuran-5,3'-pyrrolo[2,3-b]pyridine]-2-carboxylate **1l** (30 mg, 96.06 µmol) was added to 1.6 mL of a mixed solution (tetrahydrofuran:methanol: water = 10:3:3), added with sodium hydroxide (11.53 mg, 288.17 µmol), stirred at 10°C for 16 hours, and monitored by LC-MS. After the reaction was completed, the reaction solution was concentrated under reduced pressure, added with 2 mL of water, and adjusted to pH5 with 1M hydrochloric acid, extracted with ethyl acetate (10 mL×3), and concentrated under reduced pressure to obtain 2'-oxo-1',2',6,7-tetrahydro-4H-spiro[benzofuran-5,3'-pyrrolo[2,3-b]pyridine]-2-carboxylic acid **1m** (25 mg), which was directly used for the next step without purification.

MS m/z(ESI):285.2[M+1]

### Step 11

### 3-Amino-6-methyl-1-(2,2,2-trifluoroethyl)-5-(2,3,6-trifluorophenyl)piperidin-2-one hydrochloride

### tert-Butyl

(6-methyl-2-oxo-1-(2,2,2-trifluoroethyl)-5-(2,3,6-trifluorophenyl)piperidin-3-yl)carbamate **1n** (885 mg, 2.01 mmol, prepared according to the method described in patent application WO2012064910) and 4M hydrochloric acid were added to 9 mL of 1,4-dioxane solution and reacted at 25°C for 1 hour. After the reaction was completed, the reaction solution was concentrated under reduced pressure to obtain 3-amino-6-methyl-1-(2,2,2-trifluoroethyl)-5-(2,3,6-trifluorophenyl)piperidin-2-one hydrochloride **1p** (753 mg), yield: 89%.

MS m/z(ESI):341.1[M+1]

### Step 12

### N-(6-methyl-2-oxo-1-(2,2,2-trifluoroethyl)-5-(2,3,6-trifluorophenyl)piperidin-3-yl)-2'-oxo-1' ,2',6,7-tetrahydro-4H-spiro[benzofuran-5,3'-pyrrolo[2,3-b]pyridine]-2-carboxamide

2'-oxo-1',2',6,7-tetrahydro-4H-spiro[benzofuran-5,3'-pyrrolo[2,3-b]pyridine]-2-carboxylic acid **1m** (25 mg, 87.95 µmol) was added to N,N-dimethylformamide (0.5 mL), then added with 3-amino-6-methyl-1-(2,2,2-trifluoroethyl)-5-(2,3,6-trifluorophenyl)piperidin-2-one hydrochloride **1p** (26.51 mg, 70.36 µmol), N,N-diisopropylethylamine (56.72 mg, 439.73 µmol), 1-hydroxybenzotriazole (23.77 mg, 175.89 µmol), then added with 1-(3-dimethylaminopropyl)-3-ethylcarbodiimide hydrochloride (33.6 mg, 175.89 µmol), stirred at 25°C for 4 hours, and monitored by LC-MS. After the reaction was completed, the reaction solution was concentrated under reduced pressure. The resulting residue was separated by C18 reverse phase column (eluent: system B) to obtain N-(6-methyl-2-oxo-1-(2,2,2-trifluoroethyl)-5-(2,3,6-trifluorophenyl)piperidin-3-yl)-2'-oxo-1',2',6 ,7-tetrahydro-4H-spiro[benzofuran-5,3'-pyrrolo[2,3-b]pyridine]-2-carboxamide **1** (17 mg), yield: 31.87%.

MS m/z(ESI):606.8[M+1]

¹H NMR (400 MHz, CDCl₃) δ 8.09 (d, *J* = 5.2 Hz, 1H), 7.20-7.07 (m, 2H), 7.00-6.83 (m, 3H), 4.94-4.88 (m, 1H), 4.58-4.47 (dd, *J* = 12.0, 6.8 Hz, 1H), 4.01-3.93 (m, 2H),3.35-3.25 (m, 1H), 3.14 (d, *J* = 16 Hz, 1H), 3.03-2.95 (m, 1H), 2.87-2.71 (m, 3H), 2.53 (d, *J* = 16 Hz, 1H), 2.43-2.35 (m, 1H), 1.95-1.90 (m, 1H), 1.26-1.22 (m, 3H).

### Example 1A

### (R)-N-((3S,5S,6R)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1A

### Example 1B

### (R)-N-((3R,5R,6S)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1B

### Example 1C

### (S)-N-((3S,5S,6R)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1C

### Example 1D

### (S)-N-((3R,5R,6S)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1D

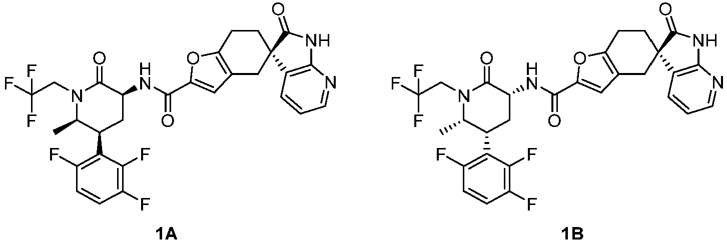

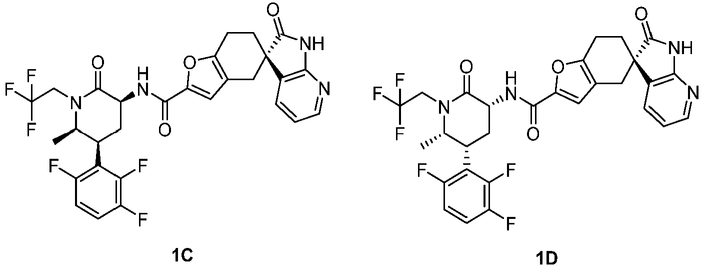

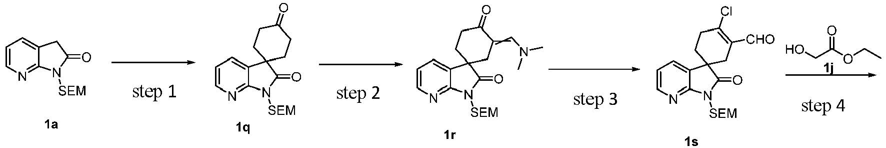

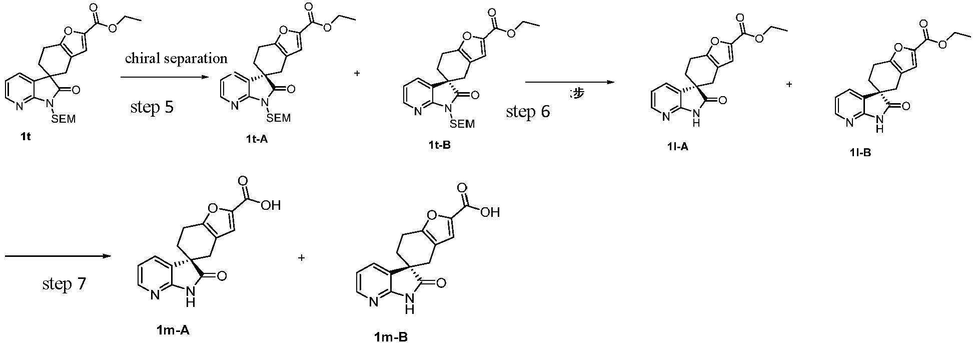

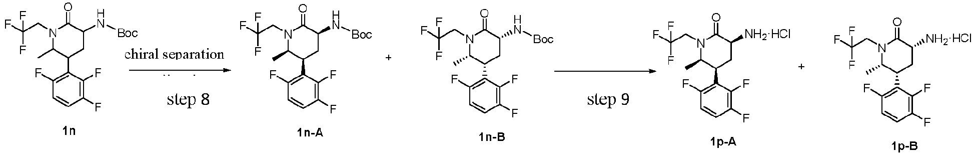

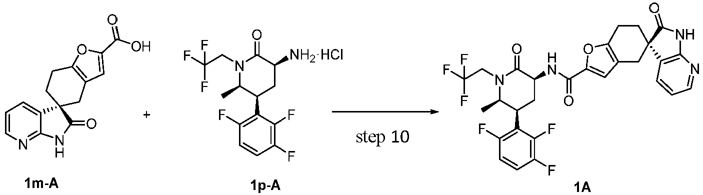

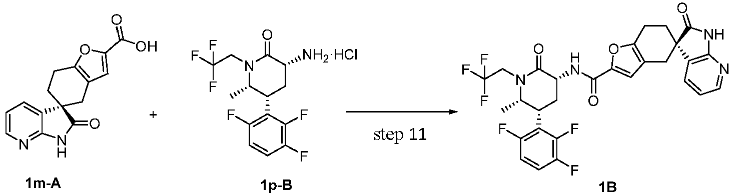

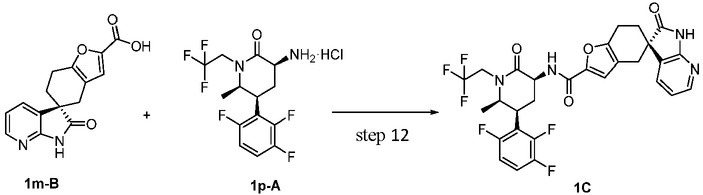

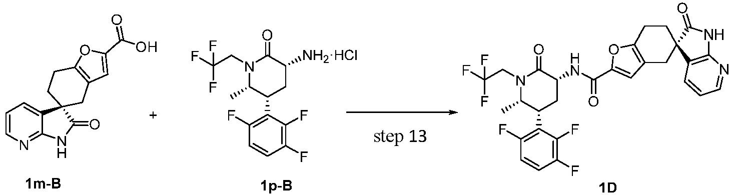

### Step 1

### 1'-((2-(trimethylsilyl)ethoxy)methyl)spiro[cyclohexane-1,3'-pyrrolo[2,3-b]pyridine]-2',4(1'H )-dione

Cesium carbonate (12.3 g, 37.87 mmol) and 1,5-dichloropentan-3-one (2.2 g, 14.19 mmol) were added sequentially to 1-((2-(trimethylsilyl)ethoxy)methyl)-1,3-dihydro-2H-pyrrolo[2,3-b]pyridin-2-one **1a** (2.5 g, 9.46 mmol) in tetrahydrofuran (100 mL) and stirred at 70°C for 48 hours. After the reaction was completed, the reaction solution was cooled to room temperature and filtered. The solid was washed with dichloromethane, and the organic phase was concentrated under reduced pressure. The resulting residue was separated and purified by column chromatography (eluent: system C) to obtain 1'-((2-(trimethylsilyl)ethoxy)methyl)spiro[cyclohexane-1,3'-pyrrolo[2,3-b]pyridine]-2',4(1'H)-di one **1q** (0.5 g), yield: 15.2%.

MS m/z(ESI):347.2[M+1]

¹H NMR (400 MHz, CDCl₃) δ 8.26 (dd, *J =* 1.3, 5.2 Hz, 1H), 7.51 (dd, *J =* 1.4, 7.4 Hz, 1H), 7.02 (dd, *J* = 5.3, 7.3 Hz, 1H), 5.29 (s, 2H), 3.79-3.60 (m, 2H), 3.23-3.05 (m, 2H), 2.50 (td, *J* = 5.3, 15.3 Hz, 2H), 2.30-2.04 (m, 4H), 1.06-0.85 (m, 2H), 0.02 (s, 9H).

### Step 2

### 3-((Dimethylamino)methylene)-1'-((2-(trimethylsilyl)ethoxy)methyl)spiro[cyclohexane-1,3'-pyrrolo[2,3-b]pyridine]-2',4(1'H)-dione

1'-((2-(Trimethylsilyl)ethoxy)methyl)spiro[cyclohexane-1,3'-pyrrolo[2,3-b]pyridine]-2',4(1' H)-dione **1q** (2.14 g, 6.18 mmol) was dissolved in dichloromethane (40 mL), added with 1-tert-butoxy-N,N,N',N'-tetramethylmethanediamine (2.37 g, 13.6 mmol), heated to 60°C and reacted for 16 hours. After the reaction was completed, the reaction solution was concentrated under reduced pressure, and the resulting residue was purified by column chromatography (eluent: System A) to obtain 3-((dimethylamino)methylene)-1'-((2-(trimethylsilyl)ethoxy)methyl)spiro[cyclohexane-1,3'-pyrr olo[2,3-b]pyridine]-2',4(1'H)-dione **1r** (2.46 g), yield: 99%.

MS m/z(ESI):402.3[M+1]

### Step 3

### 4-Chloro-2'-oxo-1'-((2-(trimethylsilyl)ethoxy)methyl)-1',2'-dihydrospiro[cyclohexane-1,3'-p yrrolo[2,3-b]pyridine]-3-ene-3-carbaldehyde

3-((dimethylamino)methylene)-1'-((2-(trimethylsilyl)ethoxy)methyl)spiro[cyclohexane-1,3'-pyrrolo[2,3-b]pyridine]-2',4(1'H)-dione **1r** (2.0 g, 4.98 mmol) was dissolved in dichloromethane (50 mL), added with phosphorus oxychloride (2.37 g, 15.44 mmol) at 0°C, and continuously stirred at 0°C for 1.5 hours. After the reaction was completed, the reaction solution was added with a saturated sodium bicarbonate solution, and extracted with dichloromethane (120 mL×3). The organic phases were combined, dried over anhydrous sodium sulfate, and concentrated under reduced pressure. The resulting residue was separated and purified by column chromatography (eluent: System A) to obtain 4-chloro-2'-oxo-1'-((2-(trimethylsilyl)ethoxy)methyl)-1',2'-dihydrospiro[cyclohexane-1,3'-pyrrol o[2,3-b]pyridine]-3-ene-3-carbaldehyde **1s** (1.47 g), yield: 67.60%.

MS m/z(ESI):393.3[M+1]

### Step 4

### Ethyl

### 2'-oxo-1'-((2-(trimethylsilyl)ethoxy)methyl)-1',2',6,7-tetrahydro-4H-spiro[benzofuran-5,3'-pyrrol o[2,3-b]pyridine]-2- carboxylate

Under nitrogen protection, 4-chloro-2'-oxo-1'-((2-(trimethylsilyl)ethoxy)methyl)-1',2'-dihydrospiro[cyclohexane-1,3'-pyrrol o[2,3-b]pyridine]-3-ene-3-carbaldehyde **1s** (1.37 g, 3.49 mmol) and ethyl 2-hydroxyacetate **1j** (1.63 g, 15.69 mmol) were dissolved in tetrahydrofuran (35 mL), added with sodium hydride (557.83 mg, 13.95 mmol, 60% purity) at 20°C, and reacted at 85°C for 3 hours. After the reaction was completed, the reaction solution was slowly poured into an ice-cold saturated ammonium chloride solution, and extracted with ethyl acetate (100 mL×3). The organic phases were combined, dried over anhydrous sodium sulfate, and concentrated under reduced pressure. The resulting residue was separated and purified by column chromatography (eluent: System A) to obtain ethyl 2'-oxo-1'-((2-(trimethylsilyl)ethoxy)methyl)-1',2',6,7-tetrahydro-4H-spiro[benzofuran-5,3'-pyrrol o[2,3-b]pyridine]-2-carboxylate **1t** (560 mg), yield: 32.66%.

MS m/z(ESI):443.3[M+1]

### Step 5

### Ethyl

### (S)-2'-oxo-1'-((2-(trimethylsilyl)ethoxy)methyl)-1',2',6,7-tetrahydro-4H-spiro[benzofuran-5,3'-py rrolo[2,3-b]pyridine]-2-carboxylate 1t-A

### Ethyl

### (R)-2'-oxo-1'-((2-(trimethylsilyl)ethoxy)methyl)-1',2',6,7-tetrahydro-4H-spiro[benzofuran-5,3'-p yrrolo[2,3-b]pyridine]-2-carboxylate 1t-B

### Ethyl

2'-oxo-1'-((2-(trimethylsilyl)ethoxy)methyl)-1',2',6,7-tetrahydro-4H-spiro[benzofuran-5,3'-pyrrol o[2,3-b]pyridine]-2-carboxylate **1t** (500 mg, 1.13 mmol) was purified by SFC chiral separation (column model: Waters SFC-150, Dnicel IG, 20×250 mm, 10 µm; mobile phase: A for CO2 and B for Ethanol; detection wavelength: 214 nm; column temperature: 40°C) to obtain a compound with single configuration (shorter retention time) and a compound with single configuration (longer retention time).

### Compound with single configuration (short retention time):

180 mg, yield: 32.4%, retention time: 1.128 min, chiral purity: 100% ee.

MS m/z(ESI):443.3[M+1]

### Compound with single configuration (longer retention time):

186 mg, yield: 33.4%, retention time: 1.522 minutes, chiral purity: 100% ee.

MS m/z(ESI):443.2[M+1]

### Step 6

### Ethyl

### (S)-2'-Oxo-1',2',6,7-tetrahydro-4H-spiro[benzofuran-5,3'-pyrrolo[2,3-b]pyridine]-2-carboxylate 11-A

### Ethyl

### (R)-2'-oxo-1',2',6,7-tetrahydro-4H-spiro[benzofuran-5,3'-pyrrolo[2,3-b]pyridine]-2-carboxylate 1l-B

The chirally resolved ethyl (S)-2'-oxo-1'-((2-(trimethylsilyl)ethoxy)methyl)-1',2',6,7-tetrahydro-4H-spiro[benzofuran-5,3'-py rrolo[2,3-b]pyridine]-2-carboxylate **1t-A** (180 mg, 406.32 µmol) or ethyl (R)-2'-oxo-1'-((2-(trimethylsilyl)ethoxy)methyl)-1',2',6,7-tetrahydro-4H-spiro[benzofuran-5,3'-p yrrolo[2,3-b]pyridine]-2-carboxylate **1t-B** (180 mg, 406.32 µmol) was dissolved in tetrahydrofuran (3 mL) and stirred at 25°C for 3 hours. After the reaction was completed, the reaction solution was concentrated under reduced pressure, added with tetrahydrofuran (3 mL) and aqueous ammonia (0.5 mL) respectively, stirred at 25°C for 0.5 h, and concentrated under reduced pressure. The resulting residue was separated and purified by column chromatography (eluent: system A) to obtain ethyl (S)-2'-oxo-1'-((2-(trimethylsilyl)ethoxy)methyl)-1',2',6,7-tetrahydro-4H-spiro[benzofuran-5,3'-py rrolo[2,3-b]pyridine]-2-carboxylate **1t-A** (114 mg), yield: 85%; ethyl (R)-2'-oxo-1'-((2-(trimethylsilyl)ethoxy)methyl)-1',2',6,7-tetrahydro-4H-spiro[benzofuran-5,3'-p yrrolo[2,3-b]pyridine]-2-carboxylate **1t-B** (126 mg), yield: 99%.

MS m/z(ESI):313.2[M+1]

MS m/z(ESI):313.2[M+1]

### Step 7

### (S)-2'-Oxo-1',2',6,7-tetrahydro-4H-spiro[benzofuran-5,3'-pyrrolo[2,3-b]pyridine]-2-carboxyl ic acid 1m-A

### (R)-2'-Oxo-1',2',6,7-tetrahydro-4H-spiro[benzofuran-5,3'-pyrrolo[2,3-b]pyridine]-2-carboxyl ic acid 1m-B

### Ethyl

(S)-2'-oxo-1'-((2-(trimethylsilyl)ethoxy)methyl)-1',2',6,7-tetrahydro-4H-spiro[benzofuran-5,3'-py rrolo[2,3-b]pyridine]-2-carboxylate **1t-A** (114 mg, 365.01 µmol) or ethyl (R)-2'-oxo-1'-((2-(trimethylsilyl)ethoxy)methyl)-1',2',6,7-tetrahydro-4H-spiro[benzofuran-5,3'-p yrrolo[2,3-b]pyridine]-2-carboxylate **1t-B** (126 mg, 403.43 µmol) was dissolved in 4.5 mL of a mixed solution (methanol:tetrahydrofuran: water = 3: 1: 0.5), added with sodium hydroxide (43.80 mg, 1.10 mmol), and stirred at 40 ° C for 1 hour. After the reaction was completed, the reaction solution was adjusted to pH6 with 1M dilute hydrochloric acid, and concentrated under reduced pressure to obtain (S)-2'-Oxo-1',2',6,7-tetrahydro-4H-spiro[benzofuran-5,3'-pyrrolo[2,3-b]pyridine]-2-carboxylic acid **1m-A** (150 mg); (R)-2'-Oxo-1',2',6,7-tetrahydro-4H-spiro[benzofuran-5,3'-pyrrolo[2,3-b]pyridine]-2-carboxylic acid **1m-B** (180 mg), which were directly used for the next step without purification.

MS m/z(ESI):285.1[M+1]

MS m/z(ESI):285.2[M+1]

### Step 8

### tert-Butyl

### ((3 S, 5 S, 6R)-6-methyl-2-oxo-1-(2,2,2-trifluoroethyl)-5-(2,3,6-trifluorophenyl)piperidin-3-yl)carb amate 1n-A

### tert-Butyl

### ((3R,5R,6S)-6-methyl-2-oxo-1-(2,2,2-trifluoroethyl)-5-(2,3,6-trifluorophenyl)piperidin-3-yl)carb amate 1n-B

After purification of tert-butyl (6-methyl-2-oxo-1-(2,2,2-trifluoroethyl)-5-(2,3,6-trifluorophenyl)piperidin-3-yl)carbamate **1n** (2.6 g, 5.90 mmol) by SFC chiral separation (column model: Waters SFC-150, Dnicel IG, 20×250 mm, 10 µm; mobile phase: A for CO2 and B for Ethanol; detection wavelength: 214 nm; column temperature: 40°C), a compound with single configuration (shorter retention time) and a compound with single configuration (longer retention time) were obtained.

### Compound with single configuration (short retention time):

1.3 g, yield: 50%, retention time: 0.900 min, chiral purity: 100% ee.

MS m/z(ESI):462.8[M+1]

### Compound with single configuration (short retention time):

1.2 g, yield: 46%, retention time: 1.239 min, chiral purity: 99.8% ee.

MS m/z(ESI):462.8[M+1]

### Step 9

### (3S,5S,6R)-3-Amino-6-methyl-1-(2,2,2-trifluoroethyl)-5-(2,3,6-trifluorophenyl)piperidin-2-one hydrochloride 1p-A

### (3R,5R,6S)-3-Amino-6-methyl-1-(2,2,2-trifluoroethyl)-5-(2,3,6-trifluorophenyl)piperidin-2-one hydrochloride 1p-B

### tert-Butyl

### ((3 S, 5 S, 6R)-6-methyl-2-oxo-1-(2,2,2-trifluoroethyl)-5-(2,3,6-trifluorophenyl)piperidin-3-yl)carb amate 1n-A (500 mg, 1.14 mmol) or

### tert-Butyl

((3R,5R,6S)-6-methyl-2-oxo-1-(2,2,2-trifluoroethyl)-5-(2,3,6-trifluorophenyl)piperidin-3-yl)carb amate **1n-B** (500 mg, 1.14 mmol) was dissolved in 6 mL of a hydrochloric acid-dioxane solution, and reacted at 25°C for 2 hours. After the reaction was completed, the reaction solution was concentrated under reduced pressure to obtain (3S,5S,6R)-3-Amino-6-methyl-1-(2,2,2-trifluoroethyl)-5-(2,3,6-trifluorophenyl)piperidin-2-one hydrochloride **1p-A** and (3R,5R,6S)-3-Amino-6-methyl-1-(2,2,2-trifluoroethyl)-5-(2,3,6-trifluorophenyl)piperidin-2-one hydrochloride **1p-B,** which were directly used for the next step without purification.

MS m/z(ESI):314.2[M+1]

MS m/z(ESI):314.2[M+1]

### Step 10

### (R)-N-((3S,5S,6R)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1A

(S)-2'-Oxo-1',2',6,7-tetrahydro-4H-spiro[benzofuran-5,3'-pyrrolo[2,3-b]pyridine]-2-carboxyl ic acid **1m-A** (7 mg, 24.62 µmol), (3S,5S,6R)-3-amino-6-methyl-1-(2,2,2-trifluoroethyl)-5-(2,3,6-trifluorophenyl)piperidin-2-one hydrochloride **1p-A** (10.20 mg, 27.09 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol) and stirred at 25°C for 18 hours. After the reaction was completed, the reaction solution was concentrated under reduced pressure and the resulting residue was separated by C18 reverse phase column (eluent: system B) to obtain (R)-N-((3S,5S,6R)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzofuran-5,3'-pyrrolo[2,3-b]pyridine]-2-carboxamide **1A** (7 mg), yield: 46.8%.

MS m/z(ESI):607.3[M+1]

¹H NMR (400 MHz, CDCl₃) δ 8.14 (dd, *J* = 5.2, 1.2 Hz, 1H), 7.14-7.06 (m, 2H), 6.99 (s, 1H), 6.90-6.83 (m, 2H), 4.94-4.82 (m, 1H), 4.60-4.55 (m, 1H), 3.99-3.92 (m, 2H), 3.37-3.27 (m, 1H), 3.11 (d, *J =* 16.0 Hz, 1H), 2.99-2.71 (m, 4H), 2.52 (d, *J =* 8.0 Hz, 1H), 2.41-2.33 (m, 1H), 1.94-1.89 (m, 1H), 1.23 (t, *J =* 6.4 Hz, 3H).

### Step 11

(R)-N-((3R,5R,6S)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide **1B**

(S)-2'-Oxo-1',2',6,7-tetrahydro-4H-spiro[benzofuran-5,3'-pyrrolo[2,3-b]pyridine]-2-carboxyl ic acid **1m-A** (7 mg, 24.62 µmol), (3R,5R,6S)-3-amino-6-methyl-1-(2,2,2-trifluoroethyl)-5-(2,3,6-trifluorophenyl)piperidin-2-one hydrochloride **1p-B** (10.20 mg, 24.62 µmol) g, 27.09 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol) and stirred at 25°C for 18 hours. After the reaction was completed, the reaction solution was concentrated under reduced pressure and the resulting residue was separated by C18 reverse phase column (eluent: system B) to obtain (R)-N-((3R,5R,6S)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzofuran-5,3'-pyrrolo[2,3-b]pyridine]-2-carboxamide **1B** (3.8 mg), yield: 25.4%.

MS m/z(ESI):607.4[M+1]

¹H NMR (400 MHz, CDCl₃) δ 8.13-8.12 (m, 1H), 7.14-7.08 (m, 2H), 6.99 (s, 1H), 6.89-6.83 (m, 2H), 4.94-4.79 (m, 1H), 4.50-4.45 (m, 1H), 3.99-3.92 (m, 2H), 3.35-3.25 (m, 1H), 3.13 (d, *J* = 16.0 Hz, 1H), 3.01-2.76 (m, 4H), 2.52 (d, *J =* 16.0 Hz, 1H), 2.41-2.34 (m, 1H), 1.92 (dd, *J =* 13.2, 5.6 Hz, 1H), 1.25 (t, *J =* 6.4 Hz, 3H).

### Step 12

### (S)-N-((3S,5S,6R)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1C

(R)-2'-Oxo-1',2',6,7-tetrahydro-4H-spiro[benzofuran-5,3'-pyrrolo[2,3-b]pyridine]-2-carboxyl ic acid **1m-B** (7 mg, 24.62 µmol), (3S,5S,6R)-3-amino-6-methyl-1-(2,2,2-trifluoroethyl)-5-(2,3,6-trifluorophenyl)piperidin-2-one hydrochloride **1p-A** (10.20 mg, 24.62 µmol) g, 27.09 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol) and stirred at 25°C for 18 hours. After the reaction was completed, the reaction solution was concentrated under reduced pressure and the resulting residue was separated by C18 reverse phase column (eluent: system B) to obtain (S)-N-((3S,5S,6R)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzofuran-5,3'-pyrrolo[2,3-b]pyridine]-2-carboxamide **1C** (10 mg), yield: 66.9%.

MS m/z(ESI):607.4[M+1]

¹H NMR (400 MHz, CDCl₃) δ 8.14-8.12 (m, 1H), 7.13-7.09 (m, 2H), 6.99 (s, 1H), 6.89-6.84 (m, 2H), 4.92-4.86 (m, 1H), 4.50-4.45 (m, 1H), 3.99-3.92 (m, 2H), 3.32-3.27 (m, 1H), 3.13 (d, *J* = 16.0 Hz, 1H), 3.01-2.73 (m, 4H), 2.52 (d, *J* = 16.0 Hz, 1H), 2.42-2.34 (m, 1H), 1.93-1.89 (m, 1H), 1.25 (t, *J* = 6.4 Hz, 3H).

### Step 13

### (S)-N-((3R,5R,6S)-6-methyl-2-oxo-1-(2,2,2-trifluoroethyl)-5-(2,3,6-trifluorophenyl)piperidi n-3-yl)-2'-oxo-1',2',6,7-tetrahydro-4H-spiro[benzofuran-5,3'-pyrrolo[2,3-b]pyridine]-2-carboxam ide 1D

(R)-2'-Oxo-1',2',6,7-tetrahydro-4H-spiro[benzofuran-5,3'-pyrrolo[2,3-b]pyridine]-2-carboxyl ic acid **1m-B** (7 mg, 24.62 µmol), (3R,5R,6S)-3-amino-6-methyl-1-(2,2,2-trifluoroethyl)-5-(2,3,6-trifluorophenyl)piperidin-2-one hydrochloride **1p-B** (10.20 mg, 27.09 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol), and stirred at 25°C for 18 hours. After the reaction was completed, the reaction solution was concentrated under reduced pressure and the resulting residue was separated by C18 reverse phase column (eluent: system B) to obtain (S)-N-((3R,5R,6S)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzofuran-5,3'-pyrrolo[2,3-b]pyridine]-2-carboxamide **1D** (5 mg), yield: 33.5%.

MS m/z(ESI):607.4[M+1]

¹H NMR (400 MHz, CDCl₃) δ 8.13 (d, *J* = 5.2 Hz, 1H), 7.14-7.07 (m, 2H), 6.99 (s, 1H), 6.91-6.84 (m, 2H), 4.94-4.79 (m, 1H), 4.60-4.55 (m, 1H), 3.99-3.94 (m, 2H), 3.38-3.27 (m, 1H), 3.12 (d, *J* = 16.0 Hz, 1H), 3.00-2.71 (m, 4H), 2.52 (d, *J* = 16.0 Hz, 1H), 2.41-2.33 (m, 1H), 1.94-1.90 (m, 1H), 1.23 (t, *J* = 6.0 Hz, 3H).

### Example 2

### N-(6-methyl-2-oxo-1-(2,2,2-trifluoroethyl)-5-(2,3,6-trifluorophenyl)piperidin-3-yl)-2'-oxo-1' ,2',6,7-tetrahydro-4H-spiro[benzo[b]thiophene-5,3'-pyrrolo[2,3-b]pyridine]-2-carboxamide

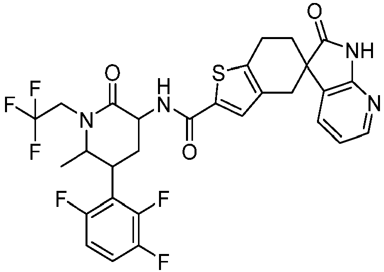

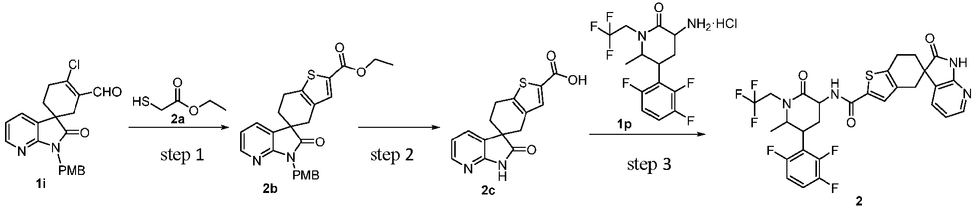

### Step 1

### Ethyl

### 1'-(4-methoxybenzyl)-2'-oxo-1',2',6,7-tetrahydro-4H-spiro[benzo[b]thiophene-5,3'-pyrrolo[2,3-b ]pyridine]-2-carboxylate

4-Chloro-1'-(4-methoxybenzyl)-2'-oxo-1',2'-dihydrospiro[cyclohexane-1,3'-pyrrolo[2,3-b]py ridine]-3-ene-3-carbaldehyde **1i** (150 mg, 391.81 µmol) and ethyl 2-mercaptoacetate **2a** (306.51 mg, 2.55 mmol) were dissolved in N,N-dimethylformamide (4 mL), added with sodium hydride (101.87 mg, 2.55 mmol, 60% purity) at 0°C, stirred for 1 hour under nitrogen protection, then stirred at 25°C for 30 minutes, and monitored by LC-MS. After the reaction was completed, the reaction solution was poured into saturated ammonium chloride solution (5 mL), extracted with ethyl acetate (20 mL×3), dried over anhydrous sodium sulfate, concentrated under reduced pressure, and purified by thin layer plate to obtain ethyl 1'-(4-methoxybenzyl)-2'-oxo-1',2',6,7-tetrahydro-4H-spiro[benzo[b]thiophene-5,3'-pyrrolo[2,3-b ]pyridine]-2-carboxylate **2b** (130 mg), yield: 66.58%.

MS m/z(ESI):449.4[M+1]

¹H NMR (400 MHz, CDCl₃) δ 8.16 (dd, *J* = 5.6, 1.6 Hz, 1H), 7.47 (s, 1H), 7.43 - 7.40 (m, 2H), 6.97 (dd, *J* = 5.6, 1.6 Hz, 1H), 6.84-6.78 (m, 3H), 4.96 (dd, *J* = 14.0, 4.4 Hz, 2H), 4.33 (q, *J* = 7.2 Hz, 2H), 3.76 (s, 3H), 3.24-3.11 (m, 2H), 2.99-2.90 (m, 1H),2.61 (d, *J* = 16.0 Hz, 1H), 2.39-2.31 (m, 1H), 1.82 1.77 (m, 1H), 1.36 (t, *J* = 7.2 Hz, 3H).

### Step 2

### 1'-(4-methoxybenzyl)-2'-oxo-1',2',6,7-tetrahydro-4H-spiro[benzo[b]thiophene-5,3'-pyrrolo[2 ,3-b]pyridine]-2-carboxylic acid

### Ethyl

1'-(4-methoxybenzyl)-2'-oxo-1',2',6,7-tetrahydro-4H-spiro[benzo[b]thiophene-5,3'-pyrrolo[2,3-b ]pyridine]-2-carboxylate **2b** (20 mg, 44.59 µmol) was dissolved in trifluoroacetic acid (0.3 mL) and trifluoromethanesulfonic acid (1 mL), stirred at 130°C for 3 hours, monitored by LC-MS, and after the reaction was completed, the reaction solution was added with water (15 mL), and extracted with ethyl acetate (30 mL×3). The organic phases were combined, dried over anhydrous sodium sulfate, and concentrated under reduced pressure. The resulting residue was separated by C18 reverse phase column preparation (eluent: system B) to obtain 1'-(4-methoxybenzyl)-2'-oxo-1',2',6,7-tetrahydro-4H-spiro[benzo[b]thiophene-5,3'-pyrrolo[2,3-b ]pyridine]-2-carboxylic acid **2c** (10 mg), yield: 67.21%.

MS m/z(ESI):301.1[M+1]

### Step 3

### N-(6-methyl-2-oxo-1-(2,2,2-trifluoroethyl)-5-(2,3,6-trifluorophenyl)piperidin-3-yl)-2'-oxo-1' ,2',6,7-tetrahydro-4H-spiro[benzo[b]thiophene-5,3'-pyrrolo[2,3-b]pyridine]-2-carboxamide

1'-(4-methoxybenzyl)-2'-oxo-1',2',6,7-tetrahydro-4H-spiro[benzo[b]thiophene-5,3'-pyrrolo[2 ,3-b]pyridine]-2-carboxylic acid **2c** (10 mg, 33.30 µmol), 3-amino-6-methyl-1-(2,2,2-trifluoroethyl)-5-(2,3,6-trifluorophenyl)piperidin-2-one hydrochloride **1p** (12.54 mg, 33.30µmol), 1-hydroxybenzotriazole (9.00mg, 66.59µmol) and N,N-diisopropylethylamine (21.52mg, 166.48µmol) were dissolved in N,N-dimethylformamide (0.5mL), added with 1-(3-dimethylaminopropyl)-3-ethylcarbodiimide hydrochloride (12.77mg, 66.59µmol), stirred at 25°C for 18 hours, and monitored by LC-MS. After the reaction was completed, the reaction solution was concentrated under reduced pressure, added with water (10 mL), and extracted with ethyl acetate (30 mL×3). The organic phases were combined, dried over anhydrous sodium sulfate, and concentrated under reduced pressure. The resulting residue was separated by C18 reverse phase column (eluent: system B) to obtain N-(6-methyl-2-oxo-1-(2,2,2-trifluoroethyl)-5-(2,3,6-trifluorophenyl)piperidin-3-yl)-2'-oxo-1',2',6 ,7-tetrahydro-4H-spiro[benzo[b]thiophene-5,3'-pyrrolo[2,3-b]pyridine]-2-carboxamide 2 (6 mg), yield: 27.50%.

MS m/z(ESI):622.9[M+1]

¹H NMR (400 MHz, CDCl₃) δ 8.32-8.23 (m, 1H), 8.14-8.13 (m, 1H), 7.24-7.23 (m, 1H), 7.14-7.00 (m, 3H), 6.90-6.82 (m, 2H), 4.95-4.83 (m, 1H), 4.53-4.47 (m, 1H), 3.99-3.94 (m, 2H), 3.35-3.14 (m, 3H), 3.03-2.93 (m, 1H), 2.83-2.80 (m, 2H), 2.66-2.60 (m, 1H), 2.41-2.33 (m, 1H), 1.95-1.90 (m, 1H), 1.25 (d, *J* = 6.0 Hz, 3H).

### Example 3

### N-(6-methyl-2-oxo-1-(2,2,2-trifluoroethyl)-5-(2,3,6-trifluorophenyl)piperidin-3-yl)-2'-oxo-1' ,2',4,6-tetrahydrospiro[cyclopenta[b]thiophene-5,3'-pyrrolo[2,3-b]pyridine]-2-carboxamide

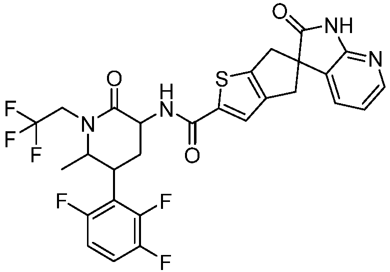

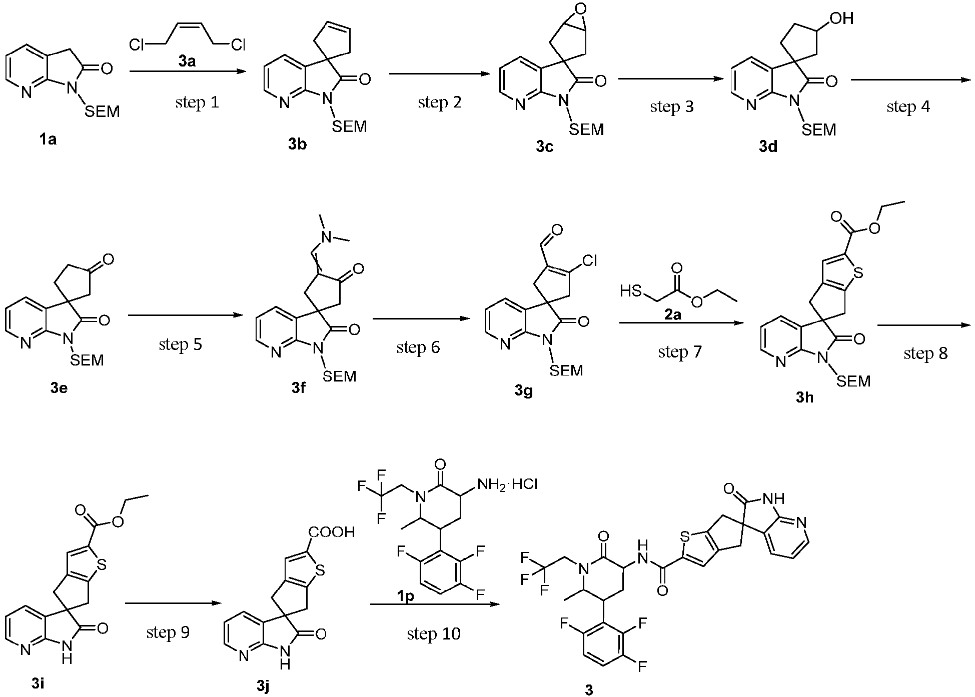

### Step 1

### 1'-((2-(Trimethylsilyl)ethoxy)methyl)spiro[cyclopentane-1,3'-pyrrolo[2,3-b]pyridine]-3-ene-2'(1'H)-one

1-((2-(Trimethylsilyl)ethoxy)methyl)-1,3-dihydro-2H-pyrrolo[2,3-b]pyridin-2-one **1a** (770 mg, 2.91 mmol) was dissolved in N,N-dimethylformamide (12 mL), added with cesium carbonate (2.37 g, 7.28 mmol) and (Z)-1,4-dichlorobut-2-ene **3a** (364.03 mg, 2.91 mmol) at 0°C, stirred at 25°C for 18 hours, added with water (20 mL), and extracted with ethyl acetate (40 mL×3). The organic phases were combined, dried over anhydrous sodium sulfate, and concentrated under reduced pressure. The resulting residue was separated and purified by column chromatography (eluent: system C) to obtain 1'-((2-(trimethylsilyl)ethoxy)methyl)spiro[cyclopentane-1,3'-pyrrolo[2,3-b]pyridine]-3-ene-2'(1' H)-one **3b** (780 mg), yield: 84.63%.

MS m/z(ESI):317.0[M+1]

¹H NMR (400 MHz, DMSO-*d*₆) δ 8.14-8.10 (m, 1H), 7.56-7.44 (m, 1H), 7.04-7.00 (m, 1H), 5.90-5.82 (m, 1H), 5.36-5.05 (m, 3H), 3.59-3.51 (m, 2H), 2.83-2.70 (m, 1H), 2.57-2.51 (m, 1H), 2.18-2.15 (m, 1H), 2.01-1.83 (m, 1H), 0.83-0.77 (m, 2H), 0.13-0.11 (m, 9H).

### Step 2

### 1'-((2-(trimethylsilyl)ethoxy)methyl)-6-oxaspiro[bicyclo[3.1.0]hexane-3,3'-pyrrolo[2,3-b]py ridine]-2'(1'H)-one

1'-((2-(Trimethylsilyl)ethoxy)methyl)spiro[cyclopentane-1,3'-pyrrolo[2,3-b]pyridine]-3-ene-2'(1'H)-one **3b** (23 g, 72.7 mmol) was dissolved in dichloromethane (400 mL), added with m-chloroperbenzoic acid (25.1 g, 14.5 mmol) at low temperature. The reaction was carried out at room temperature for 24 hours under nitrogen protection. After the reaction was completed, the reaction solution was added with dichloromethane (100 mL), and vacuum filtered. The filtrate was washed with sodium thiosulfate solution (200 mL×2) and saturated sodium bicarbonate solution (200 mL), respectively. The organic phase was washed with water (300 mL) and saturated brine (300 mL), dried over anhydrous sodium sulfate, and concentrated under reduced pressure. The resulting residue was separated and purified by column chromatography (eluent: system C) to obtain 1'-((2-(trimethylsilyl)ethoxy)methyl)-6-oxaspiro[bicyclo[3.1.0]hexane-3,3'-pyrrolo[2,3-b]pyridin e]-2'(1'H)-one **3c** (4.9 g), yield: 20.3%.

MS m/z(ESI):333.1[M+1]

### Step 3

### 3-Hydroxy-1'-((2-(trimethylsilyl)ethoxy)methyl)spiro[cyclopentane-1,3'-pyrrolo[2,3-b]pyrid in]-2'(1'H)-one

The obtained 1'-((2-(trimethylsilyl)ethoxy)methyl)-6-oxaspiro[bicyclo[3.1.0]hexane-3,3'-pyrrolo[2,3-b]pyridin e]-2'(1'H)-one **3c** (1.2 g, 3.61 mmol) was dissolved in ethanol (45 mL), added with 10% palladium on carbon (1.20 g, 988.06 µmol), and stirred at 90°C for 24 hours under nitrogen protection. After the reaction was completed, the reaction solution was filtered through diatomite and concentrated under reduced pressure. The resulting residue was separated and purified by column chromatography (eluent: system C) to obtain 3-hydroxy-1'-((2-(trimethylsilyl)ethoxy)methyl)spiro[cyclopentane-1,3'-pyrrolo[2,3-b]pyridine]-2'(1'H)-one **3d** (460 mg), yield: 34.29%.

MS m/z(ESI):335.3[M+1]

¹H NMR (400 MHz, CDCl₃) δ 8.17 (dd, *J* = 5.6, 1.6 Hz, 1H), 7.76 (dd, *J* = 7.6, 1.6 Hz, 1H), 6.98 (dd, *J =* 7.2, 1.2 Hz, 1H), 5.23 (s, 2H), 4.75-4.72 (m, 1H), 3.67-3.63 (m, 2H), 2.45 (dd, *J* = 14.4, 5.2 Hz, 1H), 2.29-2.14 (m, 3H), 2.02-1.95 (m, 1H), 1.89 (dt, *J* = 14.0, 2.4 Hz, 1H), 0.98-0.94 (m, 2H), 0.05 (s, 9H).

### Step 4

### 1'-((2-(Trimethylsilyl)ethoxy)methyl)spiro[cyclopentane-1,3'-pyrrolo[2,3-b]pyridine]-2',3(1' H)-dione

3-Hydroxy-1'-((2-(trimethylsilyl)ethoxy)methyl)spiro[cyclopentane-1,3'-pyrrolo[2,3-b]pyrid ine]-2'(1'H)-one **3d** (740 mg, 2.21 mmol) was dissolved in dichloromethane (20 mL), added with Dess-Martin periodinane (1.97 g, 4.65 mmol), and stirred at 30°C for 2 hours. After the reaction was completed, the reaction solution was concentrated under reduced pressure, and the resulting residue was purified by thin layer plate to obtain 1'-((2-(trimethylsilyl)ethoxy)methyl)spiro[cyclopentane-1,3'-pyrrolo[2,3-b]pyridine]-2',3(1'H)-di one **3e** (640 mg), yield: 78.31%.

MS m/z(ESI):332.9[M+1]

¹H NMR (400 MHz, CDCl₃) δ 8.25 (dd, *J* = 5.2, 1.6 Hz, 1H), 7.43 (dd, *J* = 7.6, 1.6 Hz, 1H), 7.00 (dd, *J* =5.2, 2.0 Hz, 1H), 5.25 (s, 2H), 3.68-3.64 (m, 2H), 2.89-2.76 (m, 2H), 2.61-2.42 (m, 3H), 2.24-2.16 (m, 1H), 0.98-0.93 (m, 2H), 0.05 (s, 9H).

### Step 5

### 3-((Dimethylamino)methylene)-1'-((2-(trimethylsilyl)ethoxy)methyl)spiro[cyclopentane-1,3' -pyrrolo[2,3-b]pyridine]-2',4(1'H)-dione

1'-((2-(Trimethylsilyl)ethoxy)methyl)spiro[cyclopentane-1,3'-pyrrolo[2,3-b]pyridine]-2',3(1' H)-dione **3e** (640 mg, 1.92 mmol) was dissolved in dichloromethane (35 mL), added with 1-tert-butoxy-N,N,N',N'-tetramethylmethanediamine (960 mg, 5.51 mmol), and stirred at 60°C for 3 hours under nitrogen protection. After the reaction was completed, the reaction solution was concentrated under reduced pressure to obtain 3-((dimethylamino)methylene)-1'-((2-(trimethylsilyl)ethoxy)methyl)spiro[cyclopentane-1,3'-pyrr olo[2,3-b]pyridine]-2',4(1'H)-dione **3f** (750 mg), which was directly used for the next step without purification.

MS m/z(ESI):388.0[M+1]

### Step 6

### 3-Chloro-2'-oxo-1'-((2-(trimethylsilyl)ethoxy)methyl)-1',2'-dihydrospiro[cyclopentane-1,3'-p yrrolo[2,3-b]pyridine]-3-ene-4-carbaldehyde

3-((Dimethylamino)methylene)-1'-((2-(trimethylsilyl)ethoxy)methyl)spiro[cyclopentane-1,3' -pyrrolo[2,3-b]pyridine]-2',4(1'H)-dione **3f** (750 mg, 1.94 mmol) was dissolved in dichloromethane (20 mL), added with phosphorus oxychloride (592.19 mg, 3.87 mmol) at 0°C and stirred at 10°C for 20 minutes under nitrogen protection. After the reaction was completed, the reaction solution was added with saturated sodium bicarbonate solution (20 mL), and extracted with dichloromethane (20 mL×3), the organic phases were combined, dried over anhydrous sodium sulfate, and concentrated under reduced pressure. The resulting residue was separated and purified by column chromatography (eluent: system C) to obtain 3-chloro-2'-oxo-1'-((2-(trimethylsilyl)ethoxy)methyl)-1',2'-dihydrospiro[cyclopentane-1,3'-pyrrol o[2,3-b]pyridine]-3-ene-4-carbaldehyde **3g** (200 mg), yield: 21.82%.

MS m/z(ESI):379.3[M+1]

¹H NMR (400 MHz, CDCl₃) δ 10.05 (s, 1H), 8.25 (dd, *J* = 5.2, 1.6 Hz, 1H), 7.47 (dd, *J* = 7.6, 7.2 Hz, 1H), 7.00 (dd, *J* =5.2, 2.0 Hz, 1H), 5.25 (s, 2H), 3.69-3.65 (m, 2H), 3.49 (dt, *J =* 18.8, 2.4 Hz, 1H), 3.19 (dt, *J* = 16.4, 2.0 Hz, 1H), 2.99-2.83 (m, 2H), 0.98-0.94 (m, 2H), 0.03 (s, 9H).

### Step 7

### Ethyl

### 2'-oxo-1'-((2-(trimethylsilyl)ethoxy)methyl)-1',2',4,6-tetrahydrospiro[cyclopenta[b]thiophene-5,3 '-pyrrolo[2,3-b]pyridine-2-carboxylate

3-Chloro-2'-oxo-1'-((2-(trimethylsilyl)ethoxy)methyl)-1',2'-dihydrospiro[cyclopentane-1,3'-p yrrolo[2,3-b]pyridine]-3-ene-4-carbaldehyde **3g** (50 mg, 131.95 µmol) and ethyl 2-mercaptoacetate **2a** (103.07 mg, 857.69 µmol) were dissolved in N,N-dimethylformamide (2.5 mL), added with sodium hydride (34.31 mg, 857.69 µmol, 60% purity) at 0°C, stirred for 1 hour under nitrogen protection and then stirred at 20°C for 1.5 hours. After the reaction was completed, saturated ammonium chloride solution (20 mL) was added to quench the reaction, and the reaction solution was extracted with ethyl acetate (40 mL×3). The organic phases were combined, dried over anhydrous sodium sulfate, and concentrated under reduced pressure. The resulting residue was purified by thin layer plate to obtain ethyl 2'-oxo-1'-((2-(trimethylsilyl)ethoxy)methyl)-1',2',4,6-tetrahydrospiro[cyclopenta[b]thiophene-5,3 '-pyrrolo[2,3-b]pyridine]-2-carboxylate **3h** (36 mg), yield: 58.29%.

MS m/z(ESI):445.3[M+1]

¹H NMR (400 MHz, CDCl₃) δ 8.23 (dd, *J* = 5.6, 1.6 Hz, 1H), 7.57(s, 1H), 7.27 (dd, *J* = 7.6,1.6 Hz, 1H), 6.91 (dd, *J* =7.2, 5.2 Hz, 1H), 5.28 (s, 2H), 4.34 (q, *J =* 6.8 Hz, 2H), 3.71-3.67 (m, 2H), 3.62-3.57 (m, 1H), 3.46-3.42 (m, 1H), 3.07 (d, *J =* 16.4 Hz, 1H), 2.92 (d, *J =* 15.2 Hz, 1H), 1.37 (t, *J* = 6.8 Hz, 3H), 1.00-0.96 (m, 2H), 0.03 (s, 9H).

### Step 8

### Ethyl

### 2'-oxo-1',2',4,6-tetrahydrospiro[cyclopenta[b]thiophene-5,3'-pyrrolo[2,3-b]pyridine]-2-carboxyla te

### Ethyl

2'-oxo-1'-((2-(trimethylsilyl)ethoxy)methyl)-1',2',4,6-tetrahydrospiro[cyclopenta[b]thiophene-5,3 '-pyrrolo[2,3-b]pyridine]-2-carboxylate **3h** (46 mg, 103.46 µmol) was dissolved in trifluoroacetic acid (1 mL) and stirred at 15°C for 2 hours. After the reaction was completed, the reaction solution was concentrated under reduced pressure, added with tetrahydrofuran (1 mL) and ammonium hydroxide (0.5 mL) respectively, stirred at 15°C for 2 hours, and concentrated under reduced pressure. The resulting residue was purified by thin layer plate to obtain ethyl 2'-oxo-1',2',4,6-tetrahydrospiro[cyclopenta[b]thiophene-5,3'-pyrrolo[2,3-b]pyridine]-2-carboxyla te **3i** (20 mg), yield: 55.34%.

MS m/z(ESI):315.2[M+1]

### Step 9

### 2'-Oxo-1',2',4,6-tetrahydrospiro[cyclopenta[b]thiophene-5,3'-pyrrolo[2,3-b]pyridine]-2-carb oxylic acid

### Ethyl

2'-oxo-1',2',4,6-tetrahydrospiro[cyclopenta[b]thiophene-5,3'-pyrrolo[2,3-b]pyridine]-2-carboxyla te **3i** (20 mg, 63.62 µmol) was dissolved in 1 mL of a mixed solution (methanol: water: tetrahydrofuran = 5: 2: 3), added with sodium hydroxide (7.63 mg, 190.86 µmol), and stirred at 40°C for 5 hours. After the reaction was completed, the reaction solution was adjusted to pH5 with 1M hydrochloric acid, and concentrated under reduced pressure to obtain 2'-oxo-1',2',4,6-tetrahydrospiro[cyclopenta[b]thiophene-5,3'-pyrrolo[2,3-b]pyridine]-2-carboxyli c acid **3j** (20 mg), which was directly used for the next step without purification.

### Step 10

### N-(6-methyl-2-oxo-1-(2,2,2-trifluoroethyl)-5-(2,3,6-trifluorophenyl)piperidin-3-yl)-2'-oxo-1' ,2',4,6-tetrahydrospiro[cyclopenta[b]thiophene-5,3'-pyrrolo[2,3-b]pyridine]-2-carboxamide

2'-Oxo-1',2',4,6-tetrahydrospiro[cyclopenta[b]thiophene-5,3'-pyrrolo[2,3-b]pyridine]-2-carb oxylic acid **3j** (9.00 mg, 31.43 µmol), 3-amino-6-methyl-1-(2,2,2-trifluoroethyl)-5-(2,3,6-trifluorophenyl)piperidin-2-one hydrochloride **1p** (11.84 mg, 31.43 µmol), 1-hydroxybenzotriazole (8.50 mg, 62.87 µmol) and N,N-diisopropylethylamine (20.31 mg, 157.17 µmol) were dissolved in N,N-dimethylformamide (0.5mL), then added with 1-(3-dimethylaminopropyl)-3-ethylcarbodiimide hydrochloride (12.05mg, 62.87µmol), and stirred at 20°C for 18 hours, concentrated under reduced pressure, and the resulting residue was separated by C18 reverse phase column preparation (eluent: system B) to obtain N-(6-methyl-2-oxo-1-(2,2,2-trifluoroethyl)-5-(2,3,6-trifluorophenyl)piperidin-3-yl)-2'-oxo-1',2',4 ,6-tetrahydrospiro[cyclopenta[b]thiophene-5,3'-pyrrolo[2,3-b]pyridine]-2-carboxamide 3 (12mg), yield: 59.59%.

MS m/z(ESI):608.7[M+1]

¹H NMR (400 MHz, CDCl₃) δ 8.14-8.13 (m, 1H), 7.36-7.34 (s, 1H), 7.30-7.28 (m, 1H),7.13-7.07 (m, 1H), 6.93-6.83 (m, 2H), 4.91-4.75 (m, 1H), 4.50-4.44 (m, 1H), 3.97-3.94 (m, 2H), 3.61 (d, *J* = 16.0 Hz, 1H), 3.47-3.42 (m, 1H), 3.34-3.26 (m, 1H), 3.09 (d, *J* = 16.4 Hz, 1H), 2.93 (d, *J* = 15.2 Hz, 1H), 2.81-2.76 (m, 2H), 1.24 (d, *J* = 6.0 Hz, 3H).

### Example 3A

### (S)-N-((3S,5S,6R)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3A

### Example 3B

### (S)-N-((3R,5R,6S)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3B

### Example 3C

### (R)-N-((3S,5S,6R)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3C

### Example 3D

### (R)-N-((3R,5R,6S)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3D

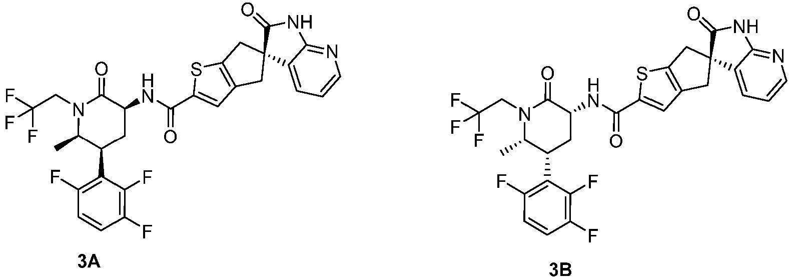

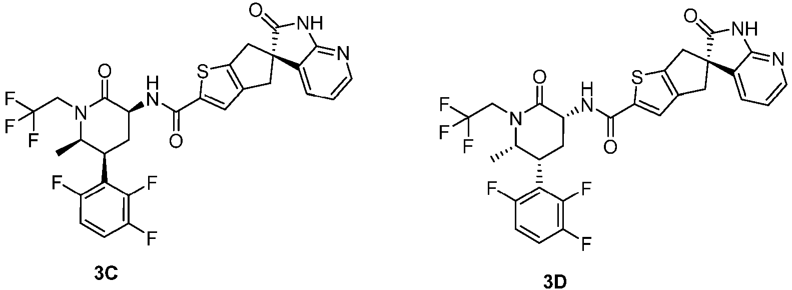

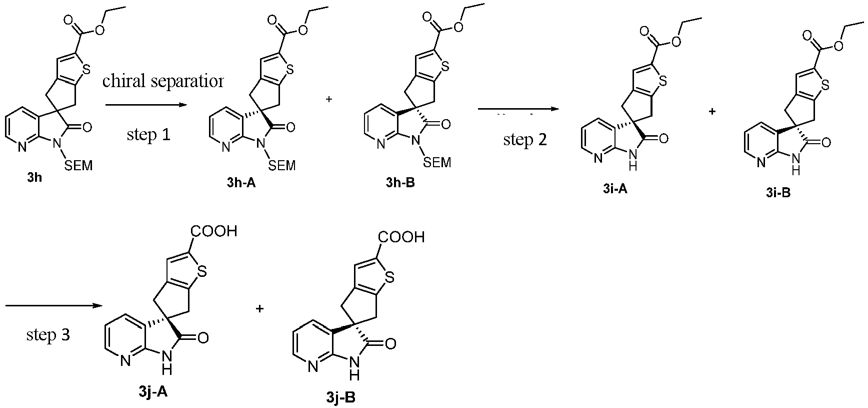

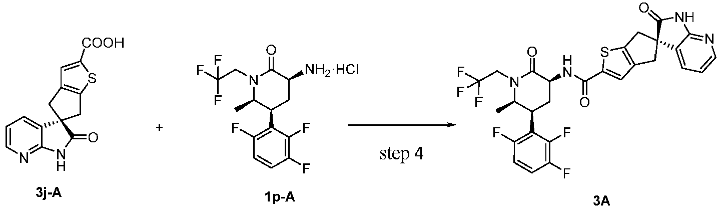

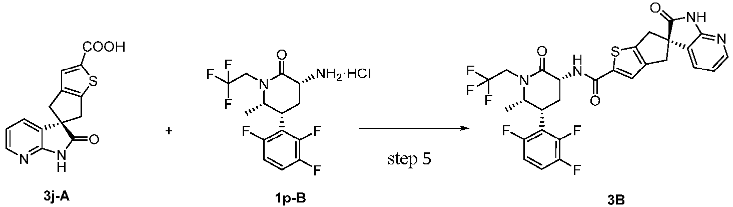

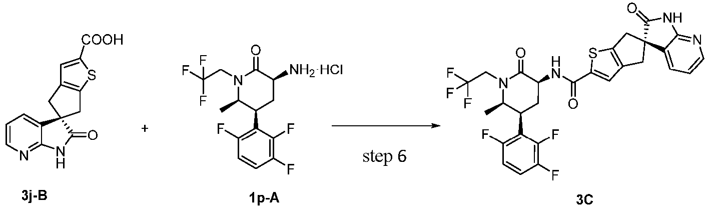

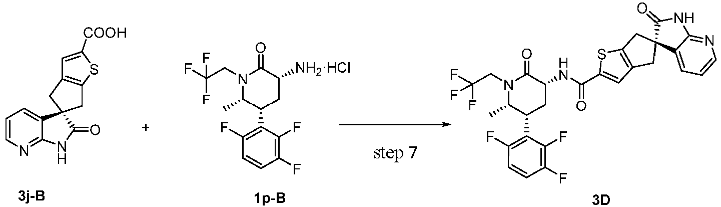

### Step 1

### Ethyl

### (S)-2'-oxo-1'-((2-(trimethylsilyl)ethoxy)methyl)-1',2',4,6-tetrahydrospiro[cyclopenta[b]thiophene -5,3'-pyrrolo[2,3-b]pyridine]-2-carboxylate 3h-A

### Ethyl

### (R)-2'-oxo-1'-((2-(trimethylsilyl)ethoxy)methyl)-1',2',4,6-tetrahydrospiro[cyclopenta[b]thiophen e-5,3'-pyrrolo[2,3-b]pyridine]-2-carboxylate 3h-B

### Ethyl

2'-oxo-1'-((2-(trimethylsilyl)ethoxy)methyl)-1',2',4,6-tetrahydrospiro[cyclopenta[b]thiophene-5,3 '-pyrrolo[2,3-b]pyridine]-2-carboxylate **3h** (410 mg, 0.92 mmol) was purified by SFC chiral separation (column model: Waters SFC-150, Dnicel IG, 20×250 mm, 10 µm; mobile phase: A for CO2 and B for Ethanol; detection wavelength: 214 nm; column temperature: 40°C) to obtain a compound with single configuration (shorter retention time) and a compound with single configuration (longer retention time).

### Compound with single configuration (short retention time):

188 mg, yield: 45.9%, retention time: 2.126 minutes, chiral purity: 100% ee.

MS m/z(ESI):445.3[M+1]

### Compound with single configuration (longer retention time):

184 mg, yield: 44.9%, retention time: 2.845 minutes, chiral purity: 100% ee.

MS m/z(ESI):445.3[M+1]

### Step 2

### Ethyl

### (S)-2'-oxo-1',2',4,6-tetrahydrospiro[cyclopenta[b]thiophene-5,3'-pyrrolo[2,3-b]pyridine]-2-carbo xylate 3i-A

### Ethyl

### (R)-2'-oxo-1',2',4,6-tetrahydrospiro[cyclopenta[b]thiophene-5,3'-pyrrolo[2,3-b]pyridine]-2-carbo xylate 3i-B

### Ethyl

(S)-2'-oxo-1'-((2-(trimethylsilyl)ethoxy)methyl)-1',2',4,6-tetrahydrospiro[cyclopenta[b]thiophene -5,3'-pyrrolo[2,3-b]pyridine]-2-carboxylate **3h-A** (188 mg, 0.92 mmol) or ethyl (R)-2'-oxo-1'-((2-(trimethylsilyl)ethoxy)methyl)-1',2',4,6-tetrahydrospiro[cyclopenta [b]thiophen e-5,3'-pyrrolo[2,3-b]pyridine]-2-carboxylate **3h-B** (184 mg, 0.92 mmol) was dissolved in trifluoroacetic acid (3 mL) and stirred at 25°C for 3 hours. After the reaction was completed, the reaction solution was concentrated under reduced pressure, added with tetrahydrofuran (3 mL) and aqueous ammonia (0.5 mL) respectively, and continuously stirred for 0.5 hour. The resulting residue was separated and purified by column chromatography (eluent: system A) to obtain ethyl (S)-2'-oxo-1',2',4,6-tetrahydrospiro[cyclopenta[b]thiophene-5,3'-pyrrolo[2,3-b]pyridine]-2-carbo xylate **3i-A** (130 mg), yield: 93.24%; ethyl (R)-2'-oxo-1',2',4,6-tetrahydrospiro[cyclopenta[b]thiophene-5,3'-pyrrolo[2,3-b]pyridine]-2-carbo xylate **3i-B** (120 mg), yield: 86.07%.

MS m/z(ESI):314.9[M+1]

MS m/z(ESI):315.1[M+1]

### Step 3

### (S)-2'-Oxo-1',2',4,6-tetrahydrospiro[cyclopenta[b]thiophene-5,3'-pyrrolo[2,3-b]pyridine]-2-c arboxylic acid 3j-A

### (R)-2'-Oxo-1',2',4,6-tetrahydrospiro[cyclopenta[b]thiophene-5,3'-pyrrolo[2,3-b]pyridine]-2-c arboxylic acid 3j-B

### Ethyl

(S)-2'-oxo-1',2',4,6-tetrahydrospiro[cyclopenta[b]thiophene-5,3'-pyrrolo[2,3-b]pyridine]-2-carbo xylate **3i-A** (130 mg, 413.54 µmol) or ethyl (R)-2'-oxo-1',2',4,6-tetrahydrospiro[cyclopenta[b]thiophene-5,3'-pyrrolo[2,3-b]pyridine]-2-carbo xylate **3i-B** (120 mg, 382.17 µmol) was dissolved in 2 mL of a mixed solution (methanol: tetrahydrofuran: water = 3: 1: 1), added with sodium hydroxide (4 9.62 mg, 1.24 mmol), stirred at 40°C for 5 hours, and adjusted to pH5 with 1 M dilute hydrochloric acid. The reaction solution was concentrated under reduced pressure to obtain (S)-2'-oxo-1',2',4,6-tetrahydrospiro[cyclopenta[b]thiophene-5,3'-pyrrolo[2,3-b]pyridine]-2-carbo xylic acid **3j-A** (220 mg), yield: 100%; (R)-2'-oxo-1',2',4,6-tetrahydrospiro[cyclopenta[b]thiophene-5,3'-pyrrolo[2,3-b]pyridine]-2-carbo xylic acid **3j-B** (210 mg), yield: 100%;

MS m/z(ESI):287.1[M+1]

MS m/z(ESI):287.1[M+1]

### Step 4

### (S)-N-((3S,5S,6R)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3A

(S)-2'-Oxo-1',2',4,6-tetrahydrospiro[cyclopenta[b]thiophene-5,3'-pyrrolo[2,3-b]pyridine]-2-c arboxylic acid **3j-A** (10 mg, 34.93 µmol), (3S,5S,6R)-3-amino-6-methyl-1-(2,2,2-trifluoroethyl)-5-(2,3,6-trifluorophenyl)piperidin-2-one hydrochloride **1p-A** (13.16 mg , 34.93µmol), 1-hydroxybenzotriazole (9.44mg, 69.86µmol) and N,N-diisopropylethylamine (22.57mg, 174.64µmol) were dissolved in N,N-dimethylformamide (0.7mL), and added with 1-(3-dimethylaminopropyl)-3-ethylcarbodiimide hydrochloride (13.39mg, 69.86µmol), and the mixture was stirred at 25°C for 18 hours. After the reaction was completed, the reaction solution was concentrated under reduced pressure and the resulting residue was separated by C18 reverse phase column (eluent: system B) to obtain (S)-N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[b]thiophene-5,3'-pyrrolo[2,3-b]pyridine]-2-carbo xamide **3A** (3.59 mg), yield: 16.9%.

MS m/z(ESI):609.0[M+1]

¹H NMR (400 MHz, CDCl₃) δ 8.13 (d, *J* = 5.2 Hz, 1H),7.36 (d, *J* = 3.6Hz, 1H), 7.30-7.27 (m, 1H) , 7.14-7.06 (m, 1H), 6.93-6.82 (m, 2H), 4.94-4.85 (m, 1H), 4.45 (dd, *J* = 10.8, 6.8 Hz, 1H), 3.98-3.90 (m, 2H), 3.61 (d, *J* = 16.0 Hz, 1H), 3.45 (d, *J* = 15.2 Hz, 1H), 3.34-3.25 (m,1H), 3.09 (d, *J =* 16.0 Hz, 1H), 2.93 (d, *J* = 15.2 Hz, 1H), 2.81-2.74 (m, 2H), 1.24 (d, *J* = 4.8 Hz, 3H).

### Step 5

### (S)-N-((3R,5R,6S)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3B

(S)-2'-Oxo-1',2',4,6-tetrahydrospiro[cyclopenta[b]thiophene-5,3'-pyrrolo[2,3-b]pyridine]-2-c arboxylic acid **3j-A** (10 mg, 34.93 µmol), (3R,5R,6S)-3-amino-6-methyl-1-(2,2,2-trifluoroethyl)-5-(2,3,6-trifluorophenyl)piperidin-2-one hydrochloride **1p-B** (13.16 mg, 34.93µmol), 1-hydroxybenzotriazole (9.44mg, 69.86µmol) and N,N-diisopropylethylamine (22.57mg, 174.64µmol) were dissolved in N,N-dimethylformamide (0.7mL), added with 1-(3-dimethylaminopropyl)-3-ethylcarbodiimide hydrochloride (13.39mg, 69.86µmol), and stirred at 25°C for 18 hours. After the reaction was completed, the reaction solution was concentrated under reduced pressure and the resulting residue was separated by C18 reverse phase column (eluent: system B) to obtain (S)-N-((3R,5R,6S)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[b]thiophene-5,3'-pyrrolo[2,3-b]pyridine]-2-carbo xamide **3B** (2.83 mg), yield: 13.3%.

MS m/z(ESI):609.3[M+1]

¹H NMR (400 MHz, CDCl₃) δ 8.11 (dd, *J* = 5.6, 1.6 Hz, 1H), 7.32-7.28 (m, 2H), 7.14-7.06 (m, 1H), 6.91 (dd, *J* = 7.6, 5.6 Hz, 1H), 6.88-6.82 (m,1H), 4.93-4.87(m, 1H), 4.43 (dd, *J* = 10.8, 7.6Hz, 1H), 3.98-3.91 (m,2H), 3.62 (d, *J* =16 Hz, 1H), 3.43 (d, *J* =15.2 Hz, 1H), 3.36-3.26 (m, 1H), 3.09 (d, *J* = 16.4Hz, 1H), 2.92 (d, *J* = 15.2 Hz, 1H), 2.83-2.74 (m, 2H), 1.25 (d, *J* = 6.4 Hz, 3H).

### Step 6

### (R)-N-((3S,5S,6R)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3C

(R)-2'-Oxo-1',2',4,6-tetrahydrospiro[cyclopenta[b]thiophene-5,3'-pyrrolo[2,3-b]pyridine]-2-c arboxylic acid **3j-B** (10 mg, 34.93 µmol), (3S,5S,6R)-3-amino-6-methyl-1-(2,2,2-trifluoroethyl)-5-(2,3,6-trifluorophenyl)piperidin-2-one hydrochloride **1p-A** (13.16 mg, 34.93µmol), 1-hydroxybenzotriazole (9.44mg, 69.86µmol) and N,N-diisopropylethylamine (22.57mg, 174.64µmol) were dissolved in N,N-dimethylformamide (0.7mL), added with 1-(3-dimethylaminopropyl)-3-ethylcarbodiimide hydrochloride (13.39mg, 69.86µmol), and stirred at 25°C for 18 hours. After the reaction was completed, the reaction solution was concentrated under reduced pressure and the resulting residue was separated by C18 reverse phase column (eluent: system B) to obtain (R)-N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[b]thiophene-5,3'-pyrrolo[2,3-b]pyridine]-2-carbo xamide **3C** (4.23 mg), yield: 19.9%.

MS m/z(ESI):609.3[M+1]

¹H NMR (400 MHz, CDCl₃) δ 8.13 (d, *J* = 5.2 Hz, 1H), 7.30-7.27 (m, 2H), 7.13-7.05 (m, 1H), 6.92-6.83 (m, 2H), 4.93-4.85 (m, 1H), 4.45 (t, *J =* 10.8 Hz, 1H), 3.98-3.89 (m, 2H), 3.61 (dd, *J* = 19.6, 4.4 Hz, 1H), 3.43 (dd, *J* = 14.8, 2.8 Hz, 1H), 3.35-3.24 (m,1H), 3.08 (dd, *J* = 16.4, 4.0 Hz, 1H), 2.92 (dd, *J =* 15.2, 4.0 Hz, 1H), 2.80-2.75 (m, 2H), 1.24 (d, *J* = 6.0 Hz, 3H).

### Step 7

### (R)-N-((3R,5R,6S)-6-methyl-2-oxo-1-(2,2,2-trifluoroethyl)-5-(2,3,6-trifluorophenyl)piperidi n-3-yl)-2'-oxo-1',2',4,6-tetrahydrospiro[cyclopenta[b]thiophene-5,3'-pyrrolo[2,3-b]pyridine]-2-ca rboxamide 3D

(R)-2'-Oxo-1',2',4,6-tetrahydrospiro[cyclopenta[b]thiophene-5,3'-pyrrolo[2,3-b]pyridine]-2-c arboxylic acid **3j-B** (10 mg, 34.93 µmol), (3R,5R,6S)-3-amino-6-methyl-1-(2,2,2-trifluoroethyl)-5-(2,3,6-trifluorophenyl)piperidin-2-one hydrochloride **1p-B** (13.16 mg, 34.93µmol), 1-hydroxybenzotriazole (9.44mg, 69.86µmol) and N,N-diisopropylethylamine (22.57mg, 174.64µmol) were dissolved in N,N-dimethylformamide (0.7mL), added with 1-(3-dimethylaminopropyl)-3-ethylcarbodiimide hydrochloride (13.39mg, 69.86µmol), and stirred at 25°C for 18 hours. After the reaction was completed, the reaction solution was concentrated under reduced pressure and the resulting residue was separated by C18 reverse phase column (eluent: system B) to obtain (R)-N-((3R,5R,6S)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[b]thiophene-5,3'-pyrrolo[2,3-b]pyridine]-2-carbo xamide **3D** (8.29 mg), yield: 39.3%.

MS m/z(ESI):609.3[M+1]

¹H NMR (400 MHz, CDCl₃)δ 8.11 (dd, *J =* 5.6, 1.2 Hz, 1H), 7.36-7.32(m, 2H), 7.14-7.06 (m, 1H), 6.98-6.92 (m, 1H), 6.88-6.83 (m, 1H), 4.97-4.87 (m, 1H), 4.43 (dd, *J =* 11.2, 6.8 Hz, 1H), 3.96-3.93 (m, 2H), 3.60 (d, *J* = 16 Hz, 1H), 3.44 (d, *J* = 15.2 Hz, 1H), 3.35-3.25 (m, 1H), 3.08 (d, *J =* 16.4 Hz, 1H), 2.92 (d, *J* = 15.2 Hz, 1H), 2.87-2.72 (m, 2H), 1.25 (d, *J* = 5.6 Hz, 3H).

### Example 4

### N-(6-methyl-2-oxo-1-(2,2,2-trifluoroethyl)-5-(2,3,6-trifluorophenyl)piperidin-3-yl)-2'-oxo-1' ,2',4,6-tetrahydrospiro[cyclopenta[b]furan-5,3'-pyrrolo[2,3-b]pyridine]-2-carboxamide

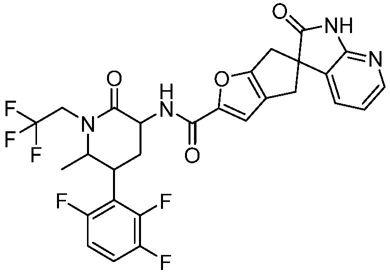

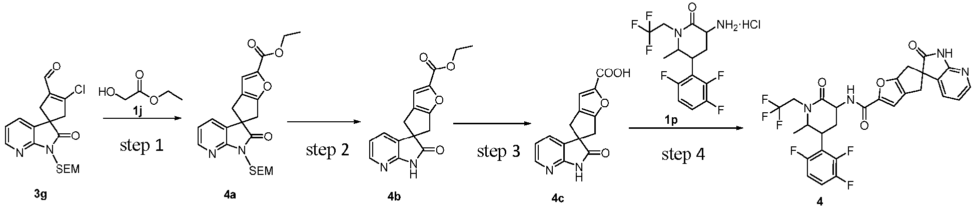

### Step 1

### Ethyl

### 2'-oxo-1'-((2-(trimethylsilyl)ethoxy)methyl)-1',2',4,6-tetrahydrospiro[cyclopenta[b]furan-5,3'-pyr rolo[2,3-b]]pyridine-2-carboxylate

3-Chloro-2'-oxo-1'-((2-(trimethylsilyl)ethoxy)methyl)-1',2'-dihydrospiro[cyclopentane-1,3'-p yrrolo[2,3-b]pyridine]-3-ene-4-carbaldehyde **3g** (120 mg, 316.69 µmol) and ethyl 2-hydroxyacetate **1j** (148 mg, 1.43 mmol) were dissolved in tetrahydrofuran (3 mL), slowly added with sodium hydride (50.66 mg, 1.27 mmol, 60% purity) at room temperature, and stirred at 85°C for 2.5 hours under nitrogen protection. After the reaction was completed, saturated ammonium chloride solution (20 mL) was added to quench the reaction, and the reaction solution was extracted with ethyl acetate (40 mL×3). The organic phases were combined, dried over anhydrous sodium sulfate, and concentrated under reduced pressure. The resulting residue was purified by thin layer plate to obtain ethyl 2'-oxo-1'-((2-(trimethylsilyl)ethoxy)methyl)-1',2',4,6-tetrahydrospiro[cyclopenta[b]furan-5,3'-pyr rolo[2,3-b]]pyridine-2-carboxylate **4a** (43 mg), yield: 31.68%.

MS m/z(ESI):429.3[M+1]

### Step 2

### Ethyl

### 2'-oxo-1',2',4,6-tetrahydrospiro[cyclopenta[b]furan-5,3'-pyrrolo[2,3-b]pyridine]-2-carboxylate

### Ethyl

2'-oxo-1'-((2-(trimethylsilyl)ethoxy)methyl)-1',2',4,6-tetrahydrospiro[cyclopenta[b]furan-5,3'-pyr rolo[2,3-b]]pyridine]-2-carboxylate **4a** (20 mg, 46.67 µmol) was dissolved in trifluoroacetic acid (1 mL) and stirred at 15°C for 2 hours. After the reaction was completed, the reaction solution was concentrated under reduced pressure, added with tetrahydrofuran (1 mL) and ammonium hydroxide (0.5 mL) respectively, stirred at 15°C for 2 hours, and concentrated under reduced pressure. The resulting residue was purified by thin layer plate to obtain ethyl 2'-oxo-1',2',4,6-tetrahydrospiro[cyclopenta[b]furan-5,3'-pyrrolo[2,3-b]pyridine]-2-carboxylate **4b** (13.6 mg), yield: 97.69%.

MS m/z(ESI):299.2[M+1]

### Step 3

### 2'-Oxo-1',2',4,6-tetrahydrospiro[cyclopenta[b]furan-5,3'-pyrrolo[2,3-b]pyridine]-2-carboxyli c acid

### Ethyl

2'-oxo-1',2',4,6-tetrahydrospiro[cyclopenta[b]furan-5,3'-pyrrolo[2,3-b]pyridine]-2-carboxylate **4b** (20 mg, 67.05 µmol) was dissolved in 1 mL of a mixed solution (methanol: water: tetrahydrofuran = 5: 2: 3), added with sodium hydroxide (8.05 mg, 201.14 µmol), and stirred at 40°C for 5 hours. After the reaction was completed, the pH was adjusted to 5 with 1M hydrochloric acid and the reaction solution was concentrated to obtain 2'-oxo-1',2',4,6-tetrahydrospiro[cyclopenta[b]furan-5,3'-pyrrolo[2,3-b]pyridine]-2-carboxylic acid **4c** (18 mg), which was directly used for the next step without purification.

MS m/z(ESI):271.1[M+1]

### Step 4

### N-(6-methyl-2-oxo-1-(2,2,2-trifluoroethyl)-5-(2,3,6-trifluorophenyl)piperidin-3-yl)-2'-oxo-1' ,2',4,6-tetrahydrospiro[cyclopenta[b]furan-5,3'-pyrrolo[2,3-b]pyridine]-2-carboxamide

2'-Oxo-1',2',4,6-tetrahydrospiro[cyclopenta[b]furan-5,3'-pyrrolo[2,3-b]pyridine]-2-carboxyli c acid **4c** (10 mg, 37.00 µmol), 3-amino-6-methyl-1-(2,2,2-trifluoroethyl)-5-(2,3,6-trifluorophenyl)piperidin-2-one hydrochloride **1p** (13.94 mg, 37.00 µmol), 1-hydroxybenzotriazole (5.00 mg, 37.00 µmol) and N,N-diisopropylethylamine (4.78 mg, 37.00 µmol) were dissolved in N,N-dimethylformamide (0.5 mL), added with 1-(3-dimethylaminopropyl)-3-ethylcarbodiimide hydrochloride (7.09 mg, 37.00 µmol), and stirred at 20°C for 18 hours under nitrogen protection. After the reaction was completed, the reaction solution was concentrated under reduced pressure and the resulting residue was separated by C18 reverse phase column (eluent: system B) to obtain N-(6-methyl-2-oxo-1-(2,2,2-trifluoroethyl)-5-(2,3,6-trifluorophenyl)piperidin-3-yl)-2'-oxo-1',2',4 ,6-tetrahydrospiro[cyclopenta[b]furan-5,3'-pyrrolo[2,3-b]pyridine]-2-carboxamide 4 (6 mg), yield: 26%.

MS m/z(ESI):592.7[M+1]

¹H NMR (400 MHz, CDCl₃) δ 8.15 (d, *J* = 5.2 Hz, 1H), 7.43-7.40 (m, 1H), 7.13-7.04 (m, 2H), 6.98-6.83 (m, 2H), 4.93-4.87 (m, 1H), 4.60-4.42 (m, 1H), 3.99-3.91 (m, 2H), 3.44-3.21 (m, 3H), 2.95-2.70 (m, 4H), 1.26-1.22 (m, 3H).

### Example 5A

### (5R)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide 5A

### Example 5B

### (5S)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide 5B

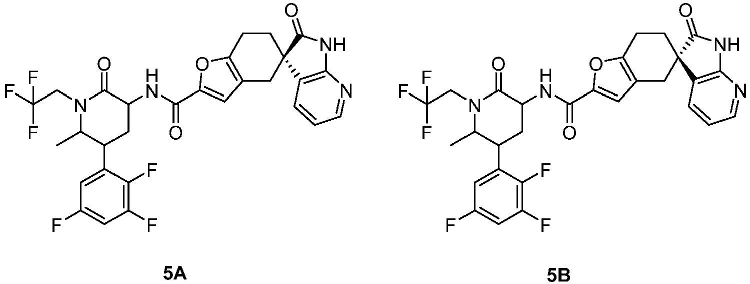

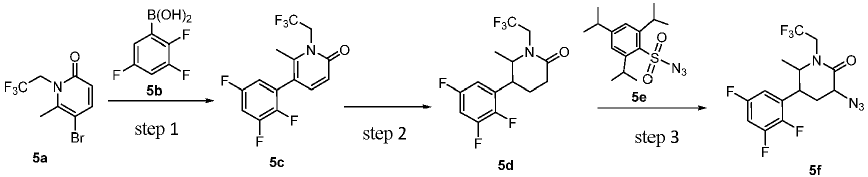

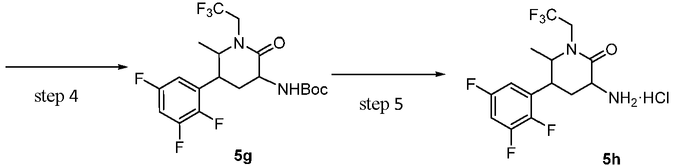

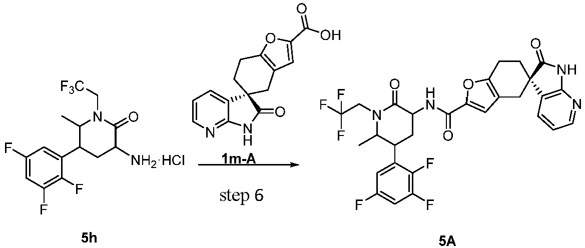

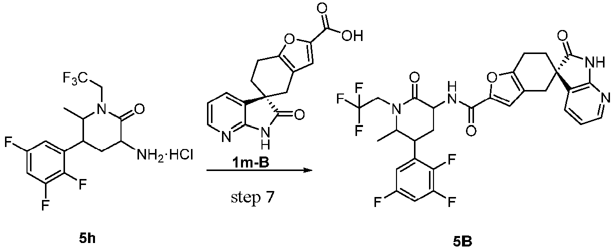

### Step 1

### 6-Methyl-1-(2,2,2-trifluoroethyl)-5-(2,3,5-trifluorophenyl)pyridin-2(1H)-one

Under nitrogen protection, 5-bromo-6-methyl-1-(2,2,2-trifluoroethyl)pyridin-2(1H)-one **5a** (1.0 g, 3.07 mmol), (2,3,5-trifluorophenyl)boronic acid **5b** (1.11 g, 6.30 mmol) and potassium phosphate (2.36 g, 11.11 mmol) were dissolved in tetrahydrofuran (30 mL), added with di(tri-tert-butylphosphine)palladium (284.99 mg, 555.46 µmol), heated to 40°C and stirred for 4 hours. After the reaction was completed, the reaction solution was filtered, concentrated under reduced pressure, and extracted with ethyl acetate (50 mL × 3). The organic phases were combined and concentrated under reduced pressure. The resulting residue was separated and purified by column chromatography (eluent: System A) to obtain 6-methyl-1-(2,2,2-trifluoroethyl)-5-(2,3,5-trifluorophenyl)pyridin-2(1H)-one **5c** (1.2 g), yield: 89.35%.

MS m/z(ESI):322.1[M+1]

### Step 2

### 6-Methyl-1-(2,2,2-trifluoroethyl)-5-(2,3,5-trifluorophenyl)piperidin-2-one

Under hydrogen protection, 6-methyl-1-(2,2,2-trifluoroethyl)-5-(2,3,5-trifluorophenyl)pyridin-2(1H)-one **5c** (3.2 g, 9.96 mmol) was dissolved in acetic acid (200 mL), and then added with platinum dioxide (3.21 g, 14.15 mmol), heated to 80°C, and reacted under high pressure for 48 hours. After the reaction was completed, the reaction solution was filtered, concentrated under reduced pressure, and extracted with ethyl acetate (50 mL×3). The organic phases were combined and concentrated under reduced pressure. The resulting residue was separated and purified by column chromatography (eluent: system A) to obtain 6-methyl-1-(2,2,2-trifluoroethyl)-5-(2,3,5-trifluorophenyl)piperidin-2-one 5d (2.8 g), yield: 86%.

MS m/z(ESI):326.2[M+1]

### Step 3

### 3-Azido-6-methyl-1-(2,2,2-trifluoroethyl)-5-(2,3,5-trifluorophenyl)piperidin-2-one

6-Methyl-1-(2,2,2-trifluoroethyl)-5-(2,3,5-trifluorophenyl)piperidin-2-one **5d** (1.7 g, 5.23 mmol) was dissolved in tetrahydrofuran (50 mL), maintained at -78°C, added with lithium bis(trimethylsilyl)amide (1M, 8.15 mL) dropwise, stirred for 1 hour, slowly added with 15 mL of 2,4,6-triisopropylbenzenesulfonyl azide **5e** (2.26 g, 7.32 mmol) in tetrahydrofuran dropwise, stirred at -78°C for 2 hours, added with acetic acid (8 mL) dropwise, stirred at -78°C for 30 minutes, heated to 25°C, and stirred for 18 hours. After the reaction was completed, the reaction solution was poured into a saturated sodium bicarbonate solution (20 mL), and extracted with ethyl acetate (50 mL×3). The organic phases were combined and concentrated under reduced pressure, and the resulting residue was separated and purified by column chromatography (eluent: System A) to obtain 3-azido-6-methyl-1-(2,2,2-trifluoroethyl)-5-(2,3,5-trifluorophenyl)piperidin-2-one **5f** (2.1 g), yield: 100%.

MS m/z(ESI):366.8[M+1]

### Step 4

### tert-Butyl

### (6-methyl-2-oxo-1-(2,2,2-trifluoroethyl)-5-(2,3,5-trifluorophenyl)piperidin-3-yl)carbamate

Under hydrogen protection, 3-azido-6-methyl-1-(2,2,2-trifluoroethyl)-5-(2,3,5-trifluorophenyl)piperidin-2-one **5f** (590 mg, 1.61 mmol) was dissolved in ethanol (15 mL), added with di-tert-butyl dicarbonate (526.76 mg, 2.42 mmol) and 10% palladium on carbon (190 mg, 156.44 µmol), and stirred at 25°C for 18 hours. After the reaction was completed, the reaction solution was filtered, concentrated under reduced pressure, and extracted with ethyl acetate (50 mL×3). The organic phases were combined and concentrated under reduced pressure. The resulting residue was separated and purified by column chromatography (eluent: system A) to obtain tert-butyl (6-methyl-2-oxo-1-(2,2,2-trifluoroethyl)-5-(2,3,5-trifluorophenyl)piperidin-3-yl)carbamate **5g** (500 mg), yield: 70%.

MS m/z(ESI):462.9[M+23]

### Step 5

### 3-Amino-6-methyl-1-(2,2,2-trifluoroethyl)-5-(2,3,5-trifluorophenyl)piperidin-2-one hydrochloride

5g of tert-butyl (6-methyl-2-oxo-1-(2,2,2-trifluoroethyl)-5-(2,3,5-trifluorophenyl)piperidin-3-yl)carbamate (100 mg, 227.08 µmol) was dissolved in 4M hydrochloric acid dioxane solution (5 mL), and stirred at 25°C for 2 hours. After the reaction was completed, the reaction solution was concentrated under reduced pressure to obtain 3-amino-6-methyl-1-(2,2,2-trifluoroethyl)-5-(2,3,5-trifluorophenyl)piperidin-2-one hydrochloride **5h** (77 mg), yield: 99.7%, which was directly used for the next step without purification.

MS m/z(ESI):341.2[M+1]

### Step 6

### (5R)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide 5A

3-Amino-6-methyl-1-(2,2,2-trifluoroethyl)-5-(2,3,5-trifluorophenyl)piperidin-2-one hydrochloride **5h** (10.20 mg, 27.09 µmol), (S)-2'-oxo-1',2',6,7-tetrahydro-4H-spiro[benzofuran-5,3'-pyrrolo[2,3-b]pyridine]-2-carboxylic acid **1m-A** (7.00 mg, 24.62 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol), and stirred at 20°C for 18 hours. After the reaction was completed, the reaction solution was concentrated under reduced pressure. The resulting residue was separated by C18 reverse phase column (eluent: system B) to obtain (5R)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide **5A** (3.89 mg), yield: 21.78%.

MS m/z(ESI):607.4[M+1]

¹H NMR (400 MHz, CDCl₃) δ 8.14 (dd, *J* = 5.2, 1.2 Hz, 1H), 7.10-7.08 (m, 1H), 7.00-6.99 (m, 1H), 6.91-6.85 (m, 2H), 6.69-6.65 (m, 1H), 4.94-4.88 (m, 1H), 4.56-4.38 (m, 1H), 4.07-3.88 (m, 2H), 3.32-3.25 (m, 1H), 3.13 (d, *J* = 16.0Hz, 1H), 3.01-2.94 (m, 1H), 2.85-2.33 (m, 5H), 1.95-1.90 (m, 1H), 1.13-1.08 (m, 3H).

### Step 7

### (5S)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide 5B

3-Amino-6-methyl-1-(2,2,2-trifluoroethyl)-5-(2,3,5-trifluorophenyl)piperidin-2-one hydrochloride **5h** (10.20 mg, 27.09 µmol), (R)-2'-oxo-1',2',6,7-tetrahydro-4H-spiro[benzofuran-5,3'-pyrrolo[2,3-b]pyridine]-2-carboxylic acid **1m-B** (7 mg, 24.62 µmol), 1-hydroxybenzotriazole (6.65 mg, 49.25 µmol) and N,N-diisopropylethylamine (15.91 mg, 123.12 µmol) were dissolved in N,N-dimethylformamide (0.5 mL), added with 1-(3-dimethylaminopropyl)-3-ethylcarbodiimide hydrochloride (9.44 mg, 49.25 µmol), and stirred at 20°C for 18 hours. After the reaction was completed, the reaction solution was concentrated under reduced pressure. The resulting residue was separated by C18 reverse phase column preparation (eluent: system B) to obtain (5 S)-N-(6-methyl-2-oxo-1-(2,2,2-trifluoroethyl)-5-(2,3,5-trifluorophenyl)piperidin-3-yl)-2'-oxo-1',2',6,7-tetrahydro-4H-spiro[benzofuran-5,3'-pyrrolo[2,3-b]pyridine]-2-carboxamide **5B** (6.86 mg), yield: 38.18%.

MS m/z(ESI):607.3[M+1]

¹H NMR (400 MHz, CDCl₃) δ 8.13 (dd, *J* = 5.2, 1.2 Hz, 1H), 7.11-7.08 (m, 1H), 7.01-6.99 (m, 1H), 6.92-6.85 (m, 2H), 6.69-6.65 (m, 1H), 4.96-4.86 (m, 1H), 4.56-4.38 (m, 1H), 4.06-3.88 (m, 2H), 3.33-3.24 (m, 1H), 3.14 (d,J = 16.0Hz, 1H), 3.02-2.93 (m, 1H), 2.84-2.33 (m, 5H), 1.95-1.90 (m, 1H), 1.13-1.08 (m, 3H).

### Example 6A

### (R)-N-((3S,5S,6R)-6-methyl-2-oxo-5-phenyl-1-(2,2,2-trifluoroethyl)piperidin-3-yl)-2'-oxo-1 ',2',6,7-tetrahydro-4H-Spiro[benzofuran-5,3'-pyrrolo[2,3-b]pyridine]-2-carboxamide 6A

### Example 6B

### (S)-N-((3S,5S,6R)-6-methyl-2-oxo-5-phenyl-1-(2,2,2-trifluoroethyl)piperidin-3-yl)-2'-oxo-1' ,2',6,7-tetrahydro-4H-spiro[benzofuran-5,3'-pyrrolo[2,3-b]pyridine]-2-carboxamide 6B

### Example 6C

### (R)-N-((3R,5R,6S)-6-methyl-2-oxo-5-phenyl-1-(2,2,2-trifluoroethyl)piperidin-3-yl)-2'-oxo-1',2',6,7-tetrahydro-4H-spiro[benzofuran-5,3'-pyrrolo[2,3-b]pyridine]-2-carboxamide 6C

### Example 6D

### (S)-N-((3R,5R,6S)-6-methyl-2-oxo-5-phenyl-1-(2,2,2-trifluoroethyl)piperidin-3-yl)-2'-oxo-1 ',2',6,7-tetrahydro-4H-spiro[benzofuran-5,3'-pyrrolo[2,3-b]pyridine]-2-carboxamide 6D

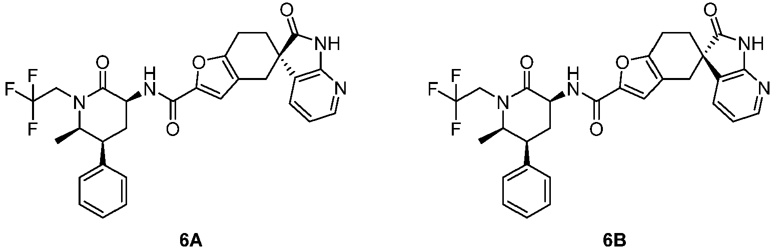

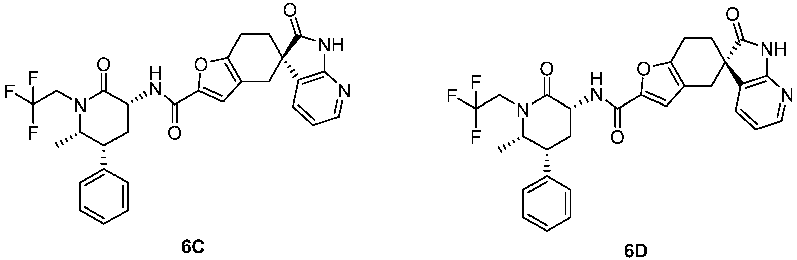

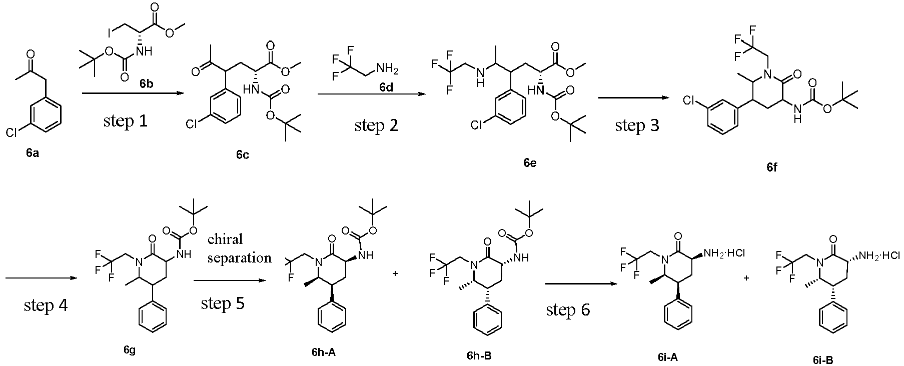

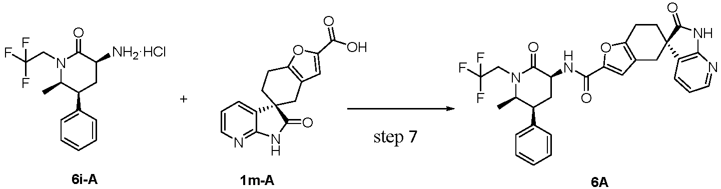

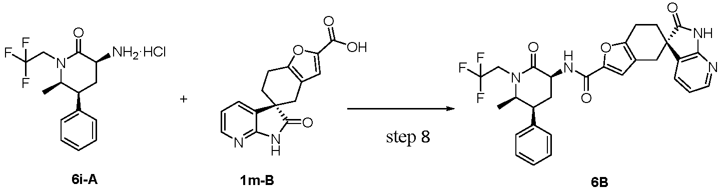

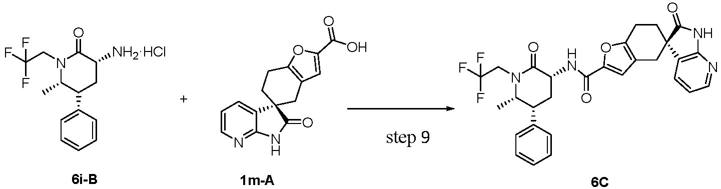

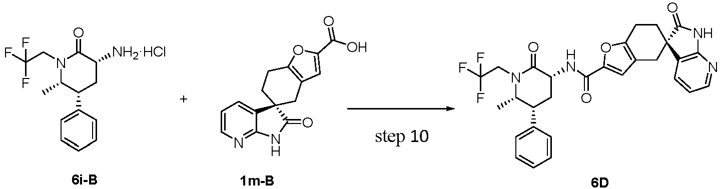

### Step 1

### Methyl (2R)-2-((tert-butyloxycarbonyl)amino)-4-(3-chlorophenyl)-5-oxohexanoate

Methyl (S)-2-((tert-butoxycarbonyl)amino)-3-iodopropionate **6b** (6.34 g, 19.27 mmol) and cesium carbonate (12.59 g, 38.55 mmol) were added to N,N-dimethylformamide (80 mL), the mixture was stirred at 25°C for 1 hour, and then 1-(3-chlorophenyl)propan-2-one 6a (3.9 g, 23.13 mmol) was added, and the mixture was stirred at 25°C for 2.5 hours. After the reaction was completed, the reaction solution was concentrated under reduced pressure, added with water (200 mL), extracted with ethyl acetate (200 mL×2), washed with saturated brine (50 mL), dried over anhydrous sodium sulfate, and concentrated under reduced pressure to obtain methyl (2R)-2-((tert-butoxycarbonyl)amino)-4-(3-chlorophenyl)-5-oxohexanoate **6c** (5.9 g), yield: 82.8%. which was directly used for the next step without purification.

MS m/z(ESI):392.1[M+23]

### Step 2

### Methyl

### (2R)-2-((tert-Butoxycarbonyl)amino)-4-(3-chlorophenyl)-5-((2,2,2-trifluoroethyl)amino)hexanoa te

Methyl (2R)-2-((tert-butoxycarbonyl)amino)-4-(3-chlorophenyl)-5-oxohexanoate **6c** (5.0 g, 13.51 mmol), 2,2,2-trifluoroethane-1-amine **6d** (5.36 g, 54.08 mmol), acetic acid (4.87 g, 81.12 mmol) and sodium triacetoxyborohydride (11.46 g, 54.08 mmol) were added to dichloroethane (130 mL), and reacted at 25°C for 16 hours. After the reaction was completed, the reaction solution was added with dichloromethane (100 mL), washed with water (200 mL×2), dried over anhydrous sodium sulfate, and concentrated under reduced pressure to obtain methyl (2R)-2-((tert-butoxycarbonyl)amino)-4-(3-chlorophenyl)-5-((2,2,2-trifluoroethyl)amino)hexanoa te **6e** (6.15 g), yield: 100%, which was directly used for the next step without purification.

MS m/z(ESI):453.1[M+1]

### Step 3

### tert-Butyl (5-(3-chlorophenyl)-6-methyl-2-oxo-1-(2,2,2-trifluoroethyl)piperidin-3-yl)carbamate

### Methyl

(2R)-2-((tert-butoxycarbonyl)amino)-4-(3-chlorophenyl)-5-((2,2,2-trifluoroethyl)amino)hexanoa te **6e** (6.15 g, 13.58 mmol) was dissolved in ethanol (100 mL), added with potassium carbonate (5.63 g, 40.74 mmol), and reacted at 20°C for 24 hours. After the reaction was completed, the reaction solution was concentrated under reduced pressure, added with water (200 mL), and extracted with ethyl acetate (200 mL×2). The organic phase was washed with saturated brine (50 mL), dried over anhydrous sodium sulfate, and concentrated under reduced pressure. The resulting residue was separated by C18 reverse phase column (eluent: system B) to obtain tert-butyl (5-(3-chlorophenyl)-6-methyl-2-oxo-1-(2,2,2-trifluoroethyl)piperidin-3-yl)carbamate **6f** (1.03 g), yield: 18.02%.

MS m/z(ESI):365.1[M-55]

### Step 4

### tert-Butyl (6-methyl-2-oxo-5-phenyl-1-(2,2,2-trifluoroethyl)piperidin-3-yl)carbamate

Under hydrogen protection, tert-butyl (5-(3-chlorophenyl)-6-methyl-2-oxo-1-(2,2,2-trifluoroethyl)piperidin-3-yl)carbamate **6f** (1.03 g, 2.45 mmol) was dissolved in methanol (20 mL), added with 10% palladium on carbon (0.2 g), and reacted at 25°C for 20 hours. After the reaction was completed, the reaction solution was filtered through diatomite, the filter cake was washed with methanol, and the filtrate was combined, and concentrated under reduced pressure to obtain tert-butyl (6-methyl-2-oxo-5-phenyl-1-(2,2,2-trifluoroethyl)piperidin-3-yl)carbamate 6g (960 mg), yield: 100%, which was directly used for the next step without purification.

MS m/z(ESI):409.3[M+23]

¹H NMR (400 MHz,CDCl₃) δ 7.35-7.31 (m, 2H), 7.27-7.25 (m, 1H), 7.18-7.17 (m, 2H), 5.37 (s, 1H), 4.95-4.84 (m, 1H), 4.18-4.14 (m, 1H), 3.87-3.81 (m, 1H), 3.55-3.50 (m, 1H), 3.26-3.15 (m, 1H), 2.63-2.58 (m, 1H), 2.44-2.35 (m, 1H), 1.45 (s, 9H), 0.95 (t, *J* = 6.8 Hz, 3H).

### Step 5

### tert-Butyl

### ((3S,5S,6R)-6-methyl-2-oxo-5-phenyl-1-(2,2,2-trifluoroethyl)piperidin-3-yl)carbamate 6h-A

### tert-Butyl

### ((3R,5R,6S)-6-methyl-2-oxo-5-phenyl-1-(2,2,2-trifluoroethyl)piperidin-3-yl)carbamate 6h-B

After purification of tert-butyl (6-methyl-2-oxo-5-phenyl-1-(2,2,2-trifluoroethyl)piperidin-3-yl)carbamate **6g** (400 mg, 1.04 mmol) by SFC chiral separation (column model: Waters SFC-150, Dnicel IG, 20×250 mm, 10 µm; mobile phase: A for CO2 and B for Ethanol; detection wavelength: 214 nm; column temperature: 40°C), a compound with single configuration (shorter retention time) and a compound with single configuration (longer retention time) were obtained.

### Compound with single configuration (short retention time):

200 mg, yield: 45%, retention time: 0.761 min, chiral purity: 100% ee.

MS m/z(ESI):409.3[M+23]

### Compound with single configuration (longer retention time):

195 mg, yield: 43.9%, retention time: 2.526 minutes, chiral purity: 100% ee.

MS m/z(ESI):409.3[M+23]

### Step 6

### (3S,5S,6R)-3-Amino-6-methyl-5-phenyl-1-(2,2,2-trifluoroethyl)piperidin-2-one hydrochloride 6i-A

### (3R,5R,6S)-3-Amino-6-methyl-5-phenyl-1-(2,2,2-trifluoroethyl)piperidin-2-one hydrochloride 6i-B

### tert-Butyl

((3S,5S,6R)-6-methyl-2-oxo-5-phenyl-1-(2,2,2-trifluoroethyl)piperidin-3-yl)carbamate **6h-A** (195 mg, 504.65 µmol) or tert-butyl ((3R,5R,6S)-6-methyl-2-oxo-5-phenyl-1-(2,2,2-trifluoroethyl)piperidin-3-yl)carbamate **6h-B** (195 mg, 504.65 µmol) was dissolved in 4M hydrochloric acid dioxane solution (6 mL), stirred at 25°C for 2 hours, and concentrated under reduced pressure to obtain (3S,5S,6R)-3-amino-6-methyl-5-phenyl-1-(2,2,2-trifluoroethyl)piperidine-2-one hydrochloride **6i-A** (150 mg), yield: 100%; (3R,5R,6S)-3-amino-6-methyl-5-phenyl-1-(2,2,2-trifluoroethyl)piperidine-2-one hydrochloride **6i-B** (150 mg), yield: 100%, which was directly subjected to the next step of reaction without purification.

MS m/z(ESI):287.2[M+1]

MS m/z(ESI):287.2[M+1]

### Step 7

### (R)-N-((3S,5S,6R)-6-methyl-2-oxo-5-phenyl-1-(2,2,2-trifluoroethyl)piperidin-3-yl)-2'-oxo-1 ',2',6,7-tetrahydro-4H-spiro[benzofuran-5,3'-pyrrolo[2,3-b]pyridine]-2-carboxamide 6A

(S)-2'-Oxo-1',2',6,7-tetrahydro-4H-spiro[benzofuran-5,3'-pyrrolo[2,3-b]pyridine]-2-carboxyl ic acid **1m-A** (10 mg, 34.93 µmol), (3S,5S,6R)-3-amino-6-methyl-5-phenyl-1-(2,2,2-trifluoroethyl)piperidin-2-one hydrochloride **6i-A** (11.15 mg, 35.18 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (R)-N-((3 S, 5 S,6R)-6-methyl-2-oxo-5-phenyl-1-(2,2,2-trifluoroethyl)piperidin-3-yl)-2'-oxo-1',2', 6,7-tetrahydro-4H-spiro[benzofuran-5,3'-pyrrolo[2,3-b]pyridine]-2-carboxamide **6A** (8.27 mg), yield: 42.55%.

MS m/z(ESI):553.4[M+1]

¹H NMR (400 MHz, CDCl₃) δ 8.13 (d, *J* = 6.0Hz, 1H), 7.34 (t, *J* = 7.2 Hz, 2H), 7.29 (s, 1H), 7.18 (d, *J* = 6.4 Hz, 2H), 7.09 (d, *J* = 8.4Hz, 1H), 6.99 (s, 1H), 6.87 (t, *J* = 8.0 Hz, 1H), 4.98-4.87 (m, 1H), 4.58-4.54(m, 1H), 3.92-3.87 (m, 1H), 3.63 (d, *J* = 12.8 Hz, 1H), 3.29-3.21 (m, 1H), 3.13 (d, *J* = 14.0 Hz, 1H), 3.00-2.93 (m, 1H), 2.84-2.74 (m, 2H), 2.55-2.44 (m, 2H), 2.40-2.33 (m, 1H), 1.95-1.89 (m, 1H), 1.01 (d, *J* = 6.8Hz, 3H).

### Example 6B

### (S)-N-((3S,5S,6R)-6-methyl-2-oxo-5-phenyl-1-(2,2,2-trifluoroethyl)piperidin-3-yl)-2'-oxo-1' ,2',6,7-tetrahydro-4H-spiro[benzofuran-5,3'-pyrrolo[2,3-b]pyridine]-2-carboxamide 6B

(R)-2'-Oxo-1',2',6,7-tetrahydro-4H-spiro[benzofuran-5,3'-pyrrolo[2,3-b]pyridine]-2-carboxyl ic acid **1m-B** (10 mg, 34.93 µmol), (3S,5S,6R)-3-amino-6-methyl-5-phenyl-1-(2,2,2-trifluoroethyl)piperidin-2-one hydrochloride **6i-A** (11.15 mg, 35.18 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (S)-N-((3S,5S,6R)-6-methyl-2-oxo-5-phenyl-1-(2,2,2-trifluoroethyl)piperidin-3-yl)-2'-oxo-1',2',6 ,7-tetrahydro-4H-spiro[benzofuran-5,3'-pyrrolo[2,3-b]pyridine]-2-carboxamide **6B** (7.14 mg), yield: 36.73%.

MS m/z(ESI):553.4[M+1]

¹H NMR (400 MHz, CDCl₃) δ 8.11 (dd, *J =* 5.6, 1.6 Hz, 1H), 7.36-7.32 (m, 2H), 7.29-7.27 (m, 1H), 7.20-7.18 (m, 2H), 7.10 (dd, *J* = 7.2, 1.6 Hz, 1H), 6.98 (d, *J* = 1.2 Hz, 1H), 6.87 (dd, *J* = 7.6, 5.6 Hz, 1H), 4.98-4.90 (m, 1H), 4.25 (t, *J =* 9.6 Hz, 1H), 3.92-3.86 (m, 1H), 3.62-3.57 (m, 1H), 3.31-3.23 (m, 1H), 3.13 (d, *J* = 14.8 Hz, 1H), 3.01-2.95 (m, 1H), 2.84-2.74 (m, 1H), 2.68-2.63 (m, 2H), 2.53 (d, *J =* 16.0 Hz, 1H), 2.41-2.33 (m, 1H), 1.94-1.89 (m,1H), 1.04 (d, *J* = 6.4 Hz, 3H).

### Example 6C

### (R)-N-((3R,5R,6S)-6-methyl-2-oxo-5-phenyl-1-(2,2,2-trifluoroethyl)piperidin-3-yl)-2'-oxo-1',2',6,7-tetrahydro-4H-spiro[benzofuran-5,3'-pyrrolo[2,3-b]pyridine]-2-carboxamide 6C

(S)-2'-Oxo-1',2',6,7-tetrahydro-4H-spiro[benzofuran-5,3'-pyrrolo[2,3-b]pyridine]-2-carboxyl ic acid **1m-A** (10 mg, 34.93 µmol), (3R,5R,6S)-3-amino-6-methyl-5-phenyl-1-(2,2,2-trifluoroethyl)piperidin-2-one hydrochloride **6i-B** (11.15 mg, 35.18 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (R)-N-((3R,5R,6S)-6-methyl-2-oxo-5-phenyl-1-(2,2,2-trifluoroethyl)piperidin-3-yl)-2'-oxo-1',2', 6,7-tetrahydro-4H-spiro[benzofuran-5,3'-pyrrolo[2,3-b]pyridine]-2-carboxamide **6C** (6.52 mg), yield: 33.54%.

MS m/z(ESI):553.3[M+1]

¹H NMR (400 MHz, CDCl₃) δ 8.12 (dd, *J =* 5.2, 1.6 Hz, 1H), 7.36-7.32 (m, 2H), 7.29-7.27 (m, 1H), 7.21-7.17(m, 2H), 7.09 (dd, *J =* 7.2, 1.2 Hz, 1H), 6.98 (d, *J =* 1.6 Hz, 1H), 6.87 (dd, *J* =7.6, 5.2 Hz, 1H), 4.98-4.90 (m, 1H), 4.45-4.40 (m, 1H), 3.92-3.86 (m, 1H), 3.62-3.57 (m, 1H), 3.30-3.20 (m, 1H), 3.13 (d, *J* = 16.8 Hz, 1H), 2.98 (dd, *J* = 17.2, 6.0 Hz, 1H), 2.84-2.75 (m, 1H), 2.68-2.63 (m, 2H), 2.53 (d, *J =* 16.0 Hz, 1H), 2.41-2.33 (m, 1H), 1.94-1.89 (m, 1H), 1.05 (d, *J* = 6.0 Hz, 3H).

### Example 6D

### (S)-N-((3R,5R,6S)-6-methyl-2-oxo-5-phenyl-1-(2,2,2-trifluoroethyl)piperidin-3-yl)-2'-oxo-1 ',2',6,7-tetrahydro-4H-spiro[benzofuran-5,3'-pyrrolo[2,3-b]pyridine]-2-carboxamide 6D

(R)-2'-Oxo-1',2',6,7-tetrahydro-4H-spiro[benzofuran-5,3'-pyrrolo[2,3-b]pyridine]-2-carboxyl ic acid **1m-B** (10 mg, 34.93 µmol), (3R,5R,6S)-3-amino-6-methyl-5-phenyl-1-(2,2,2-trifluoroethyl)piperidin-2-one hydrochloride **6i-B** (11.15 mg, 35.18 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), was added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (S)-N-((3R,5R,6S)-6-methyl-2-oxo-5-phenyl-1-(2,2,2-trifluoroethyl)piperidin-3-yl)-2'-oxo-1',2', 6,7-tetrahydro-4H-spiro[benzofuran-5,3'-pyrrolo[2,3-b]pyridine]-2-carboxamide **6D** (6.68 mg), yield: 34.37%.

MS m/z(ESI):553.4[M+1]

¹H NMR (400 MHz, CDCl₃) δ 8.12 (dd, *J* = 5.2, 1.6 Hz, 1H), 7.34 (t, *J* = 6.8 Hz, 2H), 7.28-7.27 (m,1H), 7.19-7.17 (m, 2H), 7.10 (dd, *J =* 7.6, 1.6 Hz, 1H), 6.98 (d, *J =* 2.8 Hz, 1H), 6.88 (dd, *J =* 7.6, 5.2 Hz, 1H), 4.98-4.87 (m, 1H), 4.56 (q, *J =* 6.8 Hz, 1H), 3.93-3.87 (m, 1H), 3.65-3.61 (m, 1H), 3.32-3.22 (m, 1H), 3.13 (d, *J =* 14.0 Hz, 1H), 3.00-2.94 (m, 1H), 2.85-2.74 (m, 2H), 2.55-2.47 (m, 2H), 2.41-2.33 (m, 1H), 1.94-1.89 (m, 1H), 1.01 (d, *J* = 6.4 Hz, 3H).

### Example 7A

### (5S)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamid e 7A

### Example 7B

### (5R)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamid e 7B

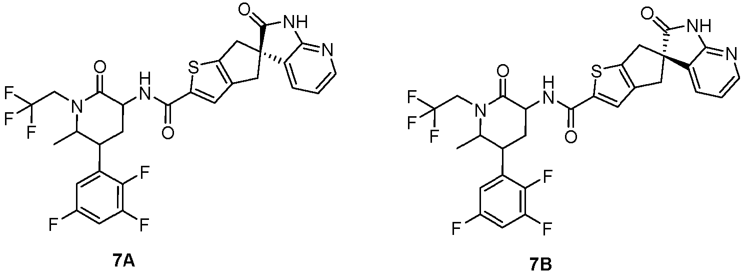

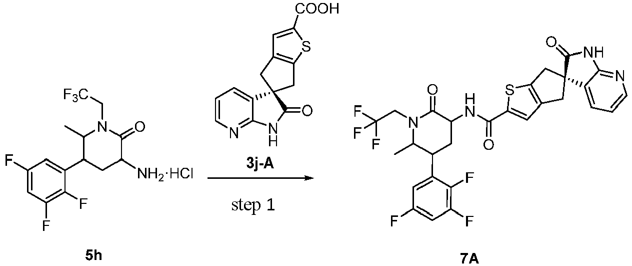

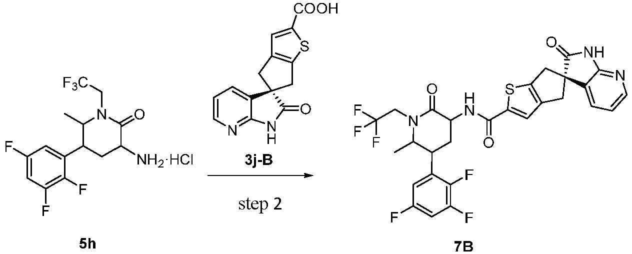

### Step 1

### (5S)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamid e 7A

(S)-2'-Oxo-1',2',4,6-tetrahydrospiro[cyclopenta[b]thiophene-5,3'-pyrrolo[2,3-b]pyridine]-2-c arboxylic acid **3j-A** (10 mg, 34.93 µmol), 3-amino-6-methyl-1-(2,2,2-trifluoroethyl)-5-(2,3,5-trifluorophenyl)piperidin-2-one hydrochloride **5h** (13.16 mg, 34.93 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 20°C for 18 hours. After the reaction was completed, the reaction solution was concentrated under reduced pressure. The resulting residue was separated by C18 reverse phase column (eluent: system B) to obtain (5 S)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamide **7A** (2.33 mg), yield: 10.96%.

MS m/z(ESI):609.4[M+1]

¹H NMR (400 MHz, CDCl₃) δ 8.13-8.12 (m, 1H), 7.36-7.33 (m, 2H), 6.99-6.89 (m, 2H), 6.71-6.68 (m, 1H), 5.07-5.03 (m, 1H), 4.42-4.33 (m, 1H), 4.08-3.89 (m, 2H), 3.67-3.61 (m, 1H), 3.49-3.42 (m, 1H), 3.34-3.25 (m, 1H), 3.13-3.10 (m, 1H), 2.97-2.90 (m, 1H), 2.70-2.58 (m, 2H), 1.13-1.12 (m, 3H).

### Step 2

### (5R)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamid e 7B

(R)-2'-Oxo-1',2',4,6-tetrahydrospiro[cyclopenta[b]thiophene-5,3'-pyrrolo[2,3-b]pyridine]-2-c arboxylic acid **3j-B** (10 mg, 34.93 µmol), 3-amino-6-methyl-1-(2,2,2-trifluoroethyl)-5-(2,3,5-trifluorophenyl)piperidin-2-one hydrochloride **5h** (13.16 mg, 34.93 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-Dimethylaminopropyl)-3-ethylcarbodiimide hydrochloride (13.39 mg, 69.86 µmol) and stirred at 20°C for 18 hours. After the reaction was completed, the reaction solution was concentrated under reduced pressure. The resulting residue was separated by C18 reverse phase column (eluent: system B) to obtain (5R)-N-(6-methyl-2-oxo-1-(2,2,2-trifluoroethyl)-5-(2,3,5-trifluorophenyl)piperidin-3-yl)-2'-oxo-1',2',4,6-tetrahydrospiro[cyclopenta[b]thiophene-5,3'-pyrrolo[2,3-b]pyridine]-2-carboxamide **7B** (10.17 mg), yield: 47.85%.

MS m/z(ESI):609.3[M+1]

¹H NMR (400 MHz, CDCl₃) δ 8.13 (dd, *J* = 5.2, 1.6 Hz, 1H), 7.34 (d, *J* = 6.8 Hz, 1H), 7.29-7.27 (m, 1H), 6.90 (dd, *J* = 7.6, 5.6 Hz, 2H), 6.70 - 6.66 (m, 1H), 4.95-4.93 (m, 1H), 4.44-4.36 (m, 1H), 4.07-4.00 (m, 1H), 3.93-3.88 (m, 1H), 3.64-3.95 (m, 1H), 3.47-3.42 (m, 1H), 3.30-3.24 (m, 1H), 3.10 (d, *J* = 16.0 Hz, 1H), 2.96-2.92 (m, 1H), 2.70-2.52 (m, 2H), 1.11-1.09 (m, 3H).

### Example 8A

### (S)-N-((3S,5S,6R)-6-methyl-2-oxo-5-phenyl-1-(2,2,2-trifluoroethyl)piperidin-3-yl)-2'-oxo-1' ,2',4,6-tetrahydrospiro[cyclopenta[b]thiophene-5,3'-pyrrolo[2,3-b]pyridine]-2-carboxamide 8A

### Example 8B

### (R)-N-((3S,5S,6R)-6-methyl-2-oxo-5-phenyl-1-(2,2,2-trifluoroethyl)piperidin-3-yl)-2'-oxo-1 ',2',4,6-tetrahydrospiro[cyclopenta[b]thiophene-5,3'-pyrrolo[2,3-b]pyridine]-2-carboxamide 8B

### Example 8C

### (S)-N-((3R,5R,6S)-6-methyl-2-oxo-5-phenyl-1-(2,2,2-trifluoroethyl)piperidin-3-yl)-2'-oxo-1 ',2',4,6-tetrahydrospiro[cyclopenta[b]thiophene-5,3'-pyrrolo[2,3-b]pyridine]-2-carboxamide 8C

### Example 8D

### (R)-N-((3R,5R,6S)-6-methyl-2-oxo-5-phenyl-1-(2,2,2-trifluoroethyl)piperidin-3-yl)-2'-oxo-1',2',4,6-tetrahydrospiro[cyclopenta[b]thiophene-5,3'-pyrrolo[2,3-b]pyridine]-2-carboxamide 8D

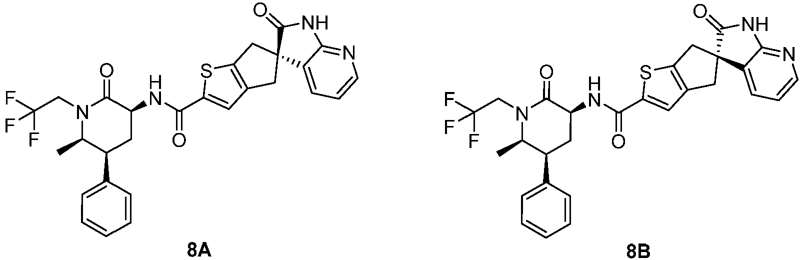

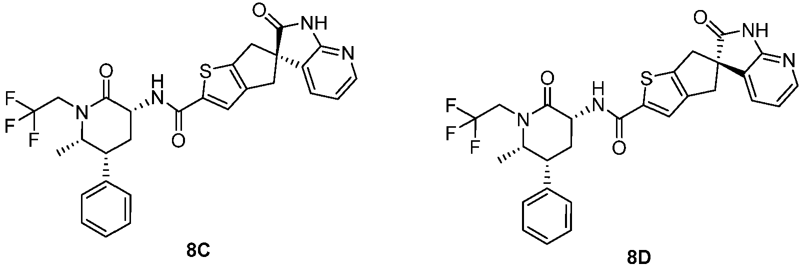

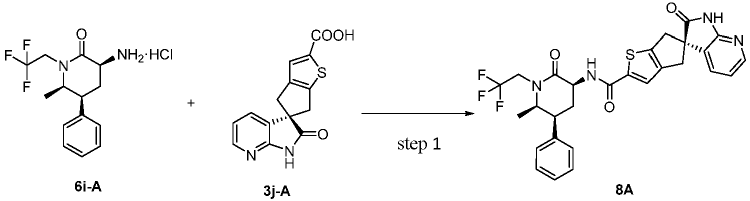

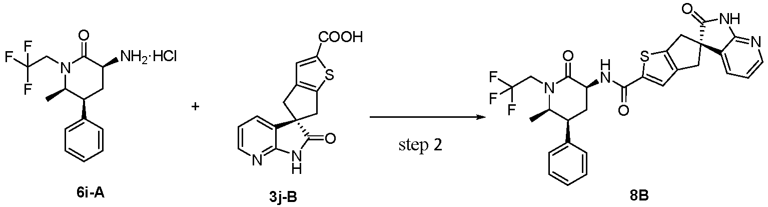

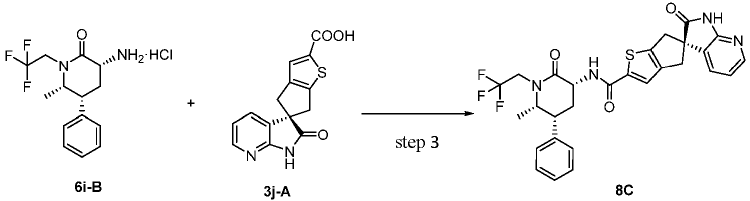

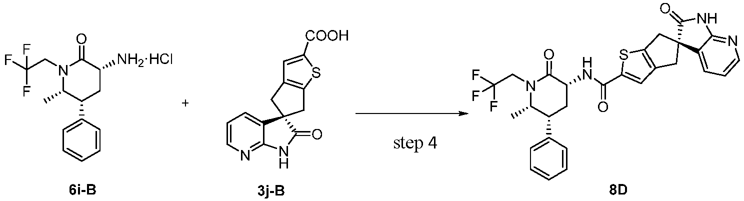

### Step 1

### (S)-N-((3S,5S,6R)-6-methyl-2-oxo-5-phenyl-1-(2,2,2-trifluoroethyl)piperidin-3-yl)-2'-oxo-1' ,2',4,6-tetrahydrospiro[cyclopenta[b]thiophene-5,3'-pyrrolo[2,3-b]pyridine]-2-carboxamide 8A

(S)-2'-Oxo-1',2',4,6-tetrahydrospiro[cyclopenta[b]thiophene-5,3'-pyrrolo[2,3-b]pyridine]-2-c arboxylic acid **3j-A** (10 mg, 34.93 µmol), (3S,5S,6R)-3-amino-6-methyl-5-phenyl-1-(2,2,2-trifluoroethyl)piperidin-2-one hydrochloride **6i-A** (10.15 mg, 31.43 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (S)-N-((3 S, 5 S, 6R)-6-methyl-2-oxo-5-phenyl-1-(2,2,2-trifluoroethyl)piperidin-3 -yl)-2'-oxo-1',2',4 ,6-tetrahydrospiro[cyclopenta[b]thiophene-5,3'-pyrrolo[2,3-b]pyridine]-2-carboxamide **8A** (5.77 mg), yield: 29.79%.

MS m/z(ESI):555.3[M+1]

¹H NMR (400 MHz, CDCl₃) δ 8.13 (dd, *J* = 5.2, 1.6 Hz, 1H), 7.36-7.33 (m, 3H), 7.28-7.26 (m, 2H), 7.25-7.18 (m, 2H), 6.90 (dd, *J* = 7.6, 5.6 Hz, 1H), 4.97-4.87 (m, 1H), 4.40 (dd, *J* = 11.6, 7.2 Hz, 1H), 3.92-3.86 (m, 1H), 3.65-3.58 (m, 2H), 3.43(d, *J* = 15.2 Hz, 1H), 3.31-3.21 (m, 1H), 3.09 (d, *J* = 16.8 Hz, 1H), 2.92 (d, *J* = 15.2 Hz, 1H), 2.75-2.70 (m, 1H), 2.58 (dd, *J =* 25.2, 12.4 Hz, 1H), 1.04(d, *J* = 6.8 Hz, 3H).

### Step 2

### (R)-N-((3S,5S,6R)-6-methyl-2-oxo-5-phenyl-1-(2,2,2-trifluoroethyl)piperidin-3-yl)-2'-oxo-1 ',2',4,6-tetrahydrospiro[cyclopenta[b]thiophene-5,3'-pyrrolo[2,3-b]pyridine]-2-carboxamide 8B

(R)-2'-Oxo-1',2',4,6-tetrahydrospiro[cyclopenta[b]thiophene-5,3'-pyrrolo[2,3-b]pyridine]-2-c arboxylic acid **3j-B** (10 mg, 34.93 µmol), (3S,5S,6R)-3-amino-6-methyl-5-phenyl-1-(2,2,2-trifluoroethyl)piperidin-2-one hydrochloride **6i-A** (10.15 mg, 31.43 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (R)-N-((3 S, 5 S,6R)-6-methyl-2-oxo-5-phenyl-1-(2,2,2-trifluoroethyl)piperidin-3-yl)-2'-oxo-1',2', 4,6-tetrahydrospiro[cyclopenta[b]thiophene-5,3'-pyrrolo[2,3-b]pyridine]-2-carboxamide **8B** (4.88 mg), yield: 25.19%.

MS m/z(ESI):555.3[M+1]

¹H NMR (400 MHz, CDCl₃) δ 8.14-8.12 (m, 1H), 7.36-7.32 (m, 3H), 7.29-7.24 (m, 2H), 7.20-7.18 (m, 2H), 6.92-6.89 (m, 1H), 4.95-4.89 (m, 1H), 6.80 (dd, *J* = 11.6, 6.8 Hz, 1H), 3.91-3.86 (m, 1H), 3.61 (d, *J* = 15.6 Hz, 2H), 3.45 (d, *J* = 15.6 Hz, 1H), 3.28-3.23 (m,1H), 3.10 (d, *J* = 15.6 Hz, 1H), 2.94 (d, *J =* 15.2 Hz, 1H), 2.75-2.71 (m, 1H), 2.60 (dd, *J* =24.4, 12.4 Hz, 1H), 1.04 (d, *J* = 6.8 Hz, 3H).

### Step 3

### (S)-N-((3R,5R,6S)-6-methyl-2-oxo-5-phenyl-1-(2,2,2-trifluoroethyl)piperidin-3-yl)-2'-oxo-1 ',2',4,6-tetrahydrospiro[cyclopenta[b]thiophene-5,3'-pyrrolo[2,3-b]pyridine]-2-carboxamide 8C

(S)-2'-Oxo-1',2',4,6-tetrahydrospiro[cyclopenta[b]thiophene-5,3'-pyrrolo[2,3-b]pyridine]-2-c arboxylic acid **3j-A** (10 mg, 34.93 µmol), (3R,5R,6S)-3-amino-6-methyl-5-phenyl-1-(2,2,2-trifluoroethyl)piperidin-2-one hydrochloride **6i-B** (10.15 mg, 31.43 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (S)-N-((3R,5R,6S)-6-methyl-2-oxo-5-phenyl-1-(2,2,2-trifluoroethyl)piperidin-3-yl)-2'-oxo-1',2', 4,6-tetrahydrospiro[cyclopenta[b]thiophene-5,3'-pyrrolo[2,3-b]pyridine]-2-carboxamide **8C** (5.25 mg), yield: 27.1%.

MS m/z(ESI):555.3[M+1]

¹H NMR (400 MHz, CDCl₃) δ 8.15-8.13 (m, 1H), 7.36-7.32 (m, 3H), 7.29-7.26 (m, 2H), 7.21-7.18 (m, 2H), 6.93-6.89 (m, 1H), 4.98-4.86 (m, 1H), 4.43-4.38 (m, 1H), 3.92-3.86 (m, 1H), 3.60 (d, *J* = 15.6 Hz, 2H), 3.45 (d, *J* = 15.2 Hz, 1H), 3.31-3.20(m, 1H), 3.09 (d, *J* = 16.4 Hz, 1H), 2.94 (d, *J* = 15.2 Hz, 1H), 2.74-2.70 (m, 1H), 2.66-2.56 (m,1H), 1.04 (d, *J =* 6.4 Hz, 3H).

### Step 4

### (R)-N-((3R,5R,6S)-6-methyl-2-oxo-5-phenyl-1-(2,2,2-trifluoroethyl)piperidin-3-yl)-2'-oxo-1',2',4,6-tetrahydrospiro[cyclopenta[b]thiophene-5,3'-pyrrolo[2,3-b]pyridine]-2-carboxamide 8D

(R)-2'-Oxo-1',2',4,6-tetrahydrospiro[cyclopenta[b]thiophene-5,3'-pyrrolo[2,3-b]pyridine]-2-c arboxylic acid **3j-B** (10 mg, 34.93 µmol), (3R,5R,6S)-3-amino-6-methyl-5-phenyl-1-(2,2,2-trifluoroethyl)piperidin-2-one hydrochloride **6i-B** (10.15 mg, 31.43 µmol), 1-hydroxybenzotriazole (9.44 mg, 69.86 µmol) and N,N-diisopropylethylamine (22.57 mg, 174.64 µmol) were dissolved in N,N-dimethylformamide (0.7 mL), added with 1-(3-dimethylaminopropyl)-3-ethylcarbodiimide hydrochloride (13.39 mg, 69.86 µmol), and stirred at 25°C for 18 hours. After the reaction was completed, the reaction solution was concentrated under reduced pressure. The resulting residue was separated by C18 reverse phase column (eluent: system B) to obtain (R)-N-((3R,5R,6S)-6-methyl-2-oxo-5-phenyl-1-(2,2,2-trifluoroethyl)piperidin-3-yl)-2'-oxo-1',2', 4,6-tetrahydrospiro[cyclopenta[b]thiophene-5,3'-pyrrolo[2,3-b]pyridine]-2-carboxamide **8D** (5.6 mg), yield: 28.9%.

MS m/z(ESI):555.3[M+1]

¹H NMR (400 MHz, CDCl₃) δ 8.13 (dd, *J* = 5.2, 1.2 Hz, 1H), 7.36-7.32 (m, 3H), 7.28-7.26 (m, 2H), 7.20-7.18 (m, 2H), 6.90 (dd, *J* = 7.2, 5.2 Hz, 1H), 4.98-4.87 (m, 1H), 4.38-4.34 (m, 1H), 3.92-3.86 (m, 1H), 3.66-3.57 (m, 2H), 3.41 (d, *J=* 15.2 Hz, 1H), 3.21-3.21 (m, 1H), 3.08 (d, *J*= 16.8 Hz, 1H), 2.91 (d, *J=* 15.2 Hz, 1H), 2.71-2.59 (m, 2H), 1.05 (d, *J*= 6.8 Hz, 3H).

### Example 9

### N-((3S,5S,6R)-6-methyl-2-oxo-1-(2,2,2-trifluoroethyl)-5-(2,3,6-trifluorophenyl)piperidin-3-yl)-2'-oxo-1',2',4,7-tetrahydro-5H-spiro[benzo[d]thiazole-6,3'-pyrrolo[2,3-b]pyridine]-2-carboxa mide

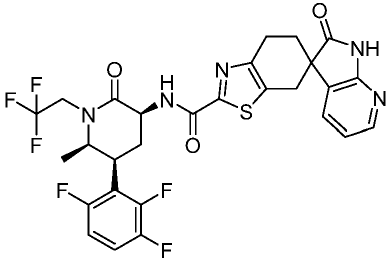

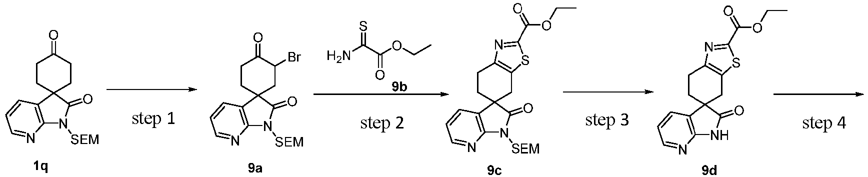

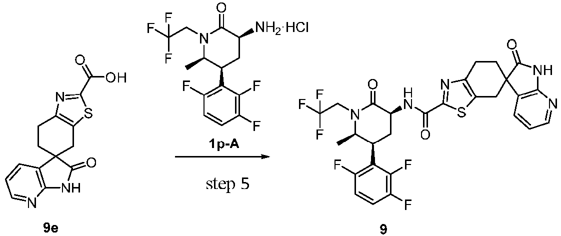

### Step 1

### 3-Bromo-1'-((2-(trimethylsilyl)ethoxy)methyl)spiro[cyclohexane-1,3'-pyrrolo[2,3-b]pyridine ]-2',4(1'H)-dione

1'-((2-(Trimethylsilyl)ethoxy)methyl)spiro[cyclohexane-1,3'-pyrrolo[2,3-b]pyridine]-2',4(1' H)-dione **1q** (1.5g, 4.33 mmol) was dissolved in tetrahydrofuran (30 mL), added with phenyltrimethylammonium tribromide (1.79 g, 4.76 mmol) in portions at 0°C, and stirred at 25°C for 16 hours. After the reaction was completed, the reaction solution was added with 100 mL of water for dilution, and extracted with ethyl acetate (50 mL×3). The organic phases were combined and concentrated under reduced pressure. The resulting residue was separated and purified by column chromatography (eluent: system C) to obtain 3-bromo-1'-((2-(trimethylsilyl)ethoxy)methyl)spiro[cyclohexane-1,3'-pyrrolo[2,3-b]pyridine]-2', 4(1'H)-dione **9a** (1.1 g), yield: 59.7%.

MS m/z(ESI):425.0[M+1]

### Step 2

### Ethyl

### 2'-Oxo-1'-((2-(trimethylsilyl)ethoxy)methyl)-1',2',4,7-tetrahydro-5H-spiro[benzo[d]thiazole-6,3'-pyrrolo[2,3-b]pyridine]-2-carboxylate

3-Bromo-1'-((2-(trimethylsilyl)ethoxy)methyl)spiro[cyclohexane-1,3'-pyrrolo[2,3-b]pyridine ]-2',4(1'H)-dione **9a** (290 mg, 0.6817 mmol) and ethyl 2-amino-2-thioacetate **9b** (99.86 mg, 0.7499 mmol) were added to anhydrous toluene (6 mL), stirred and heated to reflux for 16 hours. After the reaction was completed, the temperature was lowered to room temperature, the reaction solution was added with 10 mL of saturated sodium bicarbonate solution, stirred for 10 minutes, and extracted with ethyl acetate (20 mL×3). The organic phases were combined, washed with saturated brine, dried over anhydrous sodium sulfate, and concentrated under reduced pressure. The resulting residue was purified by thin layer plate to obtain ethyl 2'-oxo-1'-((2-(trimethylsilyl)ethoxy)methyl)-1',2',4,7-tetrahydro-5H-spiro[benzo[d]thiazole-6,3'-pyrrolo[2,3-b]pyridine]-2-carboxylate **9c** (100 mg), yield: 31.9%.

MS m/z(ESI):460.2[M+1]

### Step 3

### Ethyl

### 2'-oxo-1',2',4,7-tetrahydro-5H-spiro[benzo[d]thiazole-6,3'-pyrrolo[2,3-b]pyridine]-2-carboxylate

### Ethyl

2'-oxo-1'-((2-(trimethylsilyl)ethoxy)methyl)-1',2',4,7-tetrahydro-5H-spiro[benzo[d]thiazole-6,3'-pyrrolo[2,3-b]pyridine]-2-carboxylate **9c** (100 mg, 217.57 µmol) was dissolved in trifluoroacetic acid (3 mL) and stirred at 30°C for 3 hours. After the reaction was completed, the reaction solution was concentrated under reduced pressure. The residue was dissolved in tetrahydrofuran (3 mL), stirred at 30°C for 1 hour, and concentrated under reduced pressure. The resulting residue was separated and purified by column chromatography (eluent: System A) to obtain ethyl
2'-Oxo-1',2',4,7-tetrahydro-5H-spiro[benzo[d]thiazole-6,3'-pyrrolo[2,3-b]pyridine]-2-carboxylate **9d** (60 mg), yield: 44.38%.

MS m/z(ESI):330.1[M+1]

### Step 4

### 2'-Oxo-1',2',4,7-tetrahydro-5H-spiro[benzo[d]thiazole-6,3'-pyrrolo[2,3-b]pyridine]-2-carbox ylic acid

### Ethyl

2'-oxo-1',2',4,7-tetrahydro-5H-spiro[benzo[d]thiazole-6,3'-pyrrolo[2,3-b]pyridine]-2-carboxylate **9d** (30 mg, 48.75 µmol) was dissolved in 2 mL of a mixed solution (water: methanol: tetrahydrofuran = 2: 1: 1), added with sodium hydroxide (10.9 mg, 273 µmol), and stirred at 45°C for 1 hour. After the reaction was completed, the reaction solution was adjusted to pH 4 with 1M dilute hydrochloric acid, and concentrated under reduced pressure to obtain 2'-oxo-1',2',4,7-tetrahydro-5H-spiro[benzo[d]thiazole-6,3'-pyrrolo[2,3-b]pyridine]-2-carboxylic acid **9e** (30 mg), which was directly used for the next step without purification.

MS m/z(ESI):302.1[M+1]

### Step 5

### N-((3S,5S,6R)-6-methyl-2-oxo-1-(2,2,2-trifluoroethyl)-5-(2,3,6-trifluorophenyl)piperidin-3-yl)-2'-oxo-1',2',4,7-tetrahydro-5H-spiro[benzo[d]thiazole-6,3'-pyrrolo[2,3-b]pyridine]-2-carboxa mide

2'-Oxo-1',2',4,7-tetrahydro-5H-spiro[benzo[d]thiazole-6,3'-pyrrolo[2,3-b]pyridine]-2-carbox ylic acid **9e** (30 mg, 99.3 µmol), (3S,5S,6R)-3-amino-6-methyl-1-(2,2,2-trifluoroethyl)-5-(2,3,6-trifluorophenyl)piperidin-2-one hydrochloride **1p-A** (28.80 mg, 69.69 µmol), N,N-diisopropylethylamine (128.43 mg, 995.62 µmol) and 1-hydroxybenzotriazole (26.91 mg, 199.12 µmol) were added to N,N-dimethylformamide (2 m L), and then added with 1-(3-dimethylaminopropyl)-3-ethylcarbodiimide hydrochloride (38.03 mg, 199.12 µmol), stirred at 30°C for 16 hours, and concentrated under reduced pressure. The resulting residue was separated by C18 reverse phase column (eluent: system B) to obtain N-((3 S, 5 S, 6R)-6-methyl-2-oxo-1-(2,2,2-trifluoroethyl)-5-(2,3, 6-trifluorophenyl)piperidin-3-yl)-2 '-oxo-1',2',4,7-tetrahydro-5H-spiro[benzo[d]thiazole-6,3'-pyrrolo[2,3-b]pyridine]-2-carboxamide **9** (10.97 mg), yield: 14.19%.

MS m/z(ESI):624.4[M+1]

¹H NMR (400 MHz, DMSO-*d₆*) δ 11.20 (s, 1H), 9.24 (d, *J =* 8.4 Hz, 1H), 8.08 (d, *J* = 4.0 Hz, 1H), 7.53-7.44 (m, 1H), 7.19-7.14 (m, 2H), 6.92-6.89 (m, 1H), 4.72-4.57 (m, 2H), 3.98-3.93 (m, 1H), 3.85-3.74 (m, 2H), 3.23-3.06 (m, 4H), 2.95-2.88 (m, 1H), 2.18-2.14 (m, 2H), 2.01-1.96 (m, 1H), 1.22 (d, *J* = 6.4 Hz, 3H).

### Example 10

### N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydro-1H-spiro[cyclopenta[b]pyrrole-5,3'-pyrrolo[2,3-b]pyridine]-2-carb oxamide

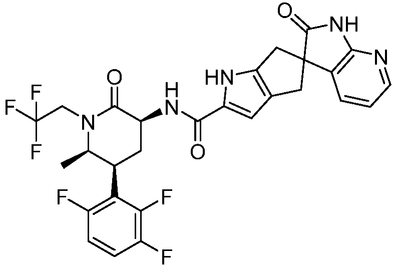

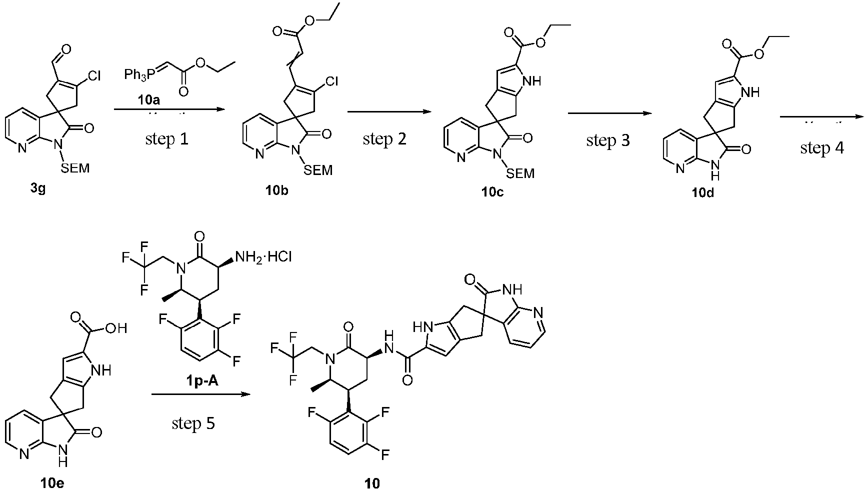

### Step 1

### Ethyl

### 3-(3-chloro-2'-oxo-1'-((2-(trimethylsilyl)ethoxy)methyl)-1',2'-dihydrospiro[cyclopentane-1,3'-py rrolo[2,3-b][pyridin]-3-en-4-yl)acrylate

3-Chloro-2'-oxo-1'-((2-(trimethylsilyl)ethoxy)methyl)-1',2'-dihydrospiro[cyclopentane-1,3'-p yrrolo[2,3-b]pyridine]-3-ene-4-carbaldehyde **3g** (160 mg, 422.25 µmol) and ethyl 2-(triphenyl-λ⁵-phosphite)acetate **10a** (147.10 mg, 422.25 µmol) were dissolved in dichloromethane (4 mL), stirred at 25°C for 3 hours, and concentrated under reduced pressure. The resulting residue was separated and purified by column chromatography (eluent: System A) to obtain ethyl 3-(3-chloro-2'-oxo-1'-((2-(trimethylsilyl)ethoxy)methyl)-1',2'-dihydrospiro[cyclopentane-1,3'-py rrolo[2,3-b]pyridine]-3-en-4-yl)acrylate **10b** (190 mg), yield: 99%.

MS m/z(ESI):449.1[M+1]

### Step 2

### Ethyl

### 2'-oxo-1'-((2-(trimethylsilyl)ethoxy)methyl)-1',2',4,6-tetrahydro-1H-spiro[cyclopenta[b]pyrrole-5,3'-pyrrolo[2,3-b]pyridine]-2-carboxylate

### Ethyl

3-(3-chloro-2'-oxo-1'-((2-(trimethylsilyl)ethoxy)methyl)-1',2'-dihydrospiro[cyclopentane-1,3'-py rrolo[2,3-b]pyridine]-3-en-4-yl)acrylate **10b** (200 mg, 445.42 µmol) was dissolved in dimethyl sulfoxide (5 mL), added with sodium azide (52.11 mg, 801.76 µmol), stirred at 70°C for 18 hours under nitrogen protection, added with 30 mL of ethyl acetate and 20 mL of water, and extracted with ethyl acetate (30 mL×3). The organic phases were combined and concentrated under reduced pressure, and the resulting residue was purified by thin layer plate to obtain ethyl 2'-oxo-1'-((2-(trimethylsilyl)ethoxy)methyl)-1',2',4,6-tetrahydro-1H-spiro[cyclopenta[b]pyrrole-5,3'-pyrrolo[2,3-b]pyridine]-2-carboxylate **10c** (62 mg), yield: 28.65%.

MS m/z(ESI):427.9[M+1]

### Step 3

### Ethyl

### 2'-oxo-1',2',4,6-tetrahydro-1H-spiro[cyclopenta[b]pyrrole-5,3'-pyrrolo[2,3-b]pyridine]-2-carboxy late

### Ethyl

2'-oxo-1'-((2-(trimethylsilyl)ethoxy)methyl)-1',2',4,6-tetrahydro-1H-spiro[cyclopenta[b]pyrrole-5,3'-pyrrolo[2,3-b]pyridine]-2-carboxylate **10c** (62 mg, 145.01 µmol) was dissolved in trifluoroacetic acid (1 mL), stirred at 25°C for 3 hours. After the reaction was completed, the reaction solution was concentrated under reduced pressure, and added with 1 mL of tetrahydrofuran, stirred at 25°C for 0.5 hour and concentrated under reduced pressure. The resulting residue was purified by thin layer plate to obtain ethyl 2'-oxo-1',2',4,6-tetrahydro-1H-spiro[cyclopenta[b]pyrrole-5,3'-pyrrolo[2,3-b]pyridine]-2-carboxy late **10d** (23 mg), yield: 48.55%.

MS m/z(ESI):298.0[M+1]

### Step 4

### 2'-Oxo-1',2',4,6-tetrahydro-1H-spiro[cyclopenta[b]pyrrole-5,3'-pyrrolo[2,3-b]pyridine]-2-car boxylic acid

### Ethyl

2'-oxo-1',2',4,6-tetrahydro-1H-spiro[cyclopenta[b]pyrrole-5,3'-pyrrolo[2,3-b]pyridine]-2-carboxy late **10d** (23 mg, 80.30 µmol) was dissolved in 2 mL of a mixed solution (water: methanol: tetrahydrofuran = 2: 1: 1), added with sodium hydroxide (9.64 mg, 240.90 µmol), and stirred at 40°C for 2 hours. After the reaction was completed, the reaction solution was adjusted to pH 3 with 1M dilute hydrochloric acid, and concentrated under reduced pressure to obtain 2'-oxo-1',2',4,6-tetrahydro-1H-spiro[cyclopenta[b]pyrrole-5,3'-pyrrolo[2,3-b]pyridine]-2-carboxy lic acid **10e** (20 mg), which was directly used for the next step without purification.

MS m/z(ESI):269.9[M+1]

### Step 5

### N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydro-1H-spiro[cyclopenta[b]pyrrole-5,3'-pyrrolo[2,3-b]pyridine]-2-carb oxamide

2'-Oxo-1',2',4,6-tetrahydro-1H-spiro[cyclopenta[b]pyrrole-5,3'-pyrrolo[2,3-b]pyridine]-2-car boxylic acid **10e** (20 mg, 74.28 µmol), (3S,5S,6R)-3-amino-6-methyl-1-(2,2,2-trifluoroethyl)-5-(2,3,6-trifluorophenyl)piperidin-2-one hydrochloride **1p-A** (50.37 mg, 133.70 µmol), 1-hydroxybenzotriazole (20.07 mg, 148.56 µmol) and N,N-diisopropylethylamine (96 mg, 742.79 µmol) were dissolved in N,N-dimethylformamide (1 mL), added with 1-(3-dimethylaminopropyl)-3-ethylcarbodiimide hydrochloride (26.39 mg, 148.56 µmol), and stirred at 25°C for 16 hours. After the reaction was completed, the reaction solution was concentrated under reduced pressure. The resulting residue was separated by C18 reverse phase column (eluent: system B) to obtain N-((3S,5S,6R)-6-methyl-2-oxo-1-(2,2,2-trifluoroethyl)-5-(2,3,6-trifluorophenyl)piperidin-3-yl)-2 '-oxo-1',2',4,6-tetrahydro-1H-spiro[cyclopenta[b]pyrrole-5,3'-pyrrolo[2,3-b]pyridine]-2-carboxa mide **10** (6.57 mg), yield: 11.91%.

MS m/z(ESI):591.8[M+1]

¹H-NMR (400 MHz, DMSO-*d₆*) δ 11.32 (s, 1H), 11.05 (s, 1H), 8.43-8.40 (m, 1H), 8.07-8.06 (m, 1H), 7.51-7.39 (m, 2H), 7.20-7.15 (m, 1H), 6.93-6.90 (m, 1H), 6.59 (s, 1H), 4.70-4.62 (m, 1H), 4.36-4.28 (m, 1H), 3.96-3.92 (m, 1H), 3.79-3.76 (m, 2H), 3.11-2.99 (m, 3H), 2.82 (d, *J* = 16.0 Hz, 1H), 2.71 (d, *J* = 14.4 Hz, 1H), 2.16-2.10 (m, 1H), 1.26-1.23 (m, 3H).

### Example 11

### N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[d]thiazole-5,3'-pyrrolo[2,3-b]pyridine]-2-carboxa mide

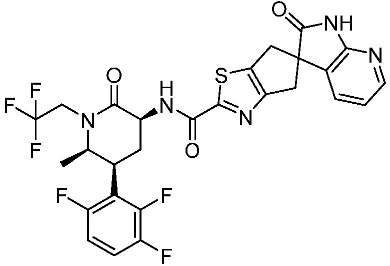

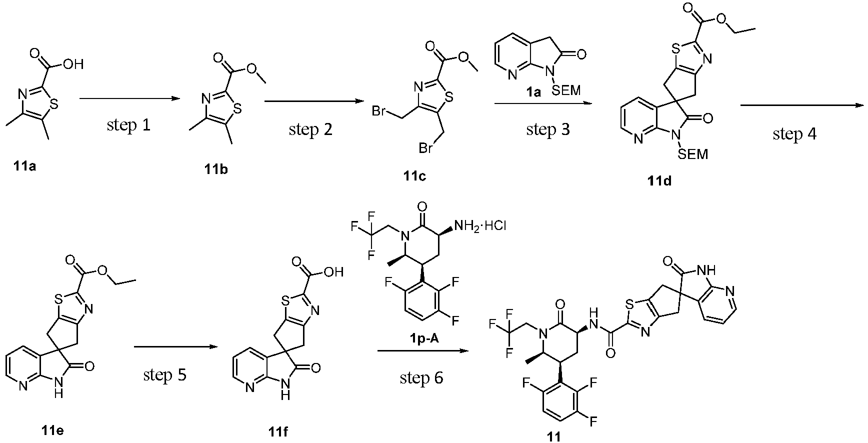

### Step 1

### Methyl 4,5-dimethylthiazole-2-carboxylate

4,5-Dimethylthiazole-2-carboxylic acid **11a** (900 mg, 5.73 mmol) and N,N-dimethylformamide (83.70 mg, 1.15 mmol) were dissolved in methanol (20 mL), and slowly added dropwise with oxalyl chloride (1.02 g, 8.02 mmol) at 0°C, and continuously stirred at 0°C for 2 hours. After the reaction was completed, the reaction solution was concentrated under reduced pressure. The resulting residue was separated and purified by column chromatography (eluent: system A) to obtain methyl 4,5-dimethylthiazole-2-carboxylate **11b** (618 mg), yield: 56.74%.

MS m/z(ESI): 172. 1[M+1]

### Step 2

### Methyl 4,5-bis(bromomethyl)thiazole-2-carboxylate

Methyl 4,5-dimethylthiazole-2-carboxylate **11b** (618 mg, 3.61 mmol) and azobisisobutyronitrile (355.62 mg, 2.17 mmol) were dissolved in dichloroethane (36 mL), added with N-bromosuccinimide (1.28 g, 7.22 mmol), and stirred at 70°C for 4 hours. After the reaction was completed, the reaction solution was concentrated under reduced pressure and purified by thin layer plate to obtain methyl 4,5-bis(bromomethyl)thiazole-2-carboxylate **11c** (360 mg), yield: 27.28%.

MS m/z(ESI):327.9[M+1]

### Step 3

### Methyl

### 2'-oxo-1'-((2-(trimethylsilyl)ethoxy)methyl)-1',2',4,6-tetrahydrospiro[cyclopenta[d]thiazole-5,3'-pyrrolo[2,3-b]pyridine-2-carboxylate

Methyl 4,5-bis(bromomethyl)thiazole-2-carboxylate **11c** (150 mg, 437.27 µmol) and 1-((2-(trimethylsilyl)ethoxy)methyl)-1,3-dihydro-2H-pyrrolo[2,3-b]pyridin-2-one **1a** (109.83 mg, 415.41 µmol) were dissolved in ethanol (7.5 mL), added with cesium carbonate (283.38 mg, 869.25 µmol), and stirred at 25°C for 16 hours. After the reaction was completed, the reaction solution was extracted with water (20 mL × 3) and ethyl acetate (30 mL × 3). The organic phases were combined and concentrated under reduced pressure. The resulting residue was separated and purified by column chromatography (eluent: System A) to obtain methyl 2'-oxo-1'-((2-(trimethylsilyl)ethoxy)methyl)-1',2',4,6-tetrahydrospiro[cyclopenta[d]thiazole-5,3'-pyrrolo[2,3-b]pyridine-2- carboxylate **11d** (50 mg), yield: 20.53%.

MS m/z(ESI):445.9[M+1]

### Step 4

### Methyl

### 2'-oxo-1',2',4,6-tetrahydrospiro[cyclopenta[d]thiazole-5,3'-pyrrolo[2,3-b]pyridine]-2-carboxylate

### Methyl

2'-oxo-1'-((2-(trimethylsilyl)ethoxy)methyl)-1',2',4,6-tetrahydrospiro[cyclopenta[d]thiazole-5,3'-pyrrolo[2,3-b]pyridine-2- carboxylate **11d** (50 mg, 112.21 µmol) and 1 mL of trifluoroacetic acid were dissolved in tetrahydrofuran (1 mL), and stirred at 25°C for 3 hours. After the reaction was completed, the reaction solution was concentrated under reduced pressure, added with tetrahydrofuran (1 mL) and aqueous ammonia (0.2 mL), stirred at 25°C for 0.5 hours, and concentrated under reduced pressure. The resulting residue was purified by thin layer plate to obtain methyl 2'-oxo-1',2',4,6-tetrahydrospiro[cyclopenta[d]thiazole-5,3'-pyrrolo[2,3-b]pyridine]-2-carboxylate **11e** (33 mg), yield: 68.08%.

MS m/z(ESI):316.1[M+1]

### Step 5

### 2'-Oxo-1',2',4,6-tetrahydrospiro[cyclopenta[d]thiazole-5,3'-pyrrolo[2,3-b]pyridine]-2-carbox ylic acid

### Methyl

2'-oxo-1',2',4,6-tetrahydrospiro[cyclopenta[d]thiazole-5,3'-pyrrolo[2,3-b]pyridine]-2-carboxylate **11e** (33 mg, 104.65 µmol) was dissolved in 1 mL of a mixed solution (water: methanol: tetrahydrofuran = 3: 1: 1), added with sodium hydroxide (12.56 mg, 313.94 µmol), and stirred at 40°C for 1 hour. After the reaction was completed, the reaction solution was adjusted to pH5 with 1M dilute hydrochloric acid, and concentrated under reduced pressure to obtain 2'-oxo-1',2',4,6-tetrahydrospiro[cyclopenta[d]thiazole-5,3'-pyrrolo[2,3-b]pyridine]-2-carboxylic acid **11f** (41 mg), yield: 98.59%, which was directly used for the next step without purification.

MS m/z(ESI):288. 1[M+1]

### Step 6

### N-((3S,5S,6R)-6-methyl-2-oxo-1-(2,2,2-trifluoroethyl)-5-(2,3,6-trifluorophenyl)piperidin-3-yl)-2'-oxo-1',2',4,6-tetrahydrospiro[cyclopenta[d]thiazole-5,3'-pyrrolo[2,3-b]pyridine]-2-carboxa mide

2'-Oxo-1',2',4,6-tetrahydrospiro[cyclopenta[d]thiazole-5,3'-pyrrolo[2,3-b]pyridine]-2-carbox ylic acid **11f** (20 mg, 57.85 µmol), (3S,5S,6R)-3-amino-6-methyl-1-(2,2,2-trifluoroethyl)-5-(2,3,6-trifluorophenyl)piperidin-2-one hydrochloride **1p-A** (34.87 mg, 92.56 µmol), 1-hydroxybenzotriazole (15.63 mg, 115.70 µmol) and triethylamine (74.76 mg, 578.48 µmol) were dissolved in N,N-dimethylformamide (1 mL), added with 1-(3-Dimethylaminopropyl)-3-ethylcarbodiimide hydrochloride (20.56 mg, 115.70 µmol), and stirred at 25°C for 18 hours. After the reaction was completed, the reaction solution was concentrated under reduced pressure. The resulting residue was separated by C18 reverse phase column (eluent: system B) to obtain N-((3 S, 5 S, 6R)-6-methyl-2-oxo-1-(2,2,2-trifluoroethyl)-5-(2,3, 6-trifluorophenyl)piperidin-3-yl)-2 '-oxo-1',2',4,6-tetrahydrospiro[cyclopenta[d]thiazole-5,3'-pyrrolo[2,3-b]pyridine]-2-carboxamide **11** (8.47 mg), yield: 19.28%.

MS m/z(ESI):609.8[M+1]

¹H NMR (400 MHz, CDCl₃) δ 11.14 (s, 1H), 9.27-9.24 (m, 1H), 8.07 (dd, *J* = 5.2, 1.6 Hz, 1H), 7.60-7.57 (m, 1H), 7.49-7.40 (m, 1H), 7.16-7.10 (m, 1H), 6.94 (dd, *J =* 7.6, 5.6 Hz, 1H), 4.69-4.52 (m, 2H), 3.95-3.94 (m, 1H), 3.82-3.70 (m, 2H), 3.24-3.05 (m, 5H), 2.15-2.10 (m, 1H), 1.18 (d, *J* = 6.4 Hz, 3H).

### Example 12

### N-((3S,5S,6R)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzo[d]oxazole-5,3'-pyrrolo[2,3-b]pyridine]-2-carboxa mide

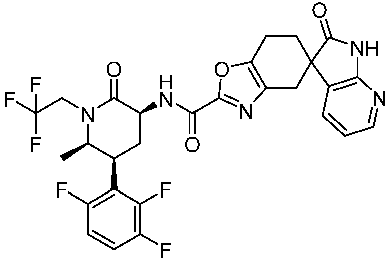

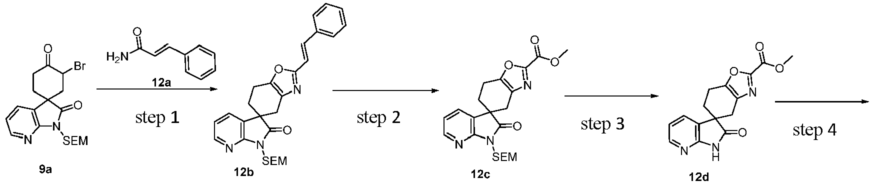

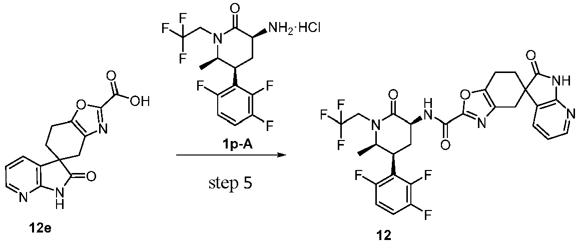

### Step 1

### (E)-2-phenylvinyl-1'-((2-(trimethylsilyl)ethoxy)methyl)-6,7-dihydro-4H-spiro[benzo[d]oxaz ol-5,3'-pyrrolo[2,3-b]pyridin]-2'(1'H)-one

3-Bromo-1'-((2-(trimethylsilyl)ethoxy)methyl)spiro[cyclohexane-1,3'-pyrrolo[2,3-b]pyridine ]-2',4(1'H)-dione **9a** (400 mg, 940.31 µmol) and benzyl acrylamide **12a** (138.39 mg, 940.31 µmol) were dissolved in toluene (4 mL) and stirred at 100°C for 18 hours. After the reaction was completed, the reaction solution was concentrated under reduced pressure. The resulting residue was separated and purified by column chromatography (eluent: System A) to obtain (E)-2-phenylvinyl-1'-((2-(trimethylsilyl)ethoxy)methyl)-6,7-dihydro-4H-spiro[benzo[d]oxazole-5,3'-pyrrolo[2,3-b]pyridine]-2'(1'H)-one **12b** (170 mg), yield: 34.35%.

MS m/z(ESI):474.1[M+1]

### Step 2

### Methyl

### 2'-oxo-1'-((2-(trimethylsilyl)ethoxy)methyl)-1',2',6,7-tetrahydro-4H-spiro[benzo[d]oxazole-5,3'-pyrrolo[2,3-b]pyridine]-2-carboxylate

(E)-2-phenylvinyl-1'-((2-(trimethylsilyl)ethoxy)methyl)-6,7-dihydro-4H-spiro[benzo[d]oxaz ol-5,3'-pyrrolo[2,3-b]pyridine]-2'(1'H)-one **12b** (150 mg, 316.70 µmol) was dissolved in a mixed solution (tetrahydrofuran:acetone: water = 6: 3: 1), added with N-methylmorpholine N-oxide (278.26 mg, 2.37 mmol) and potassium osmate (492.47 mg, 1.58 mmol), stirred at 30°C for 4 hours, added with potassium carbonate (87.41 mg, 633.40 µmol) and iodomethane (224.76 mg, 1.58 mmol), and continuously stirred for 18 hours. After the reaction was completed, the reaction solution was concentrated under reduced pressure. The resulting residue was separated and purified by column chromatography (eluent: system C) to obtain methyl 2'-oxo-1'-((2-(trimethylsilyl)ethoxy)methyl)-1',2',6,7-tetrahydro-4H-spiro[benzo[d]oxazole-5,3'-pyrrolo[2,3-b]pyridine]-2-carboxylate **12c** (42 mg), yield: 29.24%.

MS m/z(ESI):430.3[M+1]

### Step 3

### Methyl 2'-oxo-1',2',6,7-tetrahydro-4H-spiro[benzo[d]oxazole-5,3'-pyrrolo[2,3-b]pyridine]-2-carboxylate

### Methyl

2'-oxo-1'-((2-(trimethylsilyl)ethoxy)methyl)-1',2',6,7-tetrahydro-4H-spiro[benzo[d]oxazole-5,3'-pyrrolo[2,3-b]pyridine]-2-carboxylate **12c** (42 mg, 97.78 µmol) was added to trifluoroacetic acid (2 mL), stirred at 25°C for 2 hours. After the reaction was completed, the reaction solution was concentrated under reduced pressure. The residue was dissolved in tetrahydrofuran (2 mL), then added with ammonia water (0.3 mL), stirred at 25°C for 0.5 hours, and concentrated under reduced pressure. The resulting residue was purified by TLC plate to obtain methyl 2'-oxo-1',2',6,7-tetrahydro-4H-spiro[benzo[d]oxazole-5,3'-pyrrolo[2,3-b]pyridine]-2- carboxylate **12d** (20 mg), yield: 61.51%.

MS m/z(ESI):299.9[M+1]

### Step 4

### 2'-Oxo-1',2',6,7-tetrahydro-4H-spiro[benzo[d]oxazole-5,3'-pyrrolo[2,3-b]pyridine]-2-carbox ylic acid

Methyl 2'-oxo-1',2',6,7-tetrahydro-4H-spiro[benzo[d]oxazole-5,3'-pyrrolo[2,3-b]pyridine]-2-carboxylate **12d** (20 mg, 66.83 µmol) was dissolved in 1.6 mL of a mixed solution (water: methanol: tetrahydrofuran = 1: 2: 4), added with sodium hydroxide (8.02 mg, 66.83 µmol), and stirred at 25°C for 2 hours. After the reaction was completed, the reaction solution was adjusted to pH5 with 1M dilute hydrochloric acid, and concentrated under reduced pressure to obtain 2'-oxo-1',2',6,7-tetrahydro-4H-spiro[benzo[d]oxazole-5,3'-pyrrolo[2,3-b]pyridine]-2-carboxylic acid **12e** (25 mg), which was directly used for the next step without purification.

MS m/z(ESI):286.0[M+1]

### Step 5

### N-((3S,5S,6R)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzo[d]oxazole-5,3'-pyrrolo[2,3-b]pyridine]-2-carboxa mide

2'-Oxo-1',2',6,7-tetrahydro-4H-spiro[benzo[d]oxazole-5,3'-pyrrolo[2,3-b]pyridine]-2-carbox ylic acid **12e** (20 mg, 65.10 µmol), (3S,5S,6R)-3-amino-6-methyl-1-(2,2,2-trifluoroethyl)-5-(2,3, 6-trifluorophenyl)piperidin-2-one hydrochloride **1p-A** (22.15 mg, 65.10 µmol), 1-hydroxybenzotriazole (17.59 mg, 130.19 µmol) and N,N-diisopropylethylamine (42.07 mg, 325.48 µmol) were dissolved in N,N-dimethylformamide (0.5 mL), added with 1-(3-dimethylaminopropyl)-3-ethylcarbodiimide hydrochloride (24.96 mg, 130.19 µmol),and stirred at 45°C for 3 hours. After the reaction was completed, the reaction solution was concentrated under reduced pressure. The resulting residue was separated by C18 reverse phase column (eluent: system B) to obtain N-((3S,5S,6R)-6-methyl-2-oxo-1-(2,2,2-trifluoroethyl)-5-(2,3,6-trifluorophenyl)piperidin-3-yl)-2'-oxo-1',2',6,7-tetrahydro-4H-spiro[benzo[d]oxazole-5, 3'-pyrrolo[2,3-b]pyridine]-2-carboxamide **12** (2.29 mg), yield: 4.86%.

MS m/z(ESI):608.2[M+1]

¹H NMR (400 MHz, CDCl₃) δ 8.16 (d, *J =* 4.8 Hz, 1H), 7.13-7.07 (m, 2H), 6.92-6.83 (m, 2H), 4.93-4.88 (m, 1H), 4.63-4.51 (m, 1H), 4.00-3.91 (m, 2H), 3.36-3.27 (m, 2H), 2.95-2.71 (m, 5H), 2.35-2.27 (m, 1H), 1.93-1.88 (m, 1H), 1.25-1.22 (m, 3H).

### Example 13

### N-((3S,5S,6R)-6-methyl-2-oxo-1-(2,2,2-trifluoroethyl)-5-(2,3,6-trifluorophenyl)piperidin-3-yl)-2'-oxo-1,1',2',4,6,7-hexahydrospiro[indole-5,3'-pyrrolo[2,3-b]pyridine]-2-carboxamide

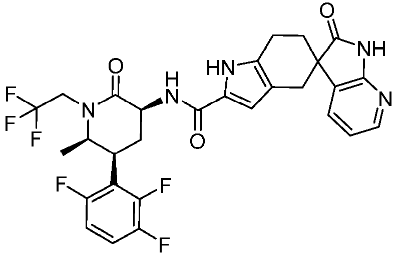

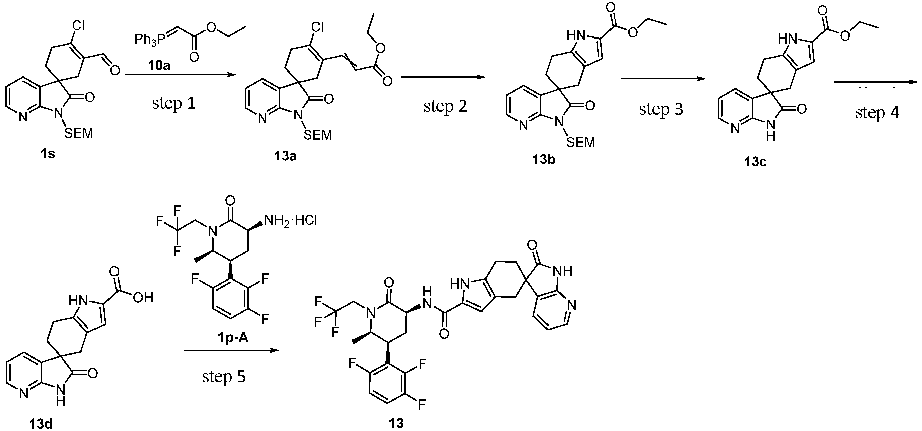

### Step 1

### Ethyl

### 3-(4-chloro-2'-oxo-1'-((2-(trimethylsilyl)ethoxy)methyl)-1',2'-dihydrospiro[cyclohexane-1,3'-pyr rolo[2,3-b)acrylate

4-Chloro-2'-oxo-1'-((2-(trimethylsilyl)ethoxy)methyl)-1',2'-dihydrospiro[cyclohexane-1,3'-p yrrolo[2,3-b]pyridine]-3-ene-3-carbaldehyde **1s** (374 mg, 951.77 µmol) and ethyl 2-(triphenyl-λ⁵-phosphite)acetate **10a** (397.89 mg, 1.14 mmol) were dissolved in dichloromethane (6.67 mL), stirred at 25°C for 16 hours. After the reaction was completed, the reaction solution was concentrated under reduced pressure. The resulting residue was separated and purified by column chromatography (eluent: System A) to obtain ethyl 3-(4-chloro-2'-oxo-1'-((2-(trimethylsilyl)ethoxy)methyl)-1',2'-dihydrospiro[cyclohexane-1,3'-pyr rolo[2,3-b]]pyridine]-3-en-3-yl)acrylate **13a** (310 mg), yield: 69.64%.

MS m/z(ESI):463.2[M+1]

### Step 2

### Ethyl

### 2'-oxo-1'-((2-(trimethylsilyl)ethoxy)methyl)-1,1',2',4,6,7-hexahydrospiro[indole-5,3'-pyrrolo[2,3-b][pyridine]-2-carboxylate

### Ethyl

3-(4-chloro-2'-oxo-1'-((2-(trimethylsilyl)ethoxy)methyl)-1',2'-dihydrospiro[cyclohexane-1,3'-pyr rolo[2,3-b]]pyridin]-3-en-3-yl)acrylate **13a** (260 mg, 561.51 µmol) was dissolved in dimethyl sulfoxide (7.8 mL), added with sodium azide (65.71 mg, 1.01 mmol), stirred at 70°C for 18 hours under nitrogen protection, added with 40 mL of ethyl acetate and 40 mL of water, and extracted with ethyl acetate (40 mL×3). The organic phases were combined and concentrated under reduced pressure, and the resulting residue was separated and purified by column chromatography (eluent: System A) to obtain ethyl 2'-oxo-1'-((2-(trimethylsilyl)ethoxy)methyl)-1,1',2',4,6,7-hexahydrospiro[indole-5,3'-pyrrolo[2,3-b][pyridine]-2-carboxylate **13b** (120 mg), yield: 35.49%.

MS m/z(ESI):442.2[M+1]

### Step 3

### Ethyl 2'-oxo-1,1',2',4,6,7-hexahydrospiro[indole-5,3'-pyrrolo[2,3-b]pyridine]-2-carboxylate

### Ethyl

2'-oxo-1'-((2-(trimethylsilyl)ethoxy)methyl)-1,1',2',4,6,7-hexahydrospiro[indole-5,3'-pyrrolo[2,3-b][pyridine]-2-carboxylate **13b** (120 mg, 271.74 µmol) was dissolved in trifluoroacetic acid (2 mL), stirred at 25°C for 3 hours. After the reaction was completed, the reaction solution was concentrated under reduced pressure, added with tetrahydrofuran (2 mL) and aqueous ammonia (0.3 mL) respectively, and continuously stirred at 25°C for 0.5 hours, and concentrated under reduced pressure, and the resulting residue was separated and purified by column chromatography (eluent: System A) to obtain ethyl 2'-oxo-1,1',2',4,6,7-hexahydrospiro[indole-5,3'-pyrrolo[2,3-b]pyridine]-2-carboxylate **13c** (80 mg), yield: 86.99%.

MS m/z(ESI):312.1[M+1]

### Step 4

### 2'-Oxo-1,1',2',4,6,7-hexahydrospiro[indole-5,3'-pyrrolo[2,3-b]pyridine]-2-carboxylic acid

Ethyl 2'-oxo-1,1',2',4,6,7-hexahydrospiro[indole-5,3'-pyrrolo[2,3-b]pyridine]-2-carboxylate **13c** (80 mg, 256.96 µmol) was dissolved in 2.5 mL of a mixed solution (water: methanol: tetrahydrofuran = 1: 3: 1), added with sodium hydroxide (30.83 mg, 770.88 µmol),and stirred at 40°C for 2 hours. After the reaction was completed, the reaction solution was adjusted to pH5 with 1M dilute hydrochloric acid, and concentrated under reduced pressure to obtain 2'-oxo-1,1',2',4,6,7-hexahydrospiro[indole-5,3'-pyrrolo[2,3-b]pyridine]-2-carboxylic acid **13d** (80 mg), which was directly used for the next step without purification.

MS m/z(ESI):284.1[M+1]

### Step 5

### N-((3S,5S,6R)-6-methyl-2-oxo-1-(2,2,2-trifluoroethyl)-5-(2,3,6-trifluorophenyl)piperidin-3-yl)-2'-oxo-1,1',2',4,6,7-hexahydrospiro[indole-5,3'-pyrrolo[2,3-b]pyridine]-2-carboxamide

2'-Oxo-1,1',2',4,6,7-hexahydrospiro[indole-5,3'-pyrrolo[2,3-b]pyridine]-2-carboxylic acid **13d** (30 mg, 87.79 µmol), (3S,5S,6R)-3-amino-6-methyl-1-(2,2,2-trifluoroethyl)-5-(2,3,6-trifluorophenyl)piperidin-2-one hydrochloride **1p-A** (59.53 mg, 158.02 µmol), 1-hydroxybenzotriazole (23.72 mg, 178.58 µmol) and N,N-diisopropylethylamine (113.46 mg, 877.90 µmol) were dissolved in N,N-dimethylformamide (1 mL), added with 1-(3-dimethylaminopropyl)-3-ethylcarbodiimide hydrochloride (31.20 mg, 175.58 µmol), and stirred at 25°C for 16 hours. After the reaction was completed, the reaction solution was concentrated under reduced pressure. The resulting residue was separated by C18 reverse phase column (eluent: system B) to obtain N-((3S,5S,6R)-6-methyl-2-oxo-1-(2,2,2-trifluoroethyl)-5-(2,3,6-trifluorophenyl)piperidin-3-yl)-2 '-oxo-1,1',2',4,6,7-hexahydrospiro[indole-5,3'-pyrrolo[2,3-b]pyridine]-2-carboxamide **13** (4.05 mg), yield: 5.37%.

MS m/z(ESI):605.8[M+1]

¹H-NMR (400 MHz, DMSO-*d₆*) δ 11.30 (s, 1H), 11.08 (s, 1H), 8.38 (t, *J =* 8.0 Hz, 1H), 8.05 (dd, *J* = 5.2, 1.6 Hz, 1H), 7.52-7.45 (m, 1H), 7.19-7.14 (m, 1H), 6.96-6.93 (m, 1H), 6.87-6.83 (m, 1H), 6.56-6.55 (m, 1H), 4.71-4.60 (m, 1H), 4.42-4.32 (m, 1H), 3.95-3.92 (m, 1H), 3.83-3.76 (m, 2H), 3.11-3.02 (m, 1H), 2.91-2.81 (m, 2H), 2.63-2.58 (m, 1H), 2.46-2.42 (m, 1H), 2.15-2.03 (m, 2H), 1.76-7.71 (m, 1H), 1.24 (d, *J =* 6.0 Hz, 3H).

### Example 13A

### (S)-N-((3S,5S,6R)-6-methyl-2-oxo-1-(2,2,2-trifluoroethyl)-5-(2,3,6-trifluorophenyl)piperidi n-3-yl)-2'-oxo-1,1',2',4,6,7-hexahydrospiro[indole-5,3'-pyrrolo[2,3-b]pyridine]-2-carboxamide 13A

### Example 13B

### (R)-N-((3S,5S,6R)-6-methyl-2-oxo-1-(2,2,2-trifluoroethyl)-5-(2,3,6-trifluorophenyl)piperidi n-3-yl)-2'-oxo-1,1',2',4,6,7-hexahydrospiro[indole-5,3'-pyrrolo[2,3-b]pyridine]-2-carboxamide 13B

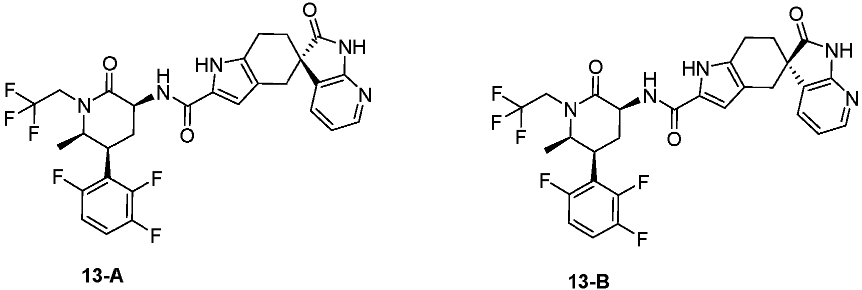

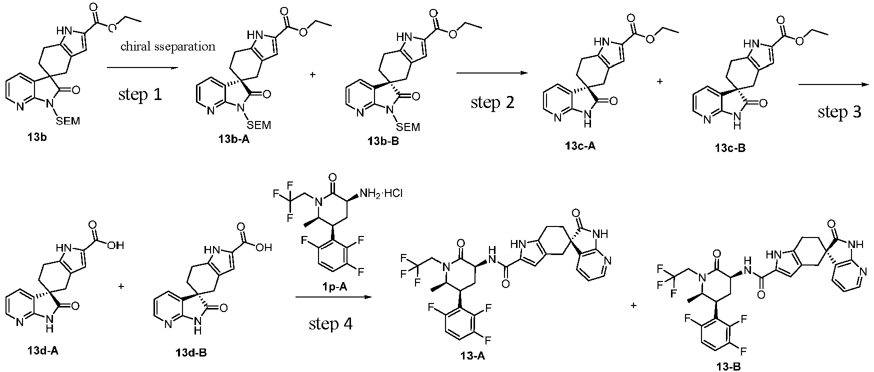

### Step 1

### Ethyl

### (S)-2'-oxo-1'-((2-(trimethylsilyl)ethoxy)methyl)-1,1',2',4,6,7-hexahydrospiro[indole-5,3'-pyrrolo[ 2,3-b]pyridine]-2-carboxylate 13b-A

### Ethyl

### (R)-2'-oxo-1'-((2-(trimethylsilyl)ethoxy)methyl)-1,1',2',4,6,7-hexahydrospiro[indole-5,3'-pyrrolo [2,3-b]pyridine]-2-carboxylate 13b-B

After purification of ethyl 2'-oxo-1'-((2-(trimethylsilyl)ethoxy)methyl)-1,1',2',4,6,7-hexahydrospiro[indole-5,3'-pyrrolo[2,3-b]pyridine]-2-carboxylate **13b** (796 mg, 1.80 mmol) by SFC chiral separation (column model: Waters SFC-150, Dnicel IG, 20×250 mm, 10 µm; mobile phase: A for CO2 and B for Ethanol; detection wavelength: 214 nm; column temperature: 40°C), a compound with single configuration (shorter retention time) and a compound with single configuration (longer retention time) were obtained.

### Compound with single configuration (short retention time):

322 mg, yield: 40.5%, retention time: 1.964 min, chiral purity: 100% ee.

MS m/z(ESI):442.2[M+1]

### Compound with single configuration (longer retention time):

317 mg, yield: 39.8%, retention time: 2.242 minutes, chiral purity: 99% ee.

MS m/z(ESI):442.2[M+1]

### Step 2

### Ethyl

### (S)-2'-oxo-1,1',2',4,6,7-hexahydrospiro[indole-5,3'-pyrrolo[2,3-b]pyridine]-2-carboxylate 13c-A

### Ethyl

### (R)-2'-Oxo-1,1',2',4,6,7-hexahydrospiro[indole-5,3'-pyrrolo[2,3-b]pyridine]-2-carboxylate 13c-B

The chirally resolved ethyl (S)-2'-oxo-1'-((2-(trimethylsilyl)ethoxy)methyl)-1,1',2',4,6,7-hexahydrospiro[indole-5,3'-pyrrolo[ 2,3-b]pyridine]-2-carboxylate **13b-A** (316.00 mg, 715.59 µmol) or ethyl (R)-2'-oxo-1'-((2-(trimethylsilyl)ethoxy)methyl)-1,1',2',4,6,7-hexahydrospiro[indole-5,3'-pyrrolo [2,3-b]pyridine]-2-carboxylate **13b-B** (311.00 mg, 704.27 µmol) was dissolved in trifluoroacetic acid (1.5 mL) and stirred at 25°C for 3 hours. After the reaction was completed, the reaction solution was concentrated under reduced pressure, added with 1.5 mL of tetrahydrofuran and 0.3 mL of aqueous ammonia, and continuously stirred at 25°C for 0.5 hour, and concentrated under reduced pressure. The resulting residue was purified by column chromatography (eluent: System A) to obtain ethyl (S)-2'-oxo-1,1',2',4,6,7-hexahydrospiro[indole-5,3'-pyrrolo[2,3-b]pyridine]-2-carboxylate **13c-A** (190 mg), yield: 85.1%, and ethyl (R)-2'-Oxo-1,1',2',4,6,7-hexahydrospiro[indole-5,3'-pyrrolo[2,3-b]pyridine]-2-carboxylate **13c-B** (195 mg), yield: 88.8%.

MS m/z(ESI):312.1[M+1]

MS m/z(ESI):312.1[M+1]

### Step 3

### (S)-2'-oxo-1,1',2',4,6,7-hexahydrospiro[indole-5,3'-pyrrolo[2,3-b]pyridine]-2-carboxylic acid 13d-A

### (R)-2'-oxo-1,1',2',4,6,7-hexahydrospiro[indole-5,3'-pyrrolo[2,3-b]pyridine]-2-carboxylic acid 13d-B

### Ethyl

(S)-2'-oxo-1,1',2',4,6,7-hexahydrospiro[indole-5,3'-pyrrolo[2,3-b]pyridine]-2-carboxylate **13c-A** (190 mg, 610.28 µmol) or ethyl (R)-2'-oxo-1,1',2',4,6,7-hexahydrospiro[indole-5,3'-pyrrolo[2,3-b]pyridine]-2-carboxylate **13c-B** (195 mg, 626.34 µmol) was dissolved in 5.5 mL of a mixed solution (methanol: tetrahydrofuran: water = 1: 3: 1), added with sodium hydroxide (73.23 mg, 1.83 mmol), and stirred at 50°C for 16 hours. After the reaction was completed, the reaction solution was concentrated under reduced pressure, adjusted to pH 5 with 1M diluted hydrochloric acid, and extracted with ethyl acetate (20 mL×5) and methanol (2 mL×5). The organic phases were combined and concentrated under reduced pressure to obtain (S)-2'-oxo-1, 1',2',4,6,7-hexahydrospiro[indole-5,3'-pyrrolo[2,3-b]pyridine]-2-carboxylic acid **13d-A** (157 mg), yield: 84.37%; (R)-2'-oxo-1,1',2',4,6,7-hexahydrospiro[indole-5,3'-pyrrolo[2,3-b]pyridine]-2-carboxylic acid **13d-B** (158 mg), yield: 80.14%, which were directly used for the next step without purification.

MS m/z(ESI):284.1[M+1]

MS m/z(ESI):284.1[M+1]

### Step 4

### (S)-N-((3S,5S,6R)-6-methyl-2-oxo-1-(2,2,2-trifluoroethyl)-5-(2,3,6-trifluorophenyl)piperidi n-3-yl)-2'-oxo-1,1',2',4,6,7-hexahydrospiro[indole-5,3'-pyrrolo[2,3-b]pyridine]-2-carboxamide 13A

### (R)-N-((3S,5S,6R)-6-methyl-2-oxo-1-(2,2,2-trifluoroethyl)-5-(2,3,6-trifluorophenyl)piperidi n-3-yl)-2'-oxo-1,1',2',4,6,7-hexahydrospiro[indole-5,3'-pyrrolo[2,3-b]pyridine]-2-carboxamide 13B

(S)-2'-oxo-1,1',2',4,6,7-hexahydrospiro[indole-5,3'-pyrrolo[2,3-b]pyridine]-2-carboxylic acid **13d-A** (20 mg, 70.60 µmol) or (R)-2'-oxo-1,1',2',4,6,7-hexahydrospiro[indole-5,3'-pyrrolo[2,3-b]pyridine]-2-carboxylic acid **13d-B** (20 mg, 70.60 µmol), (3S,5S,6R)-3-amino-6-methyl-1-(2,2,2-trifluoroethyl)-5-(2,3,6-trifluorophenyl)piperidin-2-one hydrochloride **1p-A** (23.94 mg, 63.54 µmol), 1-hydroxybenzotriazole (19.08 mg, 141.20 µmol) and N,N-diisopropylethylamine (91.25 mg, 706.01 µmol) were dissolved in N,N-dimethylformamide (1 mL), added with 1-(3-dimethylaminopropyl)-3-ethylcarbodiimide hydrochloride (25.09mg, 141.20µmol), and stirred at 25°C for 16 hours. After the reaction was completed, the reaction solution was concentrated under reduced pressure. The resulting residue was separated by C18 reverse phase column (eluent: System B) to obtain (S)-N-((3S,5S,6R)-6-methyl-2-oxo-1-(2,2,2-trifluoroethyl)-5-(2,3,6-trifluorophenyl)piperidin-3-yl)-2'-oxo-1,1',2',4,6,7-hexahydrospiro[indole-5,3'-pyrrolo[2,3 -b]pyridine]-2-carboxamide **13A** (35.88 mg), yield: 69.22%; or (R)-N-((3S,5S,6R)-6-methyl-2-oxo-1-(2,2,2-trifluoroethyl)-5-(2,3,6-trifluorophenyl)piperidin-3-yl)-2'-oxo-1,1',2',4,6,7-hexahydrospiro[indole-5,3'-pyrrolo[2,3-b]pyridine]-2-carboxamide **13B** (34.65 mg), yield: 67.52%.

MS m/z(ESI):606.2[M+1]

MS m/z(ESI):606.5[M+1]

¹H-NMR (400 MHz, DMSO-*d₆*) δ 11.28 (s, 1H), 11.08 (s, 1H), 8.36 (d, *J* = 8.0 Hz, 1H), 8.03(dd, *J* = 5.2, 1.6 Hz, 1H), 7.49-7.45 (m, 1H), 7.19-7.13 (m, 1H), 6.93 (dd, *J =* 7.2, 1.6 Hz, 1H), 6.84 (dd, *J =* 7.2, 5.2 Hz, 1H), 6.54 (d, *J =* 2.4 Hz, 1H), 4.68-4.58 (m, 1H), 4.39-4.32 (m, 1H), 3.94-3.90 (m, 1H), 3.80-3.72 (m, 2H), 3.08-2.99 (m, 1H), 2.89-2.79 (m, 2H), 2.66-2.57 (m, 1H), 2.42 (d, *J =* 14.8 Hz, 1H), 2.13-2.02 (m, 2H), 1.74-1.69 (m, 1H), 1.22 (d, *J =* 6.4 Hz, 3H).

¹HNMR (400 MHz, DMSO-*d₆*) δ 11.23 (s, 1H), 11.02 (s, 1H), 8.32 (d, *J =* 8.0 Hz, 1H), 7.97 (dd, *J =* 5.2, 1.6 Hz, 1H), 7.44-7.36 (m, 1H), 7.13-7.06 (m, 1H), 6.85 (dd, *J =* 7.2, 1.6 Hz, 1H), 6.77 (dd, *J =* 7.2, 5.2 Hz, 1H), 6.47 (d, *J =* 2.4 Hz, 1H), 4.62-4.51 (m, 1H), 4.29-4.22 (m, 1H), 3.87-3.82 (m, 1H), 3.77-3.62 (m, 2H), 3.03-2.93 (m, 1H), 2.83-2.73 (m, 2H), 2.59-2.50 (m, 1H), 2.35 (d, *J* = 15.6 Hz, 1H), 2.06-1.94 (m, 2H), 1.67-1.63 (m, 1H), 1.16 (d, *J =* 6.4 Hz, 3H).

### Biological evaluation

### Test Example 1: determination of the inhibitory effect of the compounds of the present application on the CGRP signaling pathway in CHO-K1 cells expressing human CGRP receptors

The inhibitory effect of the CGRP signaling pathway in vitro is evaluated by measuring the cAMP level. The principle is that after CGRP binds to the CGRP receptor, the CGRP signaling pathway is activated, inducing an increase in the cAMP level. Therefore, a decrease in the cAMP level indicates that the CGRP signaling pathway is inhibited. The specific experimental method is as follows:

CHO-K1 cells expressing human CGRP receptor (CGRPR/CHO-K1, PerkinElmer, ES-420-C) were cultured in F12K+10% FBS+400 µg/mL G418+10 µg/mL Blasticidin medium, and the cells were collected in the logarithmic growth phase. According to the instructions of the kit, the cells were resuspended in Stimulation Buffer comprising 0.5 mM IBMX, and 5 µL of the cell suspension was added to each well of a 96-well microplate (Cisbio, 66PL96025) at a cell density of 15,000 cells/well. 2.5 µL of gradient diluted compound solution was added to each well. After incubation at 37°C for 30 minutes, 2.5 µL of 40 ng/mL human α-CGRP (Bachem, H-1470.0500) diluted in Stimulation Buffer comprising 0.5 mM IBMX was added to each well to a final concentration of 10 ng/mL. After incubation at 37°C for 30 minutes, 5 µL of Anti-cAMP-Cryptate solution and 5 µL of cAMP-d2 solution were added to each well. After incubation at room temperature for 60 minutes, the HTRF signal was read using a microplate reader (Molecular Devices). Graphpad Prism was used to calculate the IC₅₀ value of the compound's inhibitory effect on the increase in cAMP levels based on the compound concentration and HTRF signal.

The biological activity of the compounds of the present application was determined by the above test, and the IC₅₀ values measured at [α-CGRP]=10 ng/mL are shown in Table 1 below.

**Table 1 IC₅₀ of the inhibitory effect of the compounds of the present application on the CGRP signaling pathway in CHO-K1 cells expressing human CGRP receptors**

| Compound No. | IC₅₀ (µM) |
|---|---|
| Example 1 | 0.1064 |
| Example 1A | 0.0145 |
| Example 2 | 0.2498 |
| Example 3 | 0.0647 |
| Example 3A | 0.0029 |
| Example 4 | 0.1987 |
| Compound with the best activity in Examples 5A and 5B | 0.0137 |
| Compound with the best activity in Examples 6A, 6B, 6C and 6D | 0.3739 |
| Compound with the best activity in Examples 7A and 7B | 0.0449 |
| Compound with the best activity in Examples 8A, 8B, 8C and 8D | 0.4963 |
| Atogepant | 0.0015 |

Conclusion: the compounds of the present application have a significant inhibitory effect on the CGRP signaling pathway in CHO-K1 cells expressing human CGRP receptors. The positive control compound Atogepant was prepared according to the scheme described in patent application WO2012064910, and its structure is as follows:

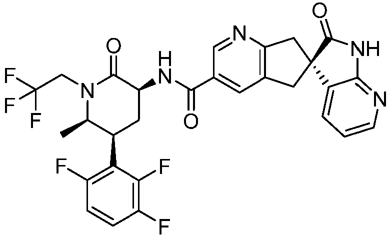

### Test Example 2: determination of the inhibitory effect of the compounds of the present application on the CGRP signaling pathway in SK-N-MC cells

The inhibitory effect of the CGRP signaling pathway in vitro is evaluated by measuring the cAMP level. The principle is that after CGRP binds to the CGRP receptor, the CGRP signaling pathway is activated, inducing an increase in the cAMP level. Therefore, a decrease in the cAMP level indicates that the CGRP signaling pathway is inhibited. The specific experimental method is as follows:
cAMP was determined using the CAMP-GS DYNAMIC KIT detection kit (Cisbio, 62AM4PEB).

SK-N-MC (ATCC, HTB-10) cells endogenously expressing CGRP receptor were cultured in EMEM+10% FBS medium and the cells were collected during the logarithmic growth phase. According to the instructions of the kit, the cells were resuspended in Stimulation Buffer comprising 0.5 mM IBMX, and 5 µL of the cell suspension was added to each well of a 96-well microplate (Cisbio, 66PL96025) at a cell density of 15,000 cells/well. 2.5 µL of gradient diluted compound solution was added to each well. After incubation at 37°C for 30 minutes, 2.5 µL of 40 ng/mL human α-CGRP (Bachem, H-1470.0500) diluted in Stimulation Buffer comprising 0.5 mM IBMX was added to each well to a final concentration of 10 ng/mL. After incubation at 37°C for 30 minutes, 5 µL of Anti-cAMP-Cryptate solution and 5 µL of cAMP-d2 solution were added to each well. After incubation at room temperature for 60 minutes, the HTRF signal was read using a microplate reader (Molecular Devices). Graphpad Prism was used to calculate the IC₅₀ value of the compound's inhibitory effect on the increase in cAMP levels based on the compound concentration and HTRF signal.

The biological activity of the compounds of the present application was determined by the above experiment, and the IC₅₀ value measured at [α-CGRP]=10 ng/mL is shown in Table 2 below.

**Table 2 IC₅₀ of the inhibitory effect of the compounds of the present application on the CGRP signaling pathway in SK-N-MC cells**

| Compound No. | IC₅₀ (nM) |
|---|---|
| Example 1A | 0.18 |
| Example 3A | 0.68 |
| Compound with the best activity in Examples 5A and 5B | 1.09 |
| Compound with the best activity in Examples 6A, 6B, 6C and 6D | 40.45 |
| Compound with the best activity in Examples 7A and 7B | 3.81 |
| Compound with the best activity in Examples 8A, 8B, 8C and 8D | 26.96 |
| Example 9 | >100 |
| Example 10 | 6.80 |
| Example 11 | 11.01 |
| Example 12 | 53.60 |
| Example 13 | 0.89 |
| Compound with the best activity in Examples 13A and 13B | 0.70 |
| Atogepant | 0.12 |

Conclusion: the compounds of the present application have a significant inhibitory effect on the CGRP signaling pathway in SK-N-MC cells.

### Test Example 3: determination of affinity of the compounds of the present application for human CGRP receptor

The cell membrane homogenate of CHO cells expressing human CGRP receptor (comprising 16 µg of protein) was incubated with 0.03 nM [¹²⁵I]h-CGRPα and gradiently diluted test samples in buffer (50 mM Hepes-NaOH (pH 7.4), 10 mM MgCl₂, 4 mM KCl, 10 mM NaCl₂, 1 mM EDTA, 1 µM phosphorylation amide, 0.3% BSA and 0.04% bacitracin) at 22°C for 90 minutes. A control group without the test sample was set up to obtain the highest binding signal of the experiment, and the non-specific binding of the experiment was determined by adding 1 µM non-isotope-labeled h-CGRPα to the control group. After the incubation, a glass fiber filter (GF/B, Packard) soaked with 0.3% PEI was placed in a 96-well cell collector (Unfilter, Packard), and the sample was quickly filtered under vacuum and rinsed several times with precooled buffer comprising 50 mM Tris-HCl and 150 mM NaCl. After the filter was dried, scintillation fluid (Microscint 0, Packard) was added and the radioactive signal value was measured using a scintillation counter (Topcount, Packard). The results are expressed as the percentage of inhibition of specific binding of the radioligand. Non-isotope labeled h-CGRPα was used as a standard sample as a control. The test samples and standard samples were tested at multiple concentrations to obtain a competition curve with [¹²⁵I]h-CGRPα, thereby calculating the IC50 of each sample. The inhibition constant Ki was further calculated using the Cheng Prusoff equation: Ki = IC50/(1+L/K_{D}), wherein L represents the concentration of [¹²⁵I]h-CGRPα in the system (0.03 nM), and K_{D} represents the dissociation constant between [¹²⁵I]h-CGRPα and the human CGRP receptor in the system (0.06 nM).

The affinity of the compounds of the present application was determined by the above experiment, and the measured Kᵢ values are shown in Table 3 below.

**Table 3 Kᵢ of the affinity determination of the compounds of the present application to human CGRP receptor**

| Compound No. | Ki (pM) |
|---|---|
| Example 1A | 17 |
| Example 3A | 19 |
| Atogepant | 9.8 |
| Ubrogepant | 27 |

Conclusion: the compounds of the present application have strong affinity to human CGRP receptor. The positive control compound Ubrogepant was prepared according to the scheme described in patent application WO2012064910, and its structure is as follows:

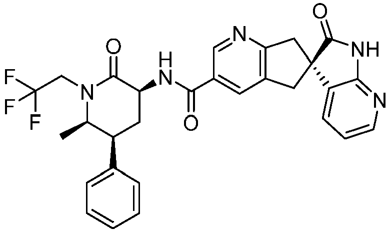

### Test Example 4: Pharmacokinetics study of the compound of the present application in SD rats by oral gavage

### 1. Purpose of the experiment

SD rats were used as test animals, and the LC/MS/MS method was used to determine the drug concentration in plasma at different times after gavage and intravenous injection of the compound of the application, so as to study the pharmacokinetic characteristics of the compound of the application in rats.

### 2. Experimental protocol

### 2.1 Experimental drugs and animals

Positive control compound Atogepant, Example 1A and Example 3A of the present application;
Eighteen healthy adult Sprague Dawley (SD) male rats were purchased from Joinn (Suzhou) New Drug Research Center Co., Ltd.

### 2.2 Drug preparation and administration

### Oral gavage group:

An appropriate amount of the compound to be tested was weighed, added with 100% polyethylene glycol 400, and vortexed to dissolve the compound. After the preparation was completed, it would be a colorless and transparent solution with a concentration of 2 mg/mL.

### Intravenous injection group:

An appropriate amount of the compound to be tested was weighed, added 90% polyethylene glycol 400 and 10% ethanol, and vortexed to dissolve the compound. After the preparation was completed, it would be a colorless and transparent solution with a concentration of 1 mg/mL.

Eighteen healthy adult SD male rats were fasted overnight and fed 4 hours after drug administration.

### 2.3 Sample collection

About 0.2 mL of blood was collected from the jugular vein before administration and 0.083 hours, 0.25 hours, 0.5 hours, 1 hour, 2 hours, 4 hours, 6 hours, 8 hours, 10 hours and 24 hours after administration, and anticoagulated with EDTA-K₂. After blood samples were collected, they were placed in labeled ice-water bath centrifuge tubes and rapidly centrifuged to separate plasma. The centrifugation conditions were: 4000 rpm, 10 minutes, 4°C. The collected plasma was stored at below -40°C for testing. The content of the tested compounds in the plasma of SD rats after gavage and intravenous administration of different compounds was determined by LC-MS/MS.

### 3. Pharmacokinetic parameter results

The pharmacokinetic parameters of the example compounds of the present application and the positive control compound Atogepant are shown in Table 4 below.

**Table 4 Pharmacokinetic parameters of the compounds of the present application and the positive control compound Atogepant**

| Compound No. | Pharmacokinetic experiments (PO 10 mg/kg) | | | |
|---|---|---|---|---|
| | Plasma concentration Cmax (ng/mL) | AUC (ng·h/mL) | Half-life period [994] T1/ 2 (h) | Bioavailability F (%) |
| Atogepant | 560±233 | 2443±1402 | 1.60±0.3 | 12.5±7.3 |
| Example 1A | 3847±1458 | 23245±7946 | 2.93±0.6 | 38.6±13.3 |
| Example 3A | 10500±3728 | 42523±8521 | 1.62±0.2 | 90.5±19.1 |

Conclusion: compared with Atogepant, Examples 1A and 3A of the present application have good pharmacokinetic absorption in SD rats, prolonged half-life, significantly improved plasma concentration, area under the curve, and bioavailability, and have good pharmacokinetic properties.

### Test Example 5: Pharmacokinetics study of the compound of the present application in Cynomolgus monkeys by oral gavage

### 1. Purpose of the experiment

The compounds of the present application were administered by gavage to cynomolgus monkeys as test animals, and the drug concentrations in plasma at different times were determined by LC/MS/MS to study the pharmacokinetic characteristics of the compounds of the present application in rats.

### 2. Experimental protocol

### 2.1 Experimental drugs and animals

Positive control compound Atogepant, Example 1A and Example 3A of the present application;
Six healthy adult male Cynomolgus monkeys, Kangshun Biotechnology Co., Ltd.

### 2.2 Drug preparation and administration

### Oral gavage group:

An appropriate amount of the compound to be tested was weighed, added with 100% polyethylene glycol 400, and vortexed to dissolve the compound. After the preparation was completed, it would be a colorless and transparent solution with a concentration of 1 mg/mL.

Six healthy adult male cynomolgus monkeys were fasted overnight and fed 4 hours after drug administration.

### 2.3 Sample collection

About 0.2 mL of blood was collected from the jugular vein before administration and 0.25 hours, 0.5 hours, 1 hour, 2 hours, 4 hours, 6 hours, 8 hours, 10 hours and 24 hours after administration, and anticoagulated with EDTA-K₂. After blood samples were collected, they were placed in labeled ice-water bath centrifuge tubes and rapidly centrifuged to separate plasma. The centrifugation conditions were: 4000 rpm, 10 minutes, 4°C. The collected plasma was stored at below -40°C for testing. The content of the test compound in the plasma of Cynomolgus monkeys after gavage of different compounds was determined by LC-MS/MS.

### 3. Pharmacokinetic parameter results

The pharmacokinetic parameters of the Example compounds of the present application and the positive control compound Atogepant are shown in Table 5 below.

**Table 5 Pharmacokinetic parameters of the compounds of the present application and the positive control compound Atogepant**

| Compound No. | Pharmacokinetic experiments on Cynomolgus monkeys (PO 2 mg/kg) | | |
|---|---|---|---|
| | Plasma concentration Cmax (ng/mL) | AUC (ng·h/mL) | Half-life period T1/2 (h) |
| Atogepant | 108±18 | 396±42 | 1.50±0.1 |
| Example 1A | 294±166 | 1840±1187 | 3.77±0.2 |
| Example 3A | 778±144 | 3588±742 | 2.96±0.3 |

Conclusion: Compared with Atogepant, Examples 1A and 3A of the present application have good pharmacokinetic absorption in Cynomolgus monkeys, prolonged half-life, significantly improved plasma concentration and area under the curve, and have good pharmacokinetic properties.

### Test Example 6: In vivo efficacy test

### 1. Purpose of the experiment

In the capsaicin-induced Cynomolgus monkey blood flow model, the inhibitory effect of the compounds of the present application on cynomolgus monkey blood flow was evaluated.

### 2. Experimental drugs

In Example 1A and Example 3A, 100% polyethylene glycol 400 was used as the vehicle.

### 3. Experimental methods and materials

### 3.1 Experimental animals and housing conditions

Experimental animals: male cynomolgus monkeys, with a body weight range of 3.2 kg to 5.80 kg during the administration period, purchased from Guangxi Xiongsen Primate Experimental Animal Breeding and Development Co., Ltd., production license: SCXK (Gui) 2016-0003; experimental animal quality certificate number: NO.0002942.

Housing conditions: animals were kept in stainless steel cages by a single cage feeding method. The cage specifications are length × width × height = 1m × 1m × 0.8m. Before anesthesia with zoletil, the animals need to be fasted (but not water). The rest of the time, the animals were fed once in the morning and afternoon, about 100g each time for one animal, supplemented with fresh fruits. The temperature of the animal room was set at 18-26°C, the humidity was 40-70%, and the lighting was alternating between light and dark for 12 hours.

### 3.2 Animal grouping

After adaptive feeding, 10 cynomolgus monkeys were randomly selected, and the basal values were recorded using a moorFLPI-2 laser speckle blood flowmeter before capsaicin stimulation. After capsaicin stimulation, blood flow was tested again, and animals with an area under the time-blood flow rate curve (AUC) ≥ 1000 (min·%) and small individual differences were selected to enter this experiment.

### 3.3 Experimental methods:

The blank vehicle control group was given 100% polyethylene glycol 400, and the drug administration group was given high and low doses of Example 1A and Example 3A (dissolved in 100% polyethylene glycol 400), respectively. The specific administration information is shown in Table 6:

**Table 6 Administration information**

| Groups | Dosage | Number | |
|---|---|---|---|
| | Bolus (µg/kg) | Infusion (µg/kg/min) | of animals |
| Blank vehicle control group | - | - | 1 |
| Example 1A | 0.3 | 0.03 | 1 |
| Example 1A | 1 | 0.1 | 1 |
| Example 3A | 0.3 | 0.03 | 1 |
| Example 3A | 1 | 0.1 | 1 |

| | | | |
|---|---|---|---|
| Note: Bolus is a large-dose push injection; Infusion is a small-dose continuous infusion. | | | |

Blood flow detection: all animals were given capsaicin once on the day of administration, and blood flow was detected at 0 min (before capsaicin administration), 5 min, 10 min, 15 min, 20 min and 30 min after capsaicin administration to monitor blood flow changes during this period. After the experiment ended that day, washout was carried out at intervals of 7-10 days, during which the experimental animals were rotated in turn. This type of experiment was repeated three times to obtain the average value of each group of data.

### 3.4 Statistics

All data were graphed and statistically analyzed using Excel and GraphPad Prism 8 software.

The blood flow change rate, AUC (area under the blood flow change rate-time curve) and blood flow inhibition rate were calculated using Excel. Blood flow change rate (%) = (blood flow at each time point - basal blood flow) / basal blood flow × 100%; trapezoidal formula for calculating blood flow change rate - area under the time curve (AUC): AUC = 1/2 (5 min blood flow change rate + 10 min blood flow change rate) × 5min + 1/2 (10 min blood flow change rate + 15 min blood flow change rate) × 5min + 1/2 (15 min blood flow change rate + 20 min blood flow change rate) × 5min + 1/2 (20 min blood flow change rate + 30 min blood flow change rate) × 10min; blood flow inhibition rate (%) = (AUC of blank vehicle control group - AUC of drug group) / AUC of blank vehicle control group × 100%.

### 4. Results

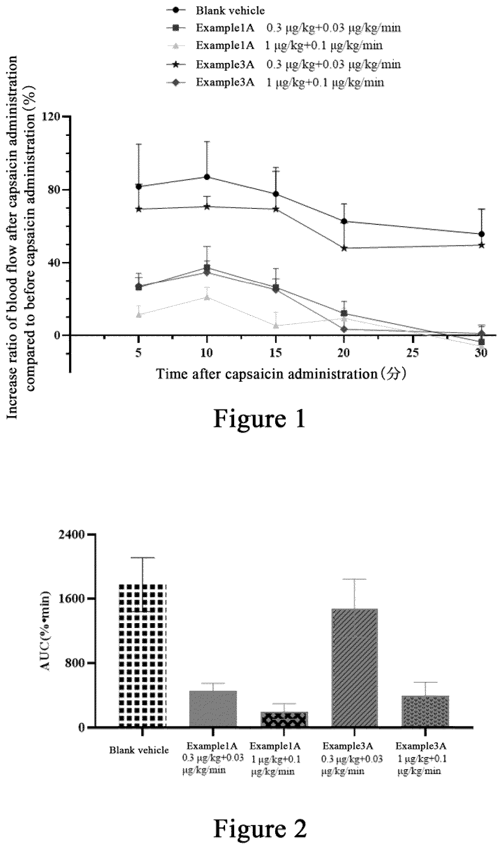

The effects of Example 1A and Example 3A on the blood flow of Cynomolgus monkeys after capsaicin stimulation are shown in Table 7, Figures 1 and 2.

**Table 7 Results of blood flow change rate, area under the blood flow change rate-time curve and blood flow inhibition rate in each group of animals after drug administration**

| Groups | N=3 | Blood flow change rate (%) | | | | | AUC (%·min) | Blood flow inhibition rate (%) |
|---|---|---|---|---|---|---|---|---|
| | | 5 min | 10 min | 15 min | 20 min | 30 min | | |
| Blank vehicle control group | MEAN ±SD | 82± 41 | 87± 33 | 78± 25 | 63± 17 | 56± 24 | 1778± 586 | 0±33 |
| Example 1A 0.3 µg/kg + 0.03 µg/kg/min | MEAN ±SD | 26± 14 | 37± 6 | 26± 18 | 12± 11 | -4± 15 | 454± 164** | 74±9** |
| Example 1A 1 µg/kg + 0.1 µg/kg/min | MEAN ±SD | 12± 8 | 21± 9 | 5±1 3 | 9±1 0 | -6±8 | 198± 169*** | 89±10*** |
| Example 3A 0.3 µg/kg + 0.03 µg/kg/min | MEAN ±SD | 69± 23 | 71± 10 | 69± 36 | 48± 24 | 49± 34 | 1481± 629 | 17±35 |
| Example 3A 1 µg/kg + 0.1 µg/kg/min | MEAN ±SD | 27± 8 | 35± 25 | 25± 11 | 3± 13 | 1±8 | 395± 288** | 78±16** |

| | | | | | | | | |
|---|---|---|---|---|---|---|---|---|
| Note: compared with the blank vehicle control group, *, **, *** indicate p≤0.05, p≤0.01, p≤0.001, respectively. | | | | | | | | |

### 5. Conclusion

Under the conditions of this experiment, compared with the blank vehicle control group, the low and high doses of Example 1A and the high dose of Example 3A can significantly inhibit the increase in skin blood flow induced by capsaicin, and the blood flow inhibition rate reaches more than 70%, while the low dose of Example 3A has no inhibitory effect on the increase in skin blood flow induced by capsaicin.

## Claims

1. A compound represented by general formula (I) or a stereoisomer, tautomer, deuterated derivative or pharmaceutically acceptable salt thereof: 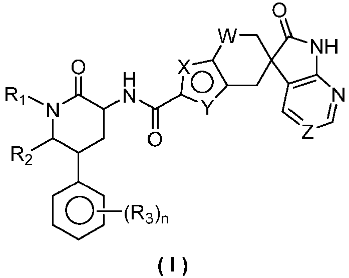 wherein
R₁ is selected from hydrogen atom, formyl, alkyl, cycloalkyl or heterocyclyl; wherein the alkyl, cycloalkyl or heterocyclyl is optionally substituted by one or more Rₐ substituents, preferably R₁ is selected from alkyl or alkyl substituted by Rₐ substituent, and further preferably R₁ is selected from C₁₋₆ alkyl or C₁₋₆ alkyl substituted by Rₐ substituent;
each Rₐ is the same or different, and is independently selected from deuterium, tritium, halogen, amino, hydroxyl, cyano, alkoxy, alkyl, cycloalkyl or heterocycloalkyl, wherein the amino, hydroxyl, cyano, alkoxy, alkyl, cycloalkyl or heterocycloalkyl is optionally substituted with one or more substituents selected from alkyl, haloalkyl, halogen, amino, hydroxyl, cyano or alkoxy;
R₂ is selected from alkyl, deuterated alkyl, aminoalkyl, haloalkyl, or hydroxyalkyl, preferably R₂ is alkyl, further preferably C₁₋₆ alkyl, further preferably methyl;
each R₃ is the same or different, and is independently selected from halogen, amino, hydroxyl, cyano, alkoxy, alkyl, cycloalkyl, heterocyclyl, aryl or heteroaryl, preferably R₃ is the same and is halogen;
X and Y are the same or different, and are each independently selected from =CR₄-, =N-, -NR₅-, -O-, -S-, -S(O)- or -S(O)₂-, preferably X is selected from =N-, -NR₅-, -O- or -S-, preferably Y is selected from =CR₄-, -O-, -NR₅-, =N- or -S-;
R₄ and R₅ are the same or different, and are each independently selected from hydrogen atom or alkyl;
W is selected from -CH₂- or a single bond;
Z is selected from =CH- or =N-; and
n is 0, 1, 2, 3, 4 or 5.

2. The compound according to claim 1 or a stereoisomer, tautomer, deuterated derivative or pharmaceutically acceptable salt thereof, which is a compound represented by general formula (II) or a stereoisomer, tautomer, deuterated derivative or pharmaceutically acceptable salt thereof: 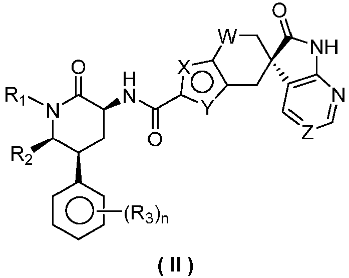 wherein the definitions of R₁, R₂, R₃, n, X, Y, W and Z are as described in claim 1.

3. The compound according to claim 2 or a stereoisomer, tautomer, deuterated derivative or pharmaceutically acceptable salt thereof, which is a compound represented by general formula (III) or a stereoisomer, tautomer, deuterated derivative or pharmaceutically acceptable salt thereof: 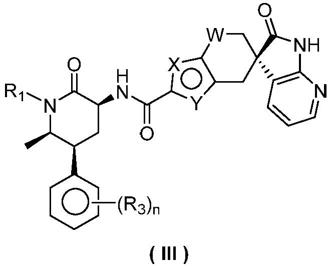 wherein the definitions of R₁, R₃, n, X, Y and W are as described in claim 1.

4. The compound according to claim 3 or a stereoisomer, tautomer, deuterated derivative or pharmaceutically acceptable salt thereof, which is a compound represented by general formula (IV) or a stereoisomer, tautomer, deuterated derivative or pharmaceutically acceptable salt thereof: 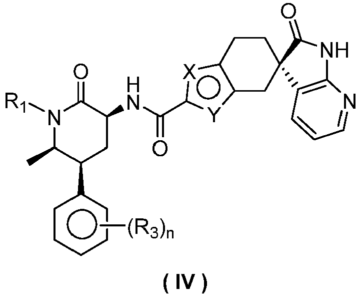 wherein the definitions of R₁, R₃, n, X and Y are as described in claim 1.

5. The compound according to claim 3 or a stereoisomer, tautomer, deuterated derivative or pharmaceutically acceptable salt thereof, which is a compound represented by general formula (V) or a stereoisomer, tautomer, deuterated derivative or pharmaceutically acceptable salt thereof: 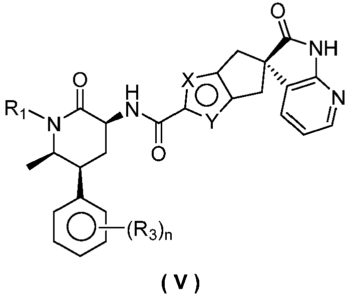 wherein the definitions of R₁, R₃, n, X and Y are as described in claim 1.

6. A compound according to any one of claims 1 to 5 or a stereoisomer, tautomer, deuterated derivative or a pharmaceutically acceptable salt thereof, wherein R₁ is selected from isopropyl, 2,2,2-trifluoroethyl, 2,2-difluoroethyl, 2-methylpropyl, 3,3,3-trifluoropropyl or 3,3,3-trifluoro-2-hydroxypropyl.

7. The compound according to any one of claims 1 to 6 or a stereoisomer, tautomer, deuterated derivative or pharmaceutically acceptable salt thereof, wherein R₁ is 2,2,2-trifluoroethyl.

8. The compound according to any one of claims 1 to 5 or a stereoisomer, tautomer, deuterated derivative or pharmaceutically acceptable salt thereof, wherein R₃ is selected from fluorine, and n is 3.

9. The compound according to any one of claims 1 to 8 or a stereoisomer, tautomer, deuterated derivative or pharmaceutically acceptable salt thereof, wherein the compound is:

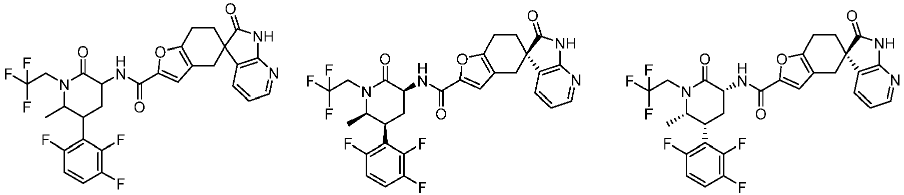

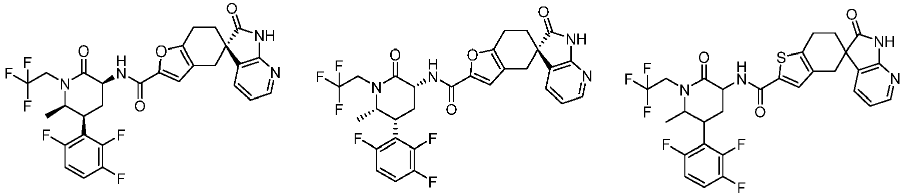

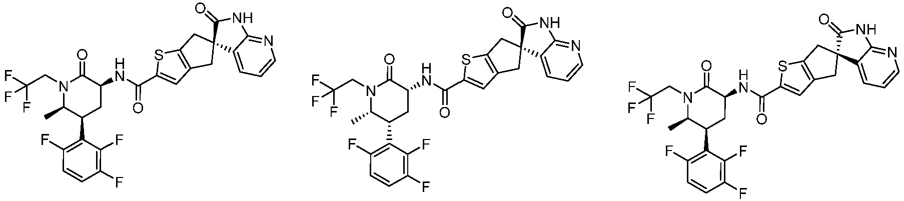

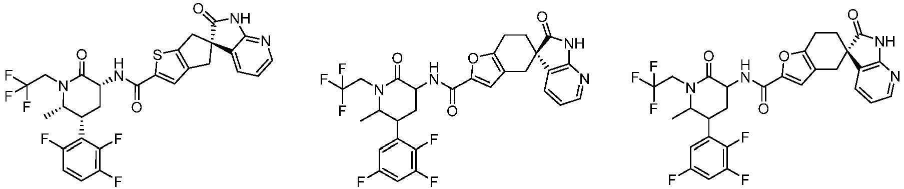

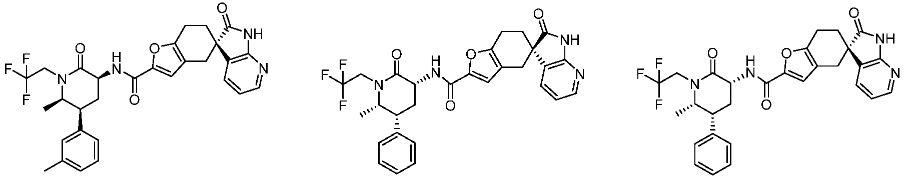

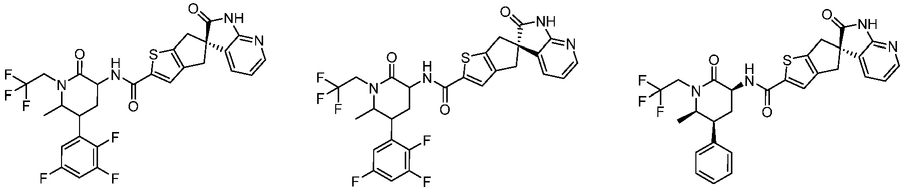

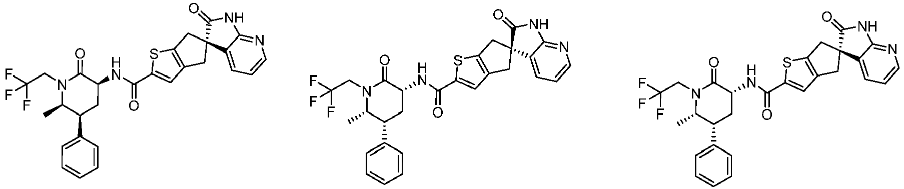

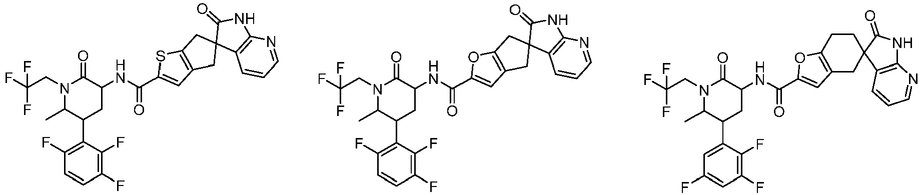

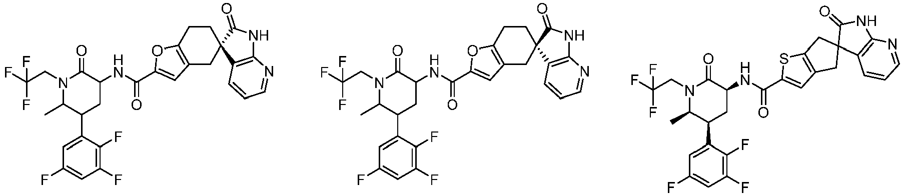

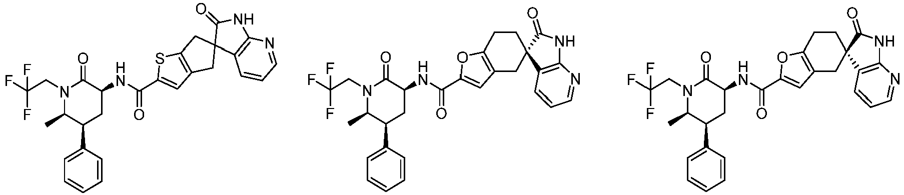

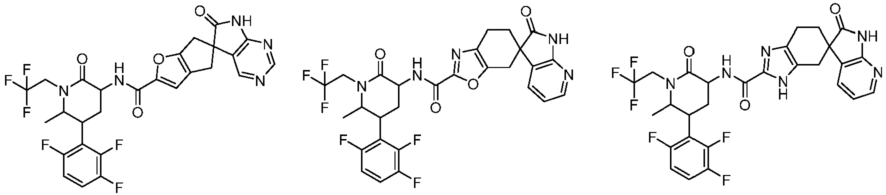

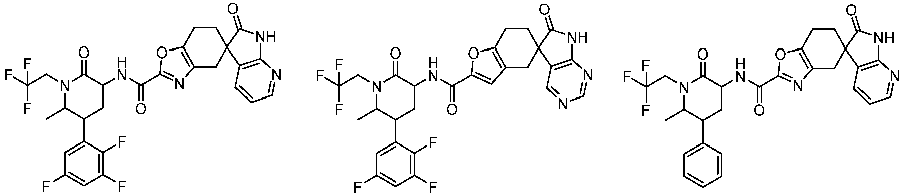

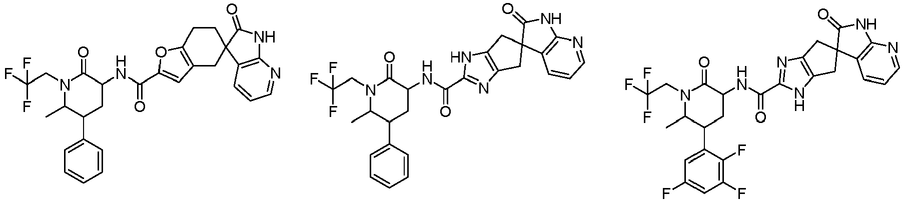

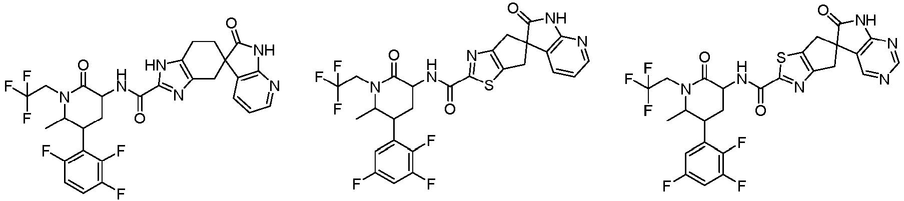

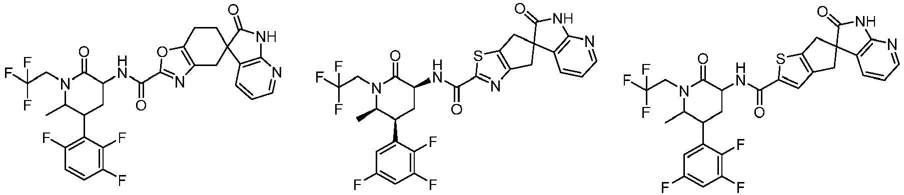

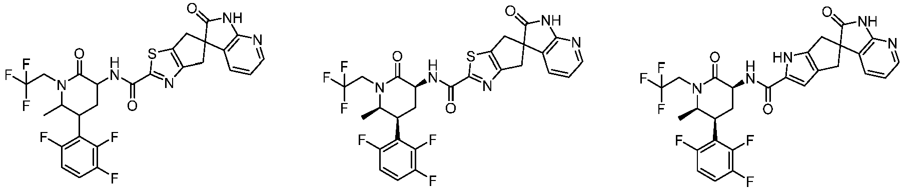

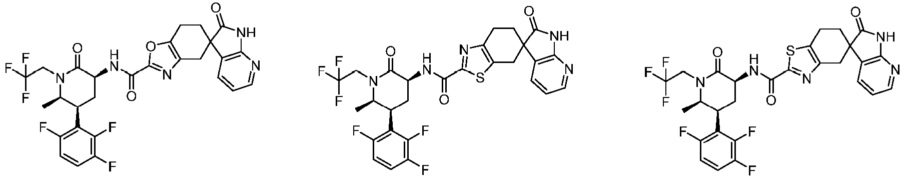

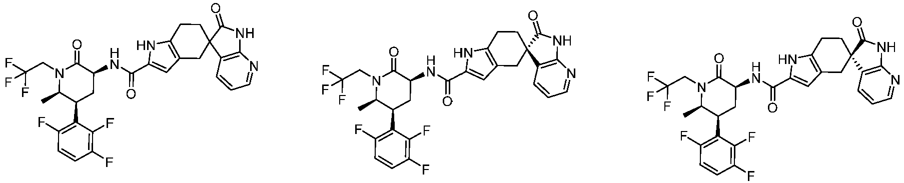

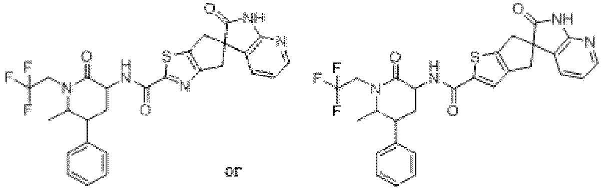

10. A pharmaceutical composition, comprising:
the compound according to any one of claims 1 to 9 or a stereoisomer, tautomer, deuterated derivative or pharmaceutically acceptable salt thereof, and a pharmaceutically acceptable carrier, excipient, or a combination thereof.

11. The compound according to any one of claims 1 to 9 or a stereoisomer, tautomer, deuterated derivative or pharmaceutically acceptable salt thereof, or the pharmaceutical composition according to claim 10 for use as a CGRP receptor antagonist.

12. The compound according to any one of claims 1 to 9 or a stereoisomer, tautomer, deuterated derivative or pharmaceutically acceptable salt thereof, or the pharmaceutical composition according to claim 10 for use as a medicament for preventing and/or treating a disease mediated by CGRP, wherein the disease mediated by CGRP is a cerebrovascular or vascular disorder disease.

13. The compound or a stereoisomer, tautomer, deuterated derivative or pharmaceutically acceptable salt thereof, or the pharmaceutical composition for use according to claim 12, wherein the cerebral vascular or vascular disorder disease mediated by CGRP is selected from the group consisting of: episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

14. The compound according to any one of claims 1 to 9 or a stereoisomer, tautomer, deuterated derivative or pharmaceutically acceptable salt thereof, or the pharmaceutical composition according to claim 10 for use in preventing and/or treating a disease mediated by CGRP, wherein the disease mediated by CGRP is a cerebrovascular or vascular disorder disease.

15. The compound or a stereoisomer, tautomer, deuterated derivative or pharmaceutically acceptable salt thereof, or the pharmaceutical composition for use according to claim 14, wherein the cerebral vascular or vascular disorder disease mediated by CGRP is selected from the group consisting of: episodic migraine, migraine without aura, chronic migraine, pure menstrual migraine, menstrual related migraine, migraine with aura, migraine in children/adolescents, hemiplegic migraine, sporadic hemiplegic migraine, basal migraine, periodic vomiting, abdominal migraine, benign paroxysmal vertigo in childhood, retinal migraine, cluster headache, dialysis headache, chronic headache of unknown cause, tension/pressure induced headache, allergy induced headache, osteoarthritis and related osteoporotic fracture pain, hot flashes related to menopause or medically induced menopause caused by surgery or medication, periodic vomiting syndrome, opioid withdrawal, psoriasis, asthma, obesity, morphine tolerance, neurodegenerative diseases, epilepsy, allergic rhinitis, rosacea, toothache, earache, otitis media, sunburn, arthralgia related to osteoarthritis and rheumatoid arthritis, cancer pain, fibromyalgia, diabetes neuropathy, gout, trigeminal neuralgia, nasal polyps, chronic sinusitis, temporomandibular syndrome, back pain, low back pain, cough, dystonic pain, inflammatory pain, postoperative incision pain, sciatica, complex regional pain syndrome, Behcet's disease, endometriosis, phantom limb syndrome, pain menstruation, pain related to childbirth, pain caused by skin burns, and chronic secondary visceral pain, such as inflammatory bowel disease (including Crohn's disease, ileitis and ulcerative colitis), gastroesophageal reflux disease, dyspepsia, irritable bowel syndrome, renal colic, cystitis, pancreatitis, and prostatitis.

## Patentansprüche

1. Eine Verbindung, dargestellt durch die allgemeine Formel (I), oder ein Stereoisomer, Tautomer, deuteriertes Derivat oder pharmazeutisch annehmbares Salz davon: 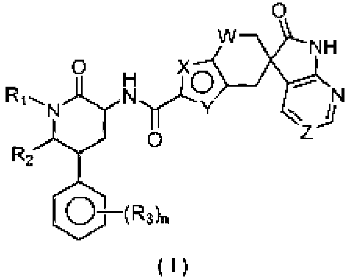 wobei
R₁ ausgewählt ist aus Wasserstoffatom, Formyl, Alkyl, Cycloalkyl oder Heterocyclyl; wobei das Alkyl, Cycloalkyl oder Heterocyclyl gegebenenfalls durch einen oder mehrere Rₐ Substituenten substituiert ist, vorzugsweise R₁ ausgewählt ist aus Alkyl oder Alkyl substituiert durch einen Rₐ Substituenten, und weiter vorzugsweise R₁ ausgewählt ist aus C₁₋₆Alkyl oder C₁₋₆ Alkyl substituiert durch einen Rₐ Substituenten;
jedes Rₐ gleich oder verschieden ist und unabhängig ausgewählt ist aus Deuterium, Tritium, Halogen, Amino, Hydroxyl, Cyano, Alkoxy, Alkyl, Cycloalkyl oder Heterocycloalkyl, wobei das Amino, Hydroxyl, Cyano, Alkoxy, Alkyl, Cycloalkyl oder Heterocycloalkyl gegebenenfalls mit einem oder mehreren Substituenten substituiert ist, ausgewählt aus Alkyl, Halogenalkyl, Halogen, Amino, Hydroxyl, Cyano oder Alkoxy;
R₂ ausgewählt ist aus Alkyl, deuteriertem Alkyl, Aminoalkyl, Halogenalkyl oder Hydroxyalkyl, vorzugsweise R₂ Alkyl ist, weiter vorzugsweise C₁₋₆ Alkyl, weiter vorzugsweise Methyl;
jedes R₃ gleich oder verschieden ist und unabhängig ausgewählt ist aus Halogen, Amino, Hydroxyl, Cyano, Alkoxy, Alkyl, Cycloalkyl, Heterocyclyl, Aryl oder Heteroaryl, vorzugsweise R₃ gleich ist und Halogen ist;
X und Y gleich oder verschieden sind und jeweils unabhängig ausgewählt sind aus =CR₄-, =N-, -NR₅-, -O-, -S-, -S(O)- oder -S(O)₂-, vorzugsweise X ausgewählt ist aus =N-, -NR₅-, -O- oder -S-, vorzugsweise Y ausgewählt ist aus =CR₄, -O-, -NR₅-, =N- oder -S-;
R₄ und R₅ gleich oder verschieden sind und jeweils unabhängig ausgewählt sind aus Wasserstoffatom oder Alkyl;
W ausgewählt ist aus -CH₂- oder einer Einfachbindung;
Z ausgewählt ist aus =CH- oder =N-; und
n gleich 0, 1, 2, 3, 4 oder 5 ist.

2. Die Verbindung nach Anspruch 1 oder ein Stereoisomer, Tautomer, deuteriertes Derivat oder pharmazeutisch annehmbares Salz davon, die eine Verbindung ist, dargestellt durch die allgemeine Formel (II), oder ein Stereoisomer, Tautomer, deuteriertes Derivat oder pharmazeutisch annehmbares Salz davon: 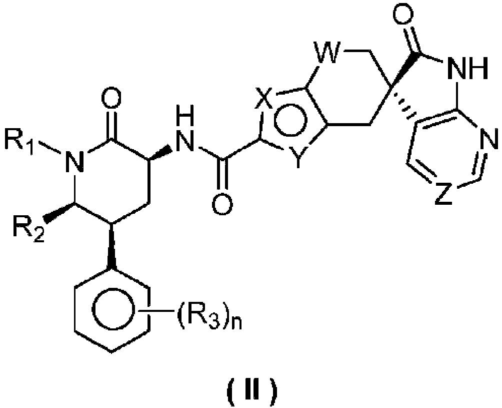 wobei die Definitionen von R₁, R₂, R₃, n, X, Y, W und Z wie in Anspruch 1 beschrieben sind.

3. Die Verbindung nach Anspruch 2 oder ein Stereoisomer, Tautomer, deuteriertes Derivat oder pharmazeutisch annehmbares Salz davon, die eine Verbindung ist, dargestellt durch die allgemeine Formel (III), oder ein Stereoisomer, Tautomer, deuteriertes Derivat oder pharmazeutisch annehmbares Salz davon: 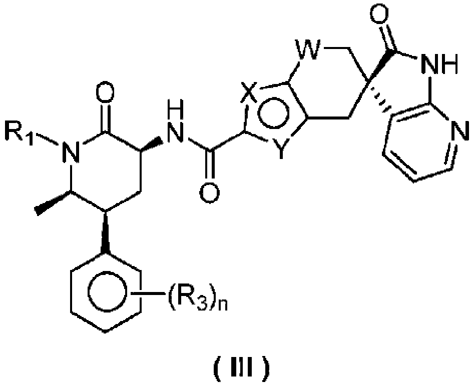 wobei die Definitionen von R₁, R₃, n, X, Y und W wie in Anspruch 1 beschrieben sind.

4. Die Verbindung nach Anspruch 3 oder ein Stereoisomer, Tautomer, deuteriertes Derivat oder pharmazeutisch annehmbares Salz davon, die eine Verbindung ist, dargestellt durch die allgemeine Formel (IV), oder ein Stereoisomer, Tautomer, deuteriertes Derivat oder pharmazeutisch annehmbares Salz davon: 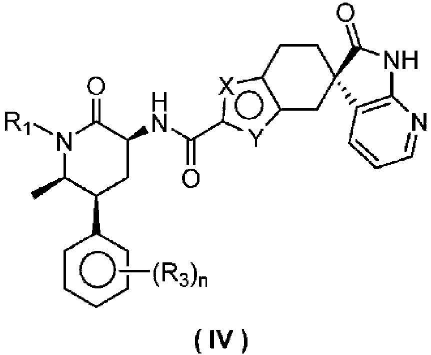 wobei die Definitionen von R₁, R₃, n, X und Y wie in Anspruch 1 beschrieben sind.

5. Die Verbindung nach Anspruch 3 oder ein Stereoisomer, Tautomer, deuteriertes Derivat oder pharmazeutisch annehmbares Salz davon, die eine Verbindung ist, dargestellt durch die allgemeine Formel (V), oder ein Stereoisomer, Tautomer, deuteriertes Derivat oder pharmazeutisch annehmbares Salz davon: 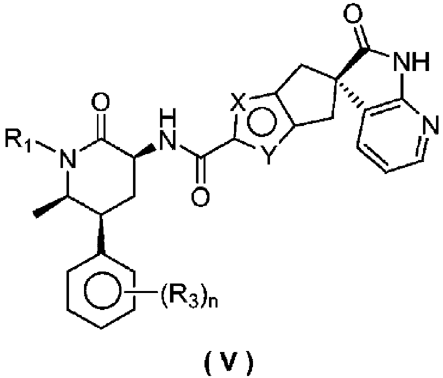 wobei die Definitionen von R₁, R₃, n, X und Y wie in Anspruch 1 beschrieben sind.

6. Eine Verbindung nach einem der Ansprüche 1 bis 5 oder ein Stereoisomer, Tautomer, deuteriertes Derivat oder ein pharmazeutisch annehmbares Salz davon, wobei R₁ ausgewählt ist aus Isopropyl, 2,2,2-Trifluorethyl, 2,2-Difluorethyl, 2-Methylpropyl, 3,3,3-Trifluorpropyl oder 3,3,3-Trifluor-2-hydroxypropyl.

7. Die Verbindung nach einem der Ansprüche 1 bis 6 oder ein Stereoisomer, Tautomer, deuteriertes Derivat oder pharmazeutisch annehmbares Salz davon, wobei R₁ 2,2,2-Trifluorethyl ist.

8. Die Verbindung nach einem der Ansprüche 1 bis 5 oder ein Stereoisomer, Tautomer, deuteriertes Derivat oder pharmazeutisch annehmbares Salz davon, wobei R₃ ausgewählt ist aus Fluor und n gleich 3 ist.

9. Die Verbindung nach einem der Ansprüche 1 bis 8 oder ein Stereoisomer, Tautomer, deuteriertes Derivat oder pharmazeutisch annehmbares Salz davon, wobei die Verbindung ist:

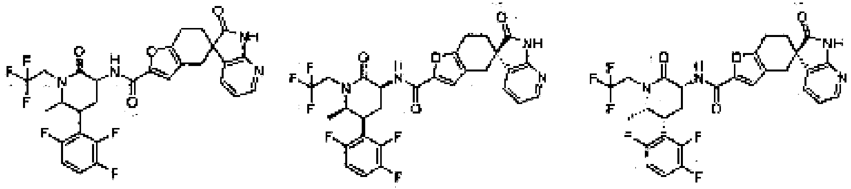

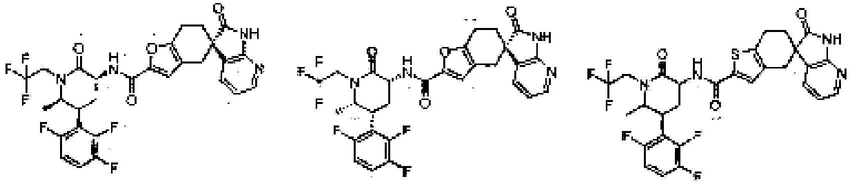

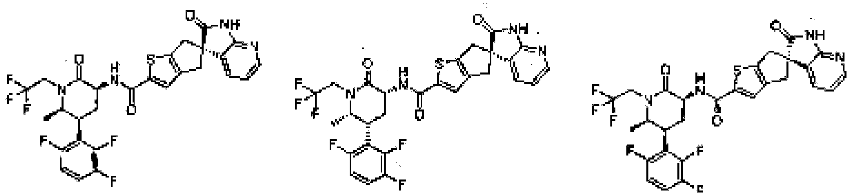

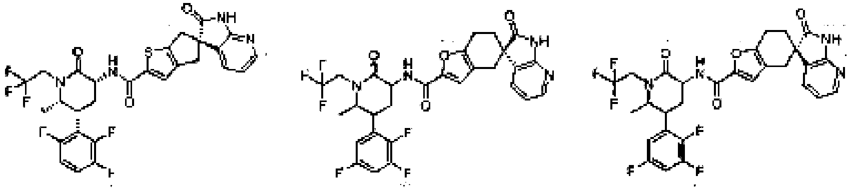

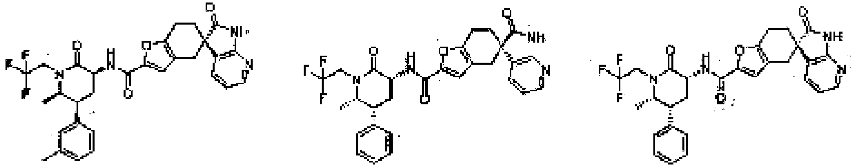

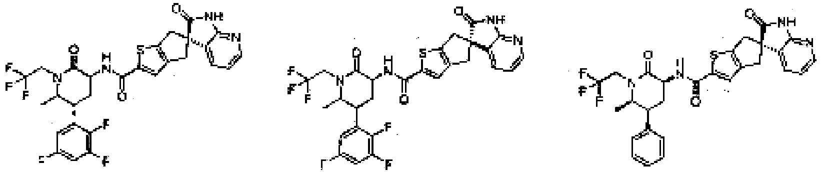

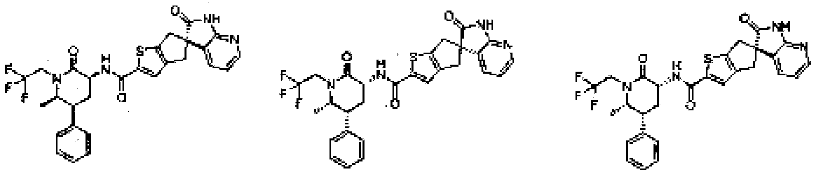

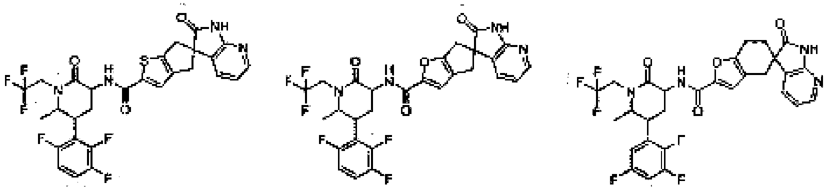

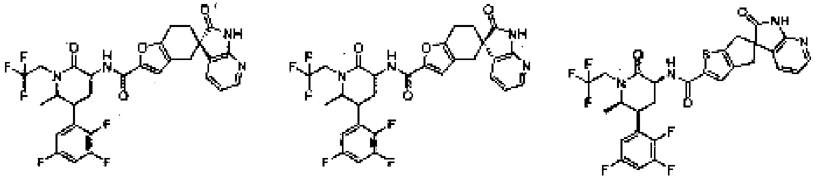

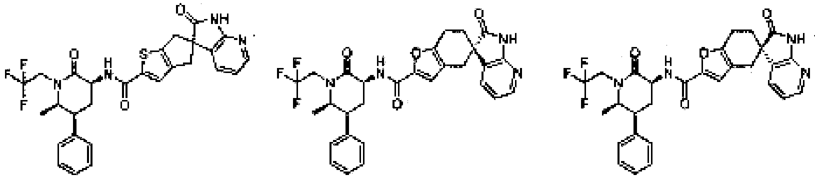

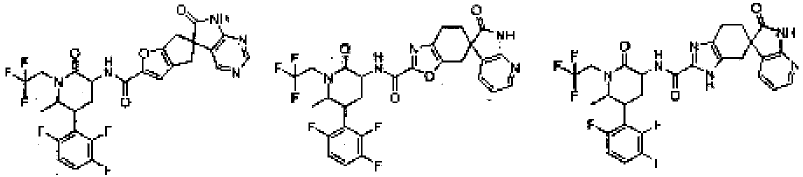

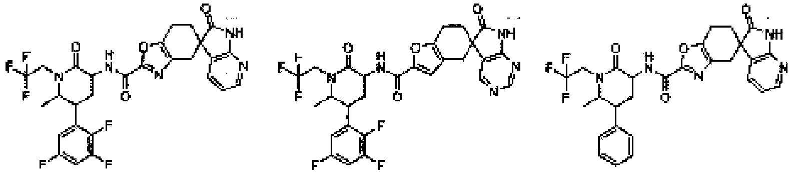

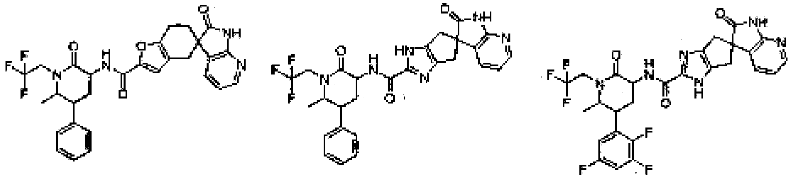

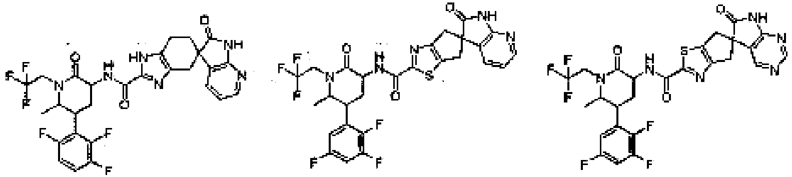

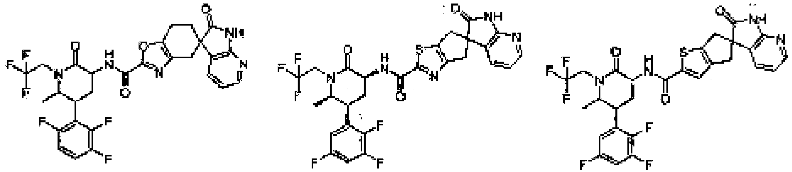

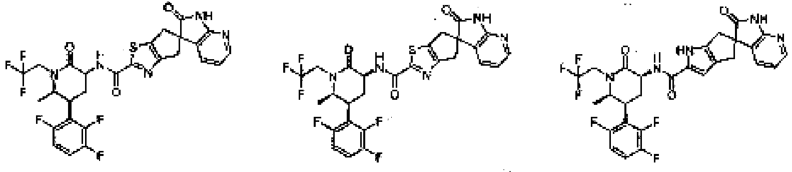

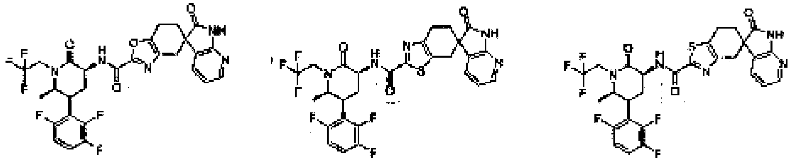

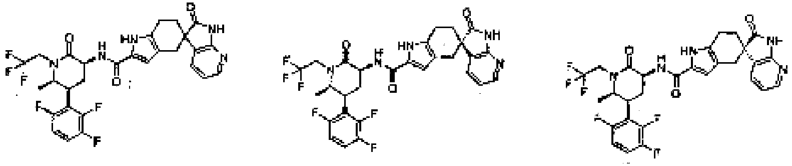

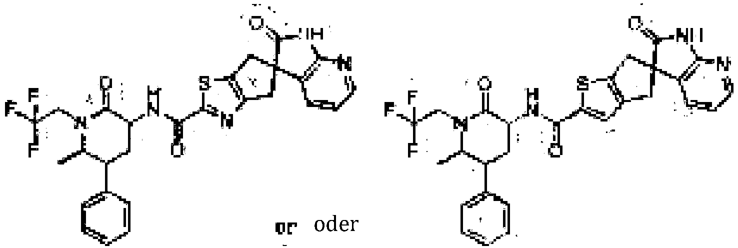

10. Eine pharmazeutische Zusammensetzung, umfassend:
die Verbindung nach einem der Ansprüche 1 bis 9 oder ein Stereoisomer, Tautomer, deuteriertes Derivat oder pharmazeutisch annehmbares Salz davon und einen pharmazeutisch annehmbaren Träger, Hilfsstoff oder eine Kombination davon.

11. Die Verbindung nach einem der Ansprüche 1 bis 9 oder ein Stereoisomer, Tautomer, deuteriertes Derivat oder pharmazeutisch annehmbares Salz davon oder die pharmazeutische Zusammensetzung nach Anspruch 10 zur Verwendung als CGRP-Rezeptorantagonist.

12. Die Verbindung nach einem der Ansprüche 1 bis 9 oder ein Stereoisomer, Tautomer, deuteriertes Derivat oder pharmazeutisch annehmbares Salz davon oder die pharmazeutische Zusammensetzung nach Anspruch 10 zur Verwendung als Medikament zur Vorbeugung und/oder Behandlung einer durch CGRP vermittelten Erkrankung, wobei die durch CGRP vermittelte Erkrankung eine zerebrovaskuläre oder vaskuläre Störungs-Erkrankung ist.

13. Die Verbindung oder ein Stereoisomer, Tautomer, deuteriertes Derivat oder pharmazeutisch annehmbares Salz davon oder die pharmazeutische Zusammensetzung zur Verwendung gemäß Anspruch 12, wobei die durch CGRP vermittelte zerebrale Gefäß- oder Gefäßstörungserkrankung ausgewählt ist aus der Gruppe, bestehend aus: episodischer Migräne, Migräne ohne Aura, chronischer Migräne, reiner menstrueller Migräne, menstruationsbezogener Migräne, Migräne mit Aura, Migräne bei Kindern/Jugendlichen, hemiplegischer Migräne, sporadischer hemiplegischer Migräne, basaler Migräne, periodischem Erbrechen, abdominaler Migräne, gutartigem paroxysmalem Schwindel im Kindesalter, retinaler Migräne, Cluster-Kopfschmerz, Dialyse-Kopfschmerz, chronischem Kopfschmerz unbekannter Ursache, spannungs-/druckinduziertem Kopfschmerz, allergieinduziertem Kopfschmerz, Osteoarthritis und damit verbundenem osteoporotischem Frakturschmerz, Hitzewallungen im Zusammenhang mit Menopause oder medizinisch induzierter Menopause, verursacht durch Operation oder Medikation, periodischem Erbrechenssyndrom, Opioid-Entzug, Psoriasis, Asthma, Adipositas, Morphin-Toleranz, neurodegenerativen Erkrankungen, Epilepsie, allergischer Rhinitis, Rosazea, Zahnschmerzen, Ohrenschmerzen, Otitis media, Sonnenbrand, Arthralgie im Zusammenhang mit Osteoarthritis und rheumatoider Arthritis, Krebsschmerz, Fibromyalgie, Diabetes-Neuropathie, Gicht, Trigeminusneuralgie, Nasenpolypen, chronischer Sinusitis, temporomandibulärem Syndrom, Rückenschmerzen, Schmerzen im unteren Rücken, Husten, dystonem Schmerz, entzündlichem Schmerz, postoperativem Inzisionsschmerz, Ischias, komplexem regionalem Schmerzsyndrom, Behcet-Krankheit, Endometriose, Phantomgliedsyndrom, Schmerzmenstruation, Schmerz im Zusammenhang mit Geburt, Schmerz, verursacht durch Hautverbrennungen, und chronischem sekundärem viszeralem Schmerz, wie entzündliche Darmerkrankung (einschließlich Morbus Crohn, Ileitis und Colitis ulcerosa), gastroösophageale Refluxkrankheit, Dyspepsie, Reizdarmsyndrom, Nierenkolik, Zystitis, Pankreatitis und Prostatitis.

14. Die Verbindung nach einem der Ansprüche 1 bis 9 oder ein Stereoisomer, Tautomer, deuteriertes Derivat oder pharmazeutisch annehmbares Salz davon oder die pharmazeutische Zusammensetzung nach Anspruch 10 zur Verwendung bei der Vorbeugung und/oder Behandlung einer durch CGRP vermittelten Erkrankung, wobei die durch CGRP vermittelte Erkrankung eine zerebrovaskuläre oder vaskuläre Störungs- Erkrankung ist.

15. Die Verbindung oder ein Stereoisomer, Tautomer, deuteriertes Derivat oder pharmazeutisch annehmbares Salz davon oder die pharmazeutische Zusammensetzung zur Verwendung gemäß Anspruch 14, wobei die durch CGRP vermittelte zerebrale Gefäß- oder Gefäßstörungserkrankung ausgewählt ist aus der Gruppe, bestehend aus: episodischer Migräne, Migräne ohne Aura, chronischer Migräne, reiner menstrueller Migräne, menstruationsbezogener Migräne, Migräne mit Aura, Migräne bei Kindern/Jugendlichen, hemiplegischer Migräne, sporadischer hemiplegischer Migräne, basaler Migräne, periodischem Erbrechen, abdominaler Migräne, gutartigem paroxysmalem Schwindel im Kindesalter, retinaler Migräne, Cluster-Kopfschmerz, Dialyse-Kopfschmerz, chronischem Kopfschmerz unbekannter Ursache, spannungs-/druckinduziertem Kopfschmerz, allergieinduziertem Kopfschmerz, Osteoarthritis und damit verbundenem osteoporotischem Frakturschmerz, Hitzewallungen im Zusammenhang mit Menopause oder medizinisch induzierter Menopause, verursacht durch Operation oder Medikation, periodischem Erbrechenssyndrom, Opioid-Entzug, Psoriasis, Asthma, Adipositas, Morphin-Toleranz, neurodegenerativen Erkrankungen, Epilepsie, allergischer Rhinitis, Rosazea, Zahnschmerzen, Ohrenschmerzen, Otitis media, Sonnenbrand, Arthralgie im Zusammenhang mit Osteoarthritis und rheumatoider Arthritis, Krebsschmerz, Fibromyalgie, Diabetes-Neuropathie, Gicht, Trigeminusneuralgie, Nasenpolypen, chronischer Sinusitis, temporomandibulärem Syndrom, Rückenschmerzen, Schmerzen im unteren Rücken, Husten, dystonem Schmerz, entzündlichem Schmerz, postoperativem Inzisionsschmerz, Ischias, komplexem regionalem Schmerz-syndrom, Behcet-Krankheit, Endometriose, Phantomgliedsyndrom, Schmerzmenstruation, Schmerz im Zusammenhang mit Geburt, Schmerz, verursacht durch Hautverbrennungen, und chronischem sekundärem viszeralem Schmerz, wie entzündliche Darmerkrankung (einschließlich Morbus Crohn, Ileitis und Colitis ulcerosa), gastroösophageale Refluxkrankheit, Dyspepsie, Reizdarmsyndrom, Nierenkolik, Zystitis, Pankreatitis und Prostatitis.

## Revendications

1. Composé représenté par la formule générale (I) ou un stéréoisomère, tautomère, dérivé deutéré ou sel pharmaceutiquement acceptable de celui-ci : 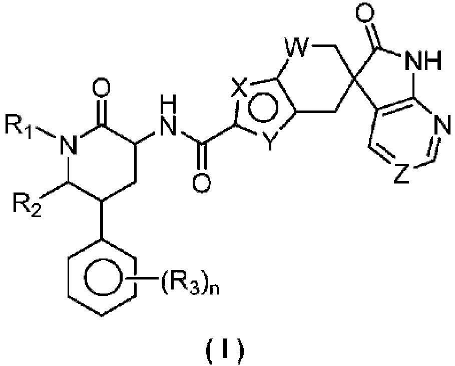 dans lequel
R₁ est choisi parmi un atome d'hydrogène, formyle, alkyle, cycloalkyle ou hétérocyclyle ; dans lequel l'alkyle, le cycloalkyle ou l'hétérocyclyle est éventuellement substitué par un ou plusieurs substituants Rₐ, de préférence R₁ est choisi parmi alkyle ou alkyle substitué par un substituant Rₐ, et plus préférablement R₁ est choisi parmi alkyle en C₁₋₆ ou alkyle en C₁₋₆ substitué par un substituant Rₐ;
chaque Rₐ est identique ou différent, et est indépendamment choisi parmi deutérium, tritium, halogène, amino, hydroxyle, cyano, alcoxy, alkyle, cycloalkyle ou hétérocycloalkyle, dans lequel l'amino, l'hydroxyle, le cyano, l'alcoxy, l'alkyle, le cycloalkyle ou l'hétérocycloalkyle est éventuellement substitué par un ou plusieurs substituants choisis parmi alkyle, haloalkyle, halogène, amino, hydroxyle, cyano ou alcoxy ;
R₂ est choisi parmi alkyle, alkyle deutéré, aminoalkyle, haloalkyle, ou hydroxyalkyle, de préférence R₂ est alkyle, plus préférablement alkyle en C₁₋₆, plus préférablement méthyle ;
chaque R₃ est identique ou différent, et est indépendamment choisi parmi halogène, amino, hydroxyle, cyano, alcoxy, alkyle, cycloalkyle, hétérocyclyle, aryle ou hétéroaryle, de préférence R₃ est identique et est halogène ;
X et Y sont identiques ou différents, et sont chacun indépendamment choisis parmi =CR₄-, =N-, -NR₅-, -O-, -S-, -S(O)- ou -S(O)₂, de préférence X est choisi parmi =N-, -NR₅, -O- ou -S-, de préférence Y est choisi parmi = CR₄-, -O-, - NR₅-, =N- ou -S- ;
R₄ et R₅ sont identiques ou différents, et sont chacun indépendamment choisis parmi un atome d'hydrogène ou alkyle ;
W est choisi parmi -CH₂- ou une liaison simple ;
Z est choisi parmi =CH- ou =N- ; et
n vaut 0, 1, 2, 3, 4 ou 5.

2. Composé selon la revendication 1 ou un stéréoisomère, tautomère, dérivé deutéré ou sel pharmaceutiquement acceptable de celui-ci, qui est un composé représenté par la formule générale (II) ou un stéréoisomère, tautomère, dérivé deutéré ou sel pharmaceutiquement acceptable de celui-ci 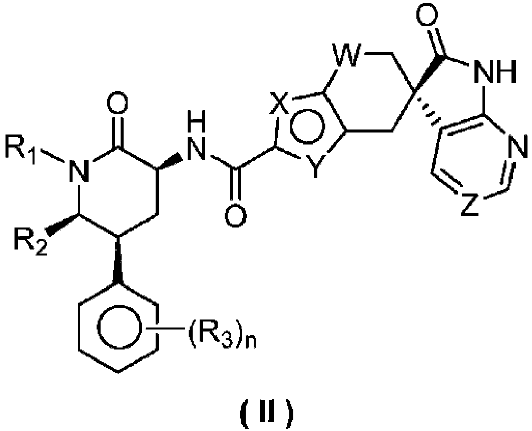 dans lequel les définitions de R₁, R₂, R₃, n, X, Y, W et Z sont telles que décrites dans la revendication 1.

3. Composé selon la revendication 2 ou un stéréoisomère, tautomère, dérivé deutéré ou sel pharmaceutiquement acceptable de celui-ci, qui est un composé représenté par la formule générale (III) ou un stéréoisomère, tautomère, dérivé deutéré ou sel pharmaceutiquement acceptable de celui-ci : 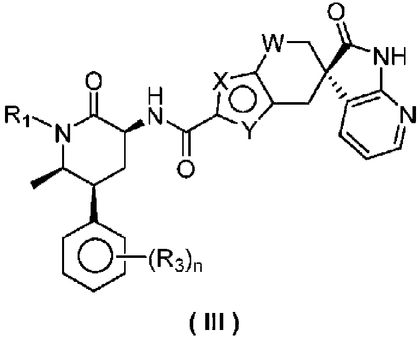 dans lequel les définitions de R₁, R₃, n, X, Y et W sont telles que décrites dans la revendication 1.

4. Composé selon la revendication 3 ou un stéréoisomère, tautomère, dérivé deutéré ou sel pharmaceutiquement acceptable de celui-ci, qui est un composé représenté par la formule générale (IV) ou un stéréoisomère, tautomère, dérivé deutéré ou sel pharmaceutiquement acceptable de celui-ci : 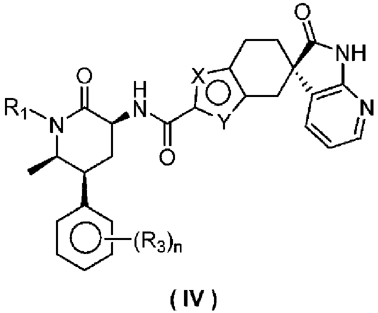 dans lequel les définitions de R₁, R₃, n, X et Y sont telles que décrites dans la revendication 1.

5. Composé selon la revendication 3 ou un stéréoisomère, tautomère, dérivé deutéré ou sel pharmaceutiquement acceptable de celui-ci, qui est un composé représenté par la formule générale (V) ou un stéréoisomère, tautomère, dérivé deutéré ou sel pharmaceutiquement acceptable de celui-ci : 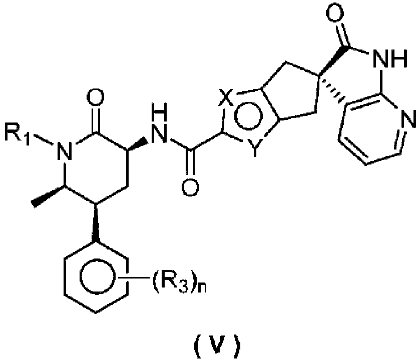 dans lequel les définitions de R₁, R₃, n, X et Y sont telles que décrites dans la revendication 1.

6. Composé selon l'une quelconque des revendications 1 à 5 ou un stéréoisomère, tautomère, dérivé deutéré ou un sel pharmaceutiquement acceptable de celui-ci, dans lequel R₁ est choisi parmi isopropyle, 2,2,2-trifluoroéthyle, 2,2-difluoroéthyle, 2-méthylpropyle, 3,3,3-trifluoropropyle ou 3,3,3-trifluoro-2-hydroxypropyle.

7. Composé selon l'une quelconque des revendications 1 à 6 ou un stéréoisomère, tautomère, dérivé deutéré ou sel pharmaceutiquement acceptable de celui-ci, dans lequel R₁ est 2,2,2-trifluoroéthyle.

8. Composé selon l'une quelconque des revendications 1 à 5 ou un stéréoisomère, tautomère, dérivé deutéré ou sel pharmaceutiquement acceptable de celui-ci, dans lequel R₃ est choisi parmi fluor, et n vaut 3.

9. Composé selon l'une quelconque des revendications 1 à 8 ou un stéréoisomère, tautomère, dérivé deutéré ou sel pharmaceutiquement acceptable de celui-ci, dans lequel le composé est :

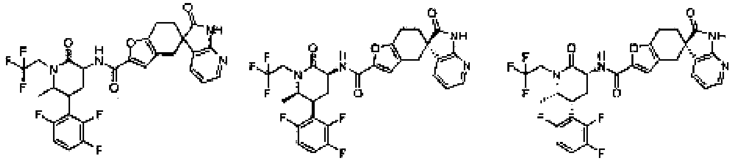

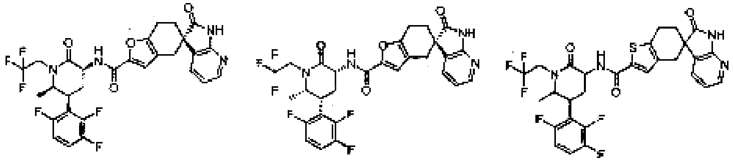

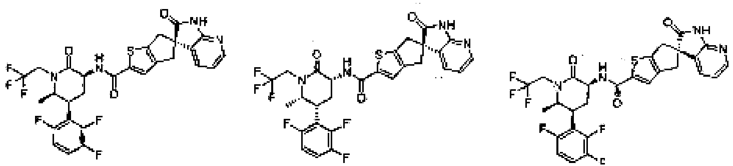

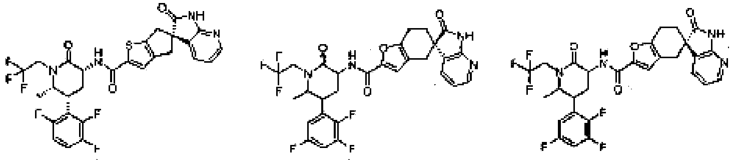

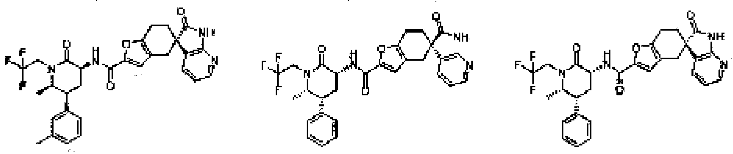

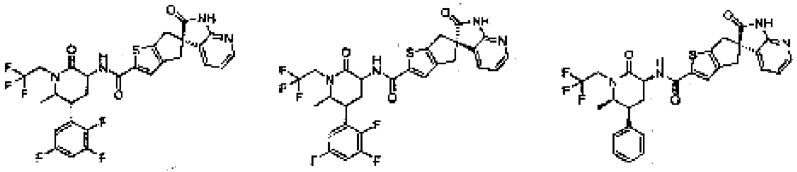

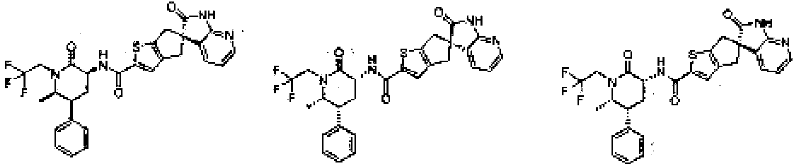

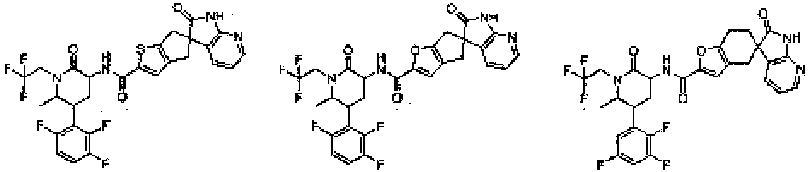

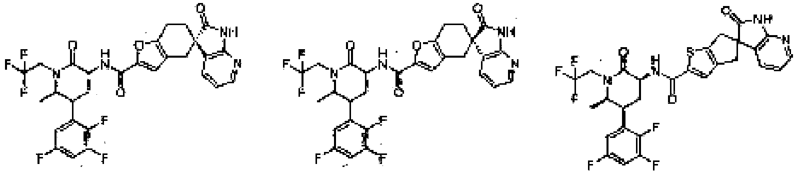

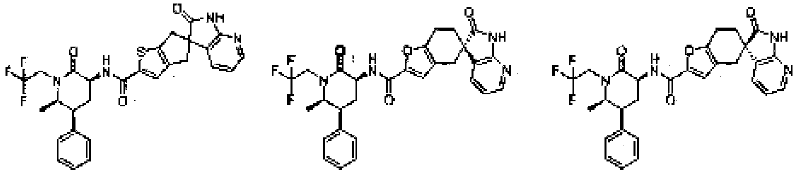

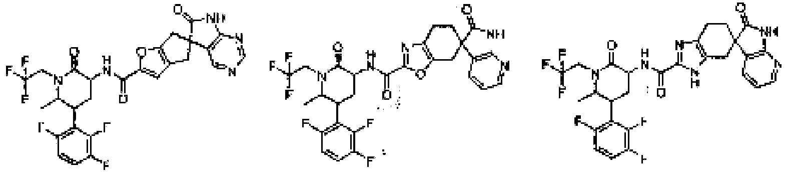

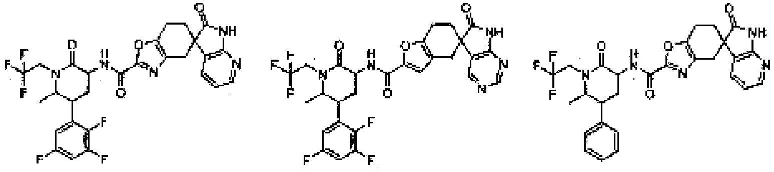

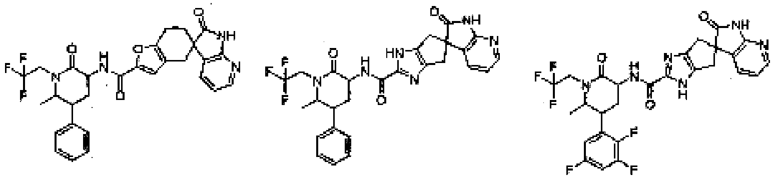

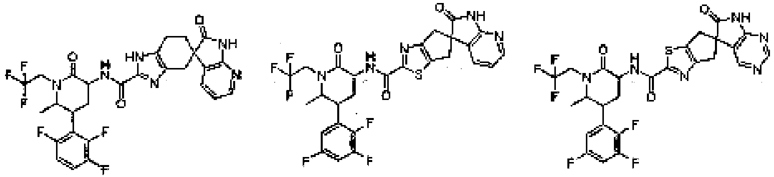

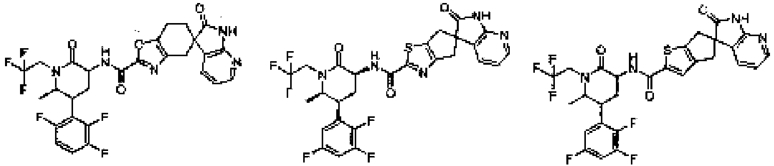

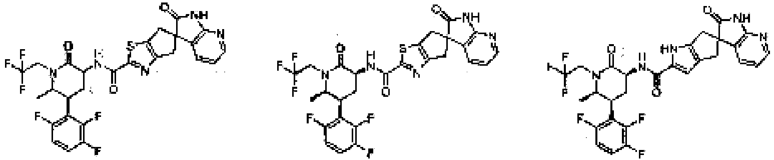

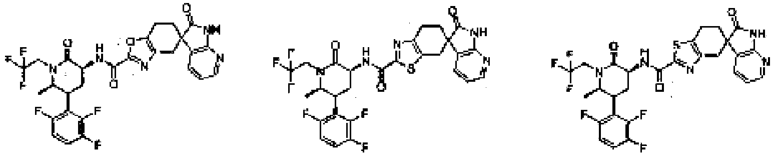

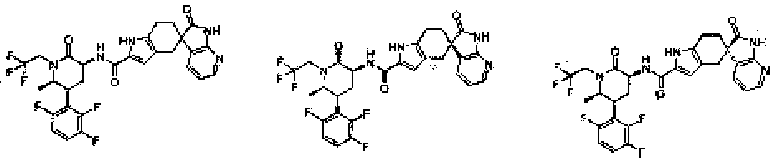

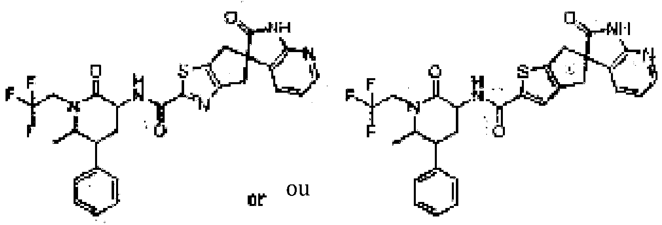

10. Composition pharmaceutique, comprenant :
le composé selon l'une quelconque des revendications 1 à 9 ou un stéréoisomère,
tautomère, dérivé deutéré ou sel pharmaceutiquement acceptable de celui-ci, et un véhicule pharmaceutiquement acceptable, un excipient, ou une combinaison de ceux-ci.

11. Composé selon l'une quelconque des revendications 1 à 9 ou un stéréoisomère, tautomère, dérivé deutéré ou sel pharmaceutiquement acceptable de celui-ci, ou composition pharmaceutique selon la revendication 10 pour une utilisation en tant qu'antagoniste du récepteur du CGRP.

12. Composé selon l'une quelconque des revendications 1 à 9 ou un stéréoisomère, tautomère, dérivé deutéré ou sel pharmaceutiquement acceptable de celui-ci, ou composition pharmaceutique selon la revendication 10 pour une utilisation en tant que médicament pour prévenir et/ou traiter une maladie médiée par le CGRP, dans lequel la maladie médiée par le CGRP est une maladie de trouble cérébrovasculaire ou vasculaire.

13. Composé ou un stéréoisomère, tautomère, dérivé deutéré ou sel pharmaceutiquement acceptable de celui-ci, ou composition pharmaceutique pour une utilisation selon la revendication 12, dans lequel la maladie de trouble vasculaire cérébral ou vasculaire médiée par le CGRP est choisie dans le groupe constitué de : migraine épisodique, migraine sans aura, migraine chronique, migraine menstruelle pure, migraine liée aux menstruations, migraine avec aura, migraine chez les enfants/adolescents, migraine hémiplégique, migraine hémiplégique sporadique, migraine basilaire, vomissements périodiques, migraine abdominale, vertige paroxystique bénin de l'enfance, migraine rétinienne, céphalée en grappes, céphalée de dialyse, céphalée chronique de cause inconnue, céphalée induite par tension/pression, céphalée induite par allergie, ostéoarthrite et douleur de fracture ostéoporotique apparentée, bouffées de chaleur liées à la ménopause ou à une ménopause induite médicalement causée par une chirurgie ou un médicament, syndrome des vomissements périodiques, sevrage des opioïdes, psoriasis, asthme, obésité, tolérance à la morphine, maladies neurodégénératives, épilepsie, rhinite allergique, rosacée, mal de dents, mal d'oreille, otite moyenne, coup de soleil, arthralgie liée à l'ostéoarthrite et à la polyarthrite rhumatoïde, douleur cancéreuse, fibromyalgie, neuropathie diabétique, goutte, névralgie du trijumeau, polypes nasaux, sinusite chronique, syndrome temporomandibulaire, douleur dorsale, lombalgie, toux, douleur dystonique, douleur inflammatoire, douleur d'incision postopératoire, sciatique, syndrome douloureux régional complexe, maladie de Behçet, endométriose, syndrome du membre fantôme, menstruation douloureuse, douleur liée à l'accouchement, douleur causée par des brûlures cutanées, et douleur viscérale secondaire chronique, telle qu'une maladie inflammatoire de l'intestin (comprenant la maladie de Crohn, l'iléite et la colite ulcéreuse), reflux gastro-œsophagien, dyspepsie, syndrome du côlon irritable, colique néphrétique, cystite, pancréatite, et prostatite.

14. Composé selon l'une quelconque des revendications 1 à 9 ou un stéréoisomère, tautomère, dérivé deutéré ou sel pharmaceutiquement acceptable de celui-ci, ou composition pharmaceutique selon la revendication 10 pour une utilisation dans la prévention et/ou le traitement d'une maladie médiée par le CGRP, dans lequel la maladie médiée par le CGRP est une maladie de trouble cérébrovasculaire ou vasculaire.

15. Composé ou un stéréoisomère, tautomère, dérivé deutéré ou sel pharmaceutiquement acceptable de celui-ci, ou composition pharmaceutique pour une utilisation selon la revendication 14, dans lequel la maladie de trouble vasculaire cérébral ou vasculaire médiée par le CGRP est choisie dans le groupe constitué de : migraine épisodique, migraine sans aura, migraine chronique, migraine menstruelle pure, migraine liée aux menstruations, migraine avec aura, migraine chez les enfants/adolescents, migraine hémiplégique, migraine hémiplégique sporadique, migraine basilaire, vomissements périodiques, migraine abdominale, vertige paroxystique bénin de l'enfance, migraine rétinienne, céphalée en grappes, céphalée de dialyse, céphalée chronique de cause inconnue, céphalée induite par tension/pression, céphalée induite par allergie, ostéoarthrite et douleur de fracture ostéoporotique apparentée, bouffées de chaleur liées à la ménopause ou à une ménopause induite médicalement causée par une chirurgie ou un médicament, syndrome des vomissements périodiques, sevrage des opioïdes, psoriasis, asthme, obésité, tolérance à la morphine, maladies neurodégénératives, épilepsie, rhinite allergique, rosacée, mal de dents, mal d'oreille, otite moyenne, coup de soleil, arthralgie liée à l'ostéoarthrite et à la polyarthrite rhumatoïde, douleur cancéreuse, fibromyalgie, neuropathie diabétique, goutte, névralgie du trijumeau, polypes nasaux, sinusite chronique, syndrome temporomandibulaire, douleur dorsale, lombalgie, toux, douleur dystonique, douleur inflammatoire, douleur d'incision postopératoire, sciatique, syndrome douloureux régional complexe, maladie de Behçet, endométriose, syndrome du membre fantôme, menstruation douloureuse, douleur liée à l'accouchement, douleur causée par des brûlures cutanées, et douleur viscérale secondaire chronique, telle qu'une maladie inflammatoire de l'intestin (comprenant la maladie de Crohn, l'iléite et la colite ulcéreuse), reflux gastro-œsophagien, dyspepsie, syndrome du côlon irritable, colique néphrétique, cystite, pancréatite, et prostatite.
